# EUROPÄISCHE PATENTSCHRIFT

(11) **EP 0 509 354 B1**
(45) Veröffentlichungstag und Bekanntmachung des Hinweises auf die Patenterteilung: **23.08.2000**
(21) Anmeldenummer: 92105879.8
(22) Anmeldetag: 06.04.1992
(51) Int. Cl.: C07D 233/64, A61K 38/00, C07D 233/56, C07D 231/12, C07D 233/60, C07D 333/24, C07D 307/54, C07D 277/30, C07D 401/12, C07D 409/12, C07D 407/12

(54) **Aminosäurederivate**
Amino acid derivatives
Dérivés d'aminoacides

(30) Priorität: 17.04.1991 CH 114691; 20.02.1992 CH 52392
(43) Veröffentlichungstag der Anmeldung: 21.10.1992
(73) Patentinhaber: F. HOFFMANN-LA ROCHE AG, 4002 Basel (CH)
(72) Erfinder: Branca, Quirico Dr., CH-4102 Binningen/BL (CH); Heitz, Marie-Paule, F-68870 Bartenheim (FR); Neidhart, Werner, F-68870 Bartenheim (FR); Stadler, Heinz, CH-4310 Rheinfelden (CH); Vieira, Eric, CH-4053 Basel (CH); Wostl, Wolfgang, W-7889 Grenzach-Wyhlen (DE)
(74) Vertreter: Kellenberger, Marcus, Dr.

(56) Entgegenhaltungen:
- EP-A- 0 189 203
- EP-A- 0 229 667
- EP-A- 0 230 266
- EP-A- 0 309 841
- EP-A- 0 332 008
- EP-A- 0 416 373
- WO-A-87/04349

## Beschreibung

Die vorliegende Erfindung betrifft Aminosäurederivate. Im speziellen betrifft sie Aminosäurederivate der allgemeinen Formel 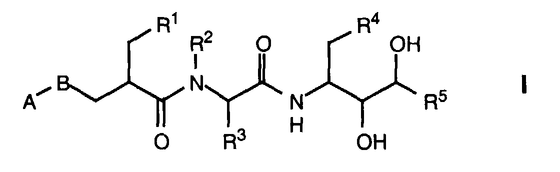
worin R¹ C₃-C₆-Cycloalkyl, Phenyl, gegebenenfalls ein- oder mehrfach substituiert durch C₁-C₆-Alkyl, C₁-C₆-Alkoxy, C₁-C₆-Alkylcarbonyloxy, Hydroxy, Halogen oder Trifluormethyl, R¹ auch Naphthyl, Thienyl, Pyridyl, Chinolyl, Isochinolyl oder Benzyl, R² Wasserstoff oder Methyl, R³ Wasserstoff, Hydroxy, C₁-C₆-Alkyl, C₁-C₆-Alkoxy, C₃-C₆-Alkenyloxy, C₁-C₆-Alkylthio, C₁-C₆-Alkylthio-C₁-C₆-alkyl, C₁-C₆-Alkoxycarbonyl, Imidazol-1-ylmethyl, Imidazol-2-ylmethyl oder Imidazol-4-ylmethyl, welche an einem Kohlenstoff- und/oder Stickstoffatom methyliert sein können, 2-Aminoimidazol-4-yl-methyl, 5-Jodimidazol-4-ylmethyl, Pyrazol-1-ylmethyl, Pyrazol-3-ylmethyl, Thiazol-2-ylmethyl, Thiazol-4-ylmethyl, Thiazol-5-ylmethyl, 2-Aminothiazol-4-ylmethyl, Thienylmethyl, Furanylmethyl, Pyridylmethyl, Aminocarbonyl, Aminocarbonyl-C₁-C₆-alkyl oder C₁-C₆-Alkoxycarbonyl-C₁-C₆-alkyl, R⁴ C₃-C₆-Cycloalkyl, gegebenenfalls substituiert durch C₁-C₆-Alkyl, Hydroxy doer Halogen, R⁴ weiterhin Phenyl oder Halophenyl, R⁵ C₃-C₆-Cycloalkyl, C₃-C₆-Cycloalkyl-C₁-C₆-alkyl oder C₁-C₆-Alkyl, B ein Schwefelatom oder eine Sulfinyl- oder Sulfonylgruppe und A die Gruppe 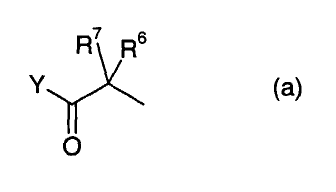 bedeuten, in welcher R⁶ und R⁷ unabhängig voneinander je Wasserstoff oder C₁-C₆-Alkyl und Y C₃-C₆-Cycloalkylamino, Sulfo-C₁-C₆-alkylamino, Bis-C₁-C₆-alkoxy-C₁-C₆-alkylamino, oder Pyridyl-C₁-C₆-alkylamino oder Morpholino-C₁-C₆-alkylamino, welche jeweils an der Aminogruppe durch C₁-C₆-Alkyl substituiert sein können, Pyrazinyl-C₁-C₆-alkylamino, welches am Stickstoffatom und/oder an der Aminogruppe durch C₁-C₆-Alkyl substituiert sein kann, 4-Piperidinyl-C₁-C₆-alkylamino, welches am Stickstoffatom durch C₁-C₆-Alkyl, Benzyl, Benzyloxycarbonyl, C₁-C₆-Alkoxycarbonyl oder Hydroxy-C₁-C₆-alkyl und/oder an der Aminogruppe durch C₁-C₆-Alkyl substituiert sein kann, C₁-C₆-Alkoxycarbonyl-C₁-C₆-alkylamino, Hydroxy-C₁-C₆-alkoxy-C₁-C₆-alkylamino, Hydroxy-C₁-C₆-alkylamino, Bishydroxy-C₁-C₆-alkylamino, den Rest eines natürlichen Aminodesoxyzuckers, oder die Gruppe (R^{a})(R^{b})N- bedeutet, in welcher R^{a} und R^{b} unabhängig voneinander je Wasserstoff oder C₁-C₆-Alkyl oder zusammen mit dem Stickstoffatom, an welches sie gebunden sind, einen gesättigten 5- oder 6-gliedrigen heterocyclischen Ring bedeuten, welcher ein zusätzliches Stickstoffatom oder ein Sauerstoff- oder Schwefelatom als weiteres Heteroatom enthalten oder eine Oxogruppe aufweisen kann, wobei das Schwefelatom auch in Form einer Sulfinyl- oder Sulfonylgruppe vorliegen kann, oder an einem oder zwei Kohlenstoffatomen durch C₁-C₆-Alkyl, Hydroxy, Hydroxy-C₁-C₆-alkyl oder C₁-C₆-Alkoxy-C₁-C₆-alkoxy oder am allfällig vorhandenen zweiten Stickstoffatom durch C₁-C₆-Alkyl Benzyl, Benzyloxycarbonyl C₁-C₆-Alkoxycarbonyl oder Hydroxy-C₁-C₆-alkyl substituiert sein kann, oder in welchem das allfällig vorhandene zweite Stickstoffatom ein Sauerstoffatom tragen kann,
in Form von optisch reinen Diastereomeren, Diastereomerengemische, diastereomeren Racematen oder Gemischen von diastereomeren Racematen sowie pharmazeutisch verwendbare Salze dieser Verbindungen.

Diese Verbindungen zeichnen sich durch wertvolle pharmakodynamische Eigenschaften aus.

Gegenstand der vorliegenden Erfindung sind die Verbindungen der Formel I und ihre pharmazeutisch verwendbaren Salze als solche und zur Anwendung als therapeutische Wirkstoffe, die Herstellung dieser Verbindungen, ferner diese enthaltende Arzneimittel und die Herstellung solcher Arzneimittel, sowie die Verwendung von Verbindungen der Formel I und ihrer pharmazeutisch verwendbaren Salze bei der Bekämpfung bzw. Verhütung von Krankheiten, bzw. bei der Verbesserung der Gesundheit, insbesondere bei der Bekämpfung bzw. Verhütung von Bluthochdruck und Herzinsuffizienz.

EP 0 309 841, EP 0 416 373, EP 0 130 266, EP 0 229 667, WO 87/04349, EP 0 189 203 und EP 0 332 008 offenbaren Renin-Inhibitoren, die insbesondere bei der Bekämpfung bzw. Verhütung von Bluthochdruck und Herzinsuffizienz eingesetzt werden können. Die Verbindungen der vorliegenden Erfindung besitzen aber überraschenderweise eine stark verbesserte antagonistische Potenz in vitro.

Die nachstehenden Definitionen der in der vorliegenden Beschreibung verwendeten allgemeinen Ausdrücke besitzen ihre Gültigkeit unabhängig davon, ob die fraglichen Ausdrücke allein oder in Kombination aufscheinen.

Der in der vorliegenden Beschreibung verwendete Ausdruck "Cycloalkyl" bedeutet gesättigte, cyclische Kohlenwasserstoffreste mit 3-6 Kohlenstoffatomen, d.h. Cyclopropyl, Cyclobutyl, Cyclopentyl und Cyclohexyl. Der Ausdruck "substituiertes Phenyl" bezeichnet durch Alkyl, Alkoxy, Alkylcarbonyloxy, Hydroxy, Halogen oder Trifluormethyl ein- oder mehrfach substituiertes Phenyl, wie 4-Hydroxyphenyl, 4-Methoxyphenyl, 4-Methylphenyl, 4-Chlorphenyl und dergleichen. Der Ausdruck "Alkyl" bedeutet geradkettige und verzweigte, gesättigte Kohlenwasserstoffreste mit 1-6, vorzugsweise 1-4, Kohlenstoffatomen, wie Methyl, Aethyl, n-Propyl, Isopropyl, n-Butyl, Isobutyl, sec.-Butyl, tert-Butyl, Pentyl, Hexyl und dergleichen. Der Ausdruck "Alkoxy" bedeutet Alkyläthergruppen, worin der Ausdruck "Alkyl" die obige Bedeutung hat, wie Methoxy, Aethoxy, Propoxy, Isopropoxy, Butoxy, Isobutoxy, sec.-Butoxy, tert-Butoxy und dergleichen. Der Ausdruck "Alkenyloxy" betrifft einen über ein Sauerstoffatom gebundenen, ungesättigten Kohlenwasserstoffrest mit 3-6 Kohlenstoffatomen, wie Allyloxy und dergleichen. Der Ausdruck "substituiertes Cycloalkyl" bedeutet durch Alkyl, Hydroxy oder Halogen ein- oder mehrfach substituiertes Cycloalkyl, wobei der Ausdruck "Cycloalkyl" die obige Bedeutung hat, wie 4-Hydroxycyclohexyl, 4-Fluorcyclohexyl, 4,4-Difluorcyclohexyl und dergleichen.

Der Ausdruck "Bisalkoxyalkylamino" bzw. "Bishydroxyalkylamino" betrifft eine Aminogruppe, welche am Stickstoffatom entweder durch zwei Alkoxyalkyl- bzw. Hydroxyalkylreste disubstituiert oder durch einen Dialkoxyalkyl- bzw. Dihydroxyalkylrest monosubstituiert ist, wie Bis(dimethoxyäthyl)amino, 1-Hydroxy-2-hydroxymethyl-2-propylamino und dergleichen.

Beispiele für gesättigte 5- und 6-gliedrige heterocyclische Ringe sind Pyrrolidinyl, Piperidinyl, Piperazinyl, Morpholinyl, Thiomorpholinyl, 2-Hydroxymethylpyrrolidinyl, 3-Hydroxypyrrolidinyl, 3,4-Dihydroxypyrrolidinyl, 4-Hydroxypiperidinyl, 4-Oxopiperidinyl, 3,5-Dimethylmorpholinyl, 4,4-Dioxothiomorpholinyl, 4-Oxothiomorpholinyl, 2,6-Dimethylmorpholinyl und dergleichen. Beispiele von natürlichen Aminodesoxyzuckern sind Glucosamin, Mannosamin, Galactosamin und dergleichen.

Der Ausdruck "pharmazeutisch verwendbare Salze" umfasst Salze mit anorganischen oder organischen Säuren, wie Salzsäure, Bromwasserstoffsäure, Salpetersäure, Schwefelsäure, Phosphorsäure, Zitronensäure, Ameisensäure, Maleinsäure, Essigsäure, Bernsteinsäure, Weinsäure, Methansulfonsäure, p-Toluolsulfonsäure und dergleichen, oder, für den Fall, dass Y Sulfoalkylamino bedeutet, auch mit anorganischen oder organischen Basen, wie Natrium- oder Kaliumhydroxyd, Ammoniak, Triäthylamin, Diisopropyläthylamin, Pyridin und dergleichen. Solche Salze können im Hinblick auf den Stand der Technik und unter Berücksichtigung der Natur der in ein Salz zu überführenden Verbindung durch jeden Fachmann ohne weiteres hergestellt werden.

Die Verbindungen der Formel I besitzen mindestens vier asymmetrische Kohlenstoffatome und liegen deshalb in Form von optisch reinen Diastereomeren, Diastereomerengemischen, diastereomeren Racematen oder Gemischen von diastereomeren Racematen vor. Die vorliegende Erfindung umfasst alle Formen. Diastereomerengemische, diastereomere Racemate oder Gemische von diastereomeren Racematen können nach üblichen Methoden aufgetrennt werden, z.B. durch Säulenchromatographie, Dünnschichtchromatographie, HPLC und dergleichen.

Bevorzugt sind solche Verbindungen der Formel I, worin R¹ Cycloalkyl, Phenyl, substituiertes Phenyl, Naphthyl, Thienyl oder Pyridyl, besonders bevorzugt Cyclohexyl, Phenyl, Naphthyl oder Thienyl, insbesondere Phenyl, bedeutet. Die bevorzugte Bedeutung von R² ist Wasserstoff. R³ bedeutet vorzugsweise Wasserstoff, Hydroxy, Alkyl, Alkoxy, Allyloxy, Alkylthio, Allkylthiomethyl, Alkoxycarbonyl, Imidazol-1-ylmethyl, Imidazol-4-ylmethyl, 2-Aminoimidazol-4-ylmethyl, 5-Jodimidazol-4-ylmethyl, Pyrazol-1-ylmethyl, Thiazol-4-ylmethyl, Thien-2-ylmethyl, Furan-2-ylmethyl, Pyridylmethyl oder Aminocarbonyl, besonders bevorzugt Wasserstoff, Hydroxy, C₁-C₄-Alkyl, C₁-C₄-Alkoxy, Allyloxy, C₁-C₄-Alkylthio, C₁-C₄-Alkylthiomethyl, C₁-C₄-Alkoxycarbonyl, Imidazol-4-ylmethyl, Thiazol-4-ylmethyl oder Pyridylmethyl, insbesondere Imidazol-4-ylmethyl oder 3-Pyridylmethyl. Weiter sind solche Verbindungen der Formel I bevorzugt, worin R⁴ Cycloalkyl oder Phenyl, besonders bevorzugt Cyclohexyl, bedeutet. R⁵ bedeutet vorzugsweise Cycloalkyl oder Alkyl, besonders bevorzugt Cyclopropyl, Isopropyl oder Isobutyl, insbesondere Cyclopropyl. Die bevorzugte Bedeutung von B ist Sulfonyl. R⁶ und R⁷ bedeuten vorzugsweise je Wasserstoff oder C₁-C₄-Alkyl, besonders bevorzugt Methyl. Y bedeutet vorzugsweise Cycloalkylamino, Sulfoalkylamino, Bisalkoxyalkylamino, Pyridylalkylamino, welches an der Aminogruppe durch Alkyl substituiert sein kann, 4-Piperidinylalkylamino, welches am Stickstoffatom durch Alkyl, Benzyl, Benzyloxycarbonyl, Alkoxycarbonyl oder Hydroxyalkyl substituiert sein kann, Alkoxycarbonylalkylamino, Hydroxyalkoxyalkylamino, Hydroxyalkylamino, Bishydroxyalkylamino oder die Gruppe (R^{a})(R^{b})N-, in welcher R^{a} und R^{b} unabhängig voneinander je Wasserstoff oder Alkyl oder zusammen mit dem Stickstoffatom, an welches sie gebunden sind, einen gesättigten 5- oder 6-gliedrigen heterocyclischen Ring bedeuten, welcher ein zusätzliches Stickstoffatom oder ein Sauerstoff- oder Schwefelatom als weiteres Heteroatom enthalten oder eine Oxogruppe aufweisen kann, wobei das Schwefelatom auch in Form einer Sulfinyl- oder Sulfonylgruppe vorliegen kann, oder an einem oder zwei Kohlenstoffatomen durch Alkyl, Hydroxy, Hydroxyalkyl oder Alkoxyalkoxy oder am allfällig vorhandenen zweiten Stickstoffatom durch Alkyl, Benzyl, Benzyloxycarbonyl, Alkoxycarbonyl oder Hydroxyalkyl substituiert sein kann. Besonders bevorzugt ist Y in der Bedeutung der Gruppe (R^{a})(R^{b})N-, in welcher R^{a} und R^{b} zusammen mit dem Stickstoffatom, an welches sie gebunden sind, einen gesättigten 5- oder 6-gliedrigen heterocyclischen Ring bedeuten, welcher ein zusätzliches Stickstoffatom oder ein Sauerstoff- oder Schwefelatom als weiteres Heteroatom enthalten kann, wobei das Schwefelatom auch in Form einer Sulfinyl- oder Sulfonylgruppe vorliegen kann, oder an einem oder zwei Kohlenstoffatomen durch Alkyl, Hydroxy, Hydroxyalkyl oder Alkoxyalkoxy oder am allfällig vorhandenen zweiten Stickstoffatom durch Alkyl, Benzyl, Benzyloxycarbonyl, Alkoxycarbonyl oder Hydroxyalkyl substituiert sein kann, oder eine Oxogruppe aufweisen kann. Ganz besonders bevorzugt bedeutet Y Pyrrolidinyl, Piperidinyl, Piperazinyl, Morpholinyl, Thiomorpholinyl, 2-Hydroxymethylpyrrolidinyl, 3-Hydroxypyrrolidinyl, 3,4-Dihydroxypyrrolidinyl, 4-Hydroxypiperidinyl, 4-Oxopiperidinyl, 3,5-Dimethylmorpholinyl, 4,4-Dioxothiomorpholinyl, 4-Oxothiomorpholinyl oder 2,6-Dimethylmorpholinyl, insbesondere Morpholinyl. Aus dem obigen folgt, dass solche Verbindungen der Formel I besonders bevorzugt sind, worin R¹ Cyclohexyl, Phenyl, Naphthyl oder Thienyl, R² Wasserstoff, R³ Wasserstoff, Hydroxy, C₁-C₄-Alkyl, C₁-C₄-Alkoxy, Allyloxy, C₁-C₄-Alkylthio, C₁-C₄-Alkylthiomethyl, C₁-C₄-Alkoxycarbonyl, Imidazol-4-ylmethyl, Thiazol-4-ylmethyl oder Pyridylmethyl, R⁴ Cyclohexyl, R⁵ Cyclopropyl, Isopropyl oder Isobutyl, B Sulfonyl, R⁶ und R⁷ je Methyl und Y Pyrrolidinyl, Piperidinyl, Piperazinyl, Morpholinyl, Thiomorpholinyl, 2-Hydroxymethylpyrrolidinyl, 3-Hydroxypyrrolidinyl, 3,4-Dihydroxypyrrolidinyl, 4-Hydroxypiperidinyl, 4-Oxopiperidinyl, 3,5-Dimethylmorpholinyl, 4,4-Dioxothiomorpholinyl, 4-Oxothiomorpholinyl oder 2,6-Dimethylmorpholinyl bedeuten.

Besondere Verbindungen der Formel I sind solche, worin R¹ Cycloalkyl, Phenyl, substituiertes Phenyl, Naphthyl, Thienyl, Pyridyl, Chinolyl, Isochinolyl oder Benzyl, R² Wasserstoff oder Methyl, R³ Wasserstoff, Hydroxy, Alkyl, Alkoxy, Alkenyloxy, Alkylthio, Alkylthioalkyl, Alkoxycarbonyl, Imidazol-2-ylmethyl oder Imidazol-4-ylmethyl, welche an einem Kohlenstoff- und/oder Stickstoffatom methyliert sein können, 2-Aminoimidazol-4-yl-methyl, 5-Jodimidazol-4-ylmethyl, Thiazol-4-ylmethyl, Thienylmethyl, Furanylmethyl, Pyridylmethyl, Aminocarbonyl, Aminocarbonylalkyl oder Alkoxycarbonylalkyl, R⁴ Cycloalkyl, substituiertes Cycloalkyl, Phenyl oder Halophenyl, R⁵ Cycloalkyl, Cycloalkylalkyl oder Alkyl, B ein Schwefelatom oder eine Sulfinyl- oder Sulfonylgruppe, R⁶ und R⁷ unabhängig voneinander je Wasserstoff oder Alkyl und Y Cycloalkylamino, Sulfoalkylamino, Bisalkoxyalkylamino, Pyridylalkylamino, 4-Piperidinylalkylamino, welches am Stickstoffatom durch Alkyl, Benzyl, Benzyloxycarbonyl, Alkoxycarbonyl oder Hydroxyalkyl substituiert sein kann, Alkoxycarbonylalkylamino, Hydroxyalkylamino, Bishydroxyalkylamino, die Gruppe (R^{a})(R^{b})N-, in welcher R^{a} und R^{b} unabhängig voneinander je Wasserstoff oder Alkyl oder zusammen mit dem Stickstoffatom, an welches sie gebunden sind, einen gesättigten 5- oder 6-gliedrigen heterocyclischen Ring bedeuten, welcher ein zusätzliches Stickstoffatom oder ein Sauerstoff- oder Schwefelatom als weiteres Heteroatom enthalten kann, wobei das Schwefelatom auch in Form einer Sulfinyl- oder Sulfonylgruppe vorliegen kann, an einem oder zwei Kohlenstoffatomen durch Alkyl, Hydroxy, Hydroxyalkyl oder Alkoxyalkoxy, am allfällig vorhandenen zweiten Stickstoffatom durch Alkyl, Benzyl, Benzyloxycarbonyl, Alkoxycarbonyl oder Hydroxyalkyl substituiert sein kann, oder eine Oxogruppe aufweisen kann, bedeuten.

Speziell bevorzugte Verbindungen der Formel I sind:
(S)-N-[(1S,2R,3S)-1-(Cyclohexylmethyl)-3-cyclopropyl-2,3-dihydroxypropyl]-α-[(S)-α-[[[1-[[2-hydroxy-1-(hydroxymethyl)-1-methyläthyl]carbamoyl]-1-methyläthyl]sulfonyl]methy]hydrocinnamamido]imidazol-4-propionamid,
(S)-α-[(R oder S)-α-[[[1-[(2-Pyridylmethyl)carbamoyl]-1-methyläthyl]sulfonyl]methyl]hydrocinnamamido]-N-[(1S,2R,3S)-1-(cyclohexylmethyl)-3-cyclopropyl-2,3-dihydroxypropyl]imidazol-4-propionamid und
(S)-α-[(R oder S)-α-[[[1-[(1-Benzyl-4-piperidinyl)carbamoyl]-1-methyläthyl]sulfonyl]methyl]hydrocinnamamido]-N-[(1S,2R,3S)-1-(cyclohexylmethyl)-3-cyclopropyl-2,3-dihydroxypropyl]imidazol-4-propionamid.

Weitere speziell bevorzugte Verbindungen der Formel I sind:
2-[2-[[(S)-2-[[(S)-1-[[(1S,2R,3S)-1-(Cyclohexylmethyl)-3-cyclopropyl-2,3-dihydroxypropyl]carbamoyl]-2-imidazol-4-yläthyl]carbamoyl]-3-phenylpropyl]sulfonyl]-2-methylpropionamido]-2-deoxy-D-glucopyranose,
(S)-α-[(R oder S)-α-[[[1-[bis(2-Methoxyäthyl)carbamoyl]-1-methyläthyl]sulfonyl]methyl]hydrocinnamamido]-N-[(1S,2R,3S)-1-(cyclohexylmethyl)-3-cyclopropyl-2,3-dihydroxypropyl]imidazol-4-propionamid, und
(S oder R)-N-[(1S,2R,3S)-1-(Cyclohexylmethyl)-3-cyclopropyl-2,3-dihydroxypropyl]-α-[(S)-α-[[[1-methyl-1-[morpholinocarbonyl]äthyl]sulfonyl]methyl]hydrocinnamamido]-3-(thiazolyl-4-yl)propionamid.

Ganz speziell bevorzugte Verbindungen der Formel I sind:
(S)-N-[(1S,2R,3S)-1-(Cyclohexylmethyl)-3-cyclopropyl-2,3-dihydroxypropyl]-α-[(S)-α-[[[1-(morpholinocarbonyl)-1-methyläthyl]sulfonyl]methyl]hydrocinnamamido]imidazol-4-propionamid,
(S)-N-[(1S,2R,3S)-1-(Cyclohexylmethyl)-3-cyclopropyl-2,3-dihydroxypropyl]-α-[(S)-α-[[[1-[(4-hydroxypiperidino)carbonyl]-1- methyläthyl]sulfonyl]methyl]hydrocinnamamido]imidazol-4-propionamid und
(S oder R)-N-[(1S,2R,3S)-1-(Cyclohexylmethyl)-3-cyclopropyl-2,3-dihydroxypropyl]-α-[(S)-α-[[[1-methyl-1-[morpholinocarbonyl]äthyl]sulfonyl]methyl]hydrocinnamamido]-3-(pyridin-3-yl)propionamid.

Verbindungen der Formel I in Form von optisch reinen Diastereomeren, Diastereomerengemischen, diastereomeren Racematen oder Gemischen von diastereomeren Racematen sowie pharmazeutisch verwendbare Salze davon können hergestellt werden, indem man
a) eine Verbindung der allgemeinen Formel 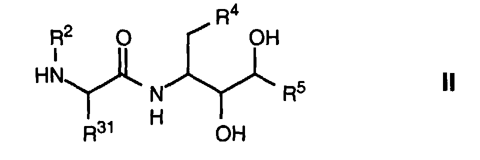
   worin R³¹ Wasserstoff, Alkyl, Alkylthioalkyl, Alkoxycarbonyl, Imidazol-1-ylmethyl, Imidazol-2-ylmethyl oder Imidazol-4-ylmethyl, welche an einem Kohlenstoff- und/oder Stickstoffatom methyliert sein können, 2-Aminoimidazol-4-yl-methyl, 5-Jodimidazol-4-ylmethyl, Pyrazol-1-ylmethyl, Pyrazol-3-ylmethyl, Thiazol-2-ylmethyl, Thiazol-4-ylmethyl, Thiazol-5-ylmethyl, 2-Aminothiazol-4-ylmethyl, Thienylmethyl, Furanylmethyl, Pyridylmethyl, Aminocarbonyl, Aminocarbonylalkyl oder Alkoxycarbonylalkyl bedeutet und R², R⁴ und R⁵ die oben angegebene Bedeutung besitzen,
      mit einer Säure der allgemeinen Formel 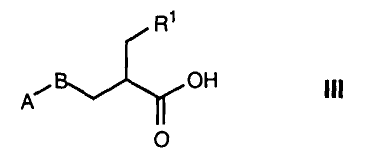
   worin A, B und R¹ die oben angegebene Bedeutung besitzen, oder einem aktivierten Derivat davon umsetzt, oder
b) eine Verbindung der allgemeinen Formel 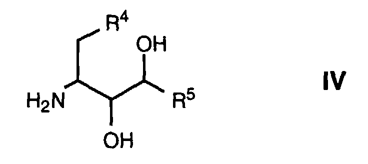
   worin R⁴ und R⁵ die oben angegebene Bedeutung besitzen,
      mit einer Säure der allgemeinen Formel 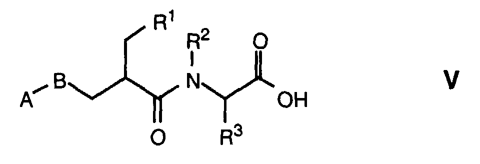
   worin A, B, R¹, R² und R³ die oben angegebene Bedeutung besitzen, oder einem aktivierten Derivat davon umsetzt, oder
c) zur Herstellung einer Verbindung der Formel I, worin A freie primäre oder sekundäre Aminogruppen und/oder freie primäre oder sekundäre Hydroxygruppen enthält, aus einer Verbindung der allgemeinen Formel 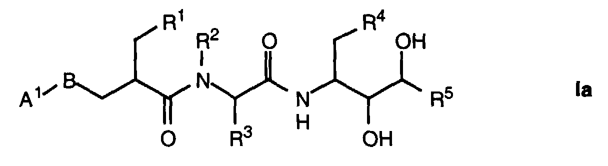
   worin A¹ die gleiche Bedeutung wie A hat, mit der Maßgabe, daß A eine N- oder O-Schutzgruppe enthält,
   die N- oder O-Schutzgruppe(n) abspaltet, und
d) erwünschtenfalls ein Gemisch diastereomerer Racemate in die diastereomeren Racemate oder optisch reinen Diastereomeren auftrennt, und/oder
e) erwünschtenfalls ein Diastereomerengemisch in die optisch reinen Diastereomeren auftrennt, und/oder
f) erwünschtenfalls eine erhaltene Verbindung in ein pharmazeutisch verwendbares Salz überführt.

Die Acylierung einer Verbindung der Formel II mit einer Säure der Formel III oder einem aktivierten Derivat davon erfolgt nach an sich in der Peptid-Chemie bekannten Methoden. Geeignete Acylierungsmittel sind insbesondere aktivierte Derivate, wie Ester, gemischte Ester, Säurehalogenide, Säureanhydride oder gemischte Säureanhydride. Die Reaktion wird in einem unter den Reaktionsbedingungen inerten organischen Lösungsmittel oder Lösungsmittelgemisch bei einer Temperatur zwischen etwa 0°C und der Raumtemperatur durchgeführt. Als Lösungsmittel kommen insbesondere aromatische Kohlenwasserstoffe, wie Benzol, Toluol oder Xylol, chlorierte Kohlenwasserstoffe, wie Methylenchlorid oder Chloroform, Äther, wie Diäthyläther, Tetrahydrofuran oder Dioxan, und dergleichen in Frage. Weiterhin kann die Acylierung in Gegenwart eines Kondensationsmittels, wie HBTU [O-Benzotriazolyl-N,N,N',N'-tetramethyluronium-hexafluorophosphat], BOP [Benzotriazol-1-yloxy-bis(dimethylamino)phosphonium-hexafluorophosphat], BOPC [Bis(2-oxo-2-oxozolidinyl)phosphinchlorid], HOBT [N-Hydroxybenzotriazol], DCC [Dicyclohexylcarbodiimid-hydrochlorid], EDC [N-(3-Dimethylaminopropyl)-N'-äthylcarbodiimid-hydrochlorid] und dergleichen, erfolgen. Die Reaktion wird zweckmäßigerweise in einem unter den Reaktionsbedingungen inerten organischen Lösungsmittel oder Lösungsmittelgemisch bei einer Temperatur zwischen etwa 0° und 50°C, vorzugsweise bei etwa Raumtemperatur, durchgeführt. Als Lösungsmittel kommen insbesondere Dimethylformamid, Methylenchlorid, Acetonitril, Tetrahydrofuran, und dergleichen in Frage.

Die Umsetzung einer Verbindung der Formel IV mit einer Säure der Formel V bzw. einem aktivierten Derivat davon erfolgt ebenfalls nach an sich in der Peptid-Chemie bekannten Methoden, d.h. unter den gleichen Bedingungen wie oben für die Acylierung einer Verbindung der Formel II angegeben wurde. Beispiele geeigneter aktivierter Verbindungen der Formel V sind ebenfalls Säurehalogenide, Säureanhydride, gemischte Anhydride, Ester, gemischte Ester, und dergleichen.

Die Abspaltung der N- bzw. O-Schutzgruppe(n) gemäß Verfahrensvariante c) erfolgt ebenfalls nach an sich bekannten Methoden in Abhängigkeit von der Art der abzuspaltenden N- oder O-Schutzgruppe. Die Abspaltung erfolgt jedoch zweckmäßigerweise durch saure oder basische Hydrolyse oder durch Hydrogenolyse. Für die saure Hydrolyse verwendet man vorteilhafterweise eine Lösung einer Mineralsäure, wie Chlorwasserstoffsäure, Bromwasserstoffsäure, Schwefelsäure, Phosphorsäure und dergleichen, in einem inerten Lösungsmittel oder Lösungsmittelgemisch. Geeignete Lösungsmittel sind Alkohole, wie Methanol oder Äthanol, Äther, wie Tetrahydrofuran oder Dioxan, chlorierte Kohlenwasserstoffe, wie Methylenchlorid, und dergleichen. Für die basische Hydrolyse können Alkalimetallhydroxide und -carbonate, wie Kalium- oder Natriumhydroxid oder Kalium- oder Natriumcarbonat, organische Amine, wie Piperidin, und dergleichen verwendet werden. Inerte organische Lösungsmittel, wie sie oben für die saure Hydrolyse genannt sind, können als Lösungsvermittler eingesetzt werden. Die Reaktionstemperatur kann für die saure und basische Hydrolyse in einem Bereich von 0°C bis Rückflußtemperatur variiert werden, wobei man vorzugsweise zwischen etwa 0°C und der Raumtemperatur arbeitet. Der tert-Butoxycarbonylrest wird zweckmäßigerweise mit wäßriger Salzsäure oder Salzsäure/Dioxan oder mit Trifluoressigsäure oder Ameisensäure in Gegenwart oder Abwesenheit eines inerten Lösungsmittels abgespalten. Die Benzyloxycarbonylgruppe kann in bekannter Weise durch saure Hydrolyse, wie weiter oben beschrieben, oder hydrogenolytisch abgespalten werden. Die Abspaltung der Acetalschutzgruppe kann in bekannter Weise durch wäßrig saure Hydrolyse oder beispielsweise mit Eisentrichlorid/Kieselgel durchgeführt werden.

Die Ausgangsstoffe der Formel II sind teilweise neu und teilweise bekannt. Diese Verbindungen können hergestellt werden, indem man eine Verbindung der Formel IV mit einer Verbindung der allgemeinen Formel 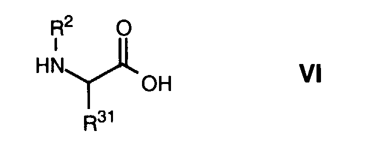
worin R² und R³¹ die oben angegebene Bedeutung besitzen,
umsetzt. Diese Umsetzung erfolgt nach in der Peptid-Chemie bekannten Methoden, d.h. unter den Reaktionsbedingungen, wie sie weiter oben für die Acylierung einer Verbindung der Formel II beschrieben sind.

Die Ausgangsstoffe der Formel IV sind ebenfalls teilweise neu und teilweise bekannt und können hergestellt werden, indem man in einer Verbindung der allgemeinen Formel 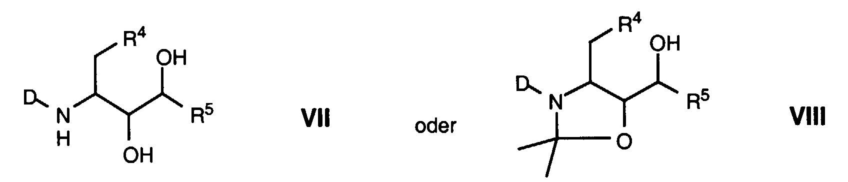
worin D eine Aminoschutzgruppe bedeutet, vorzugsweise tert-Butoxycarbonyl oder Benzyloxycarbonyl, und R⁴ und R⁵ die oben angegebene Bedeutung besitzen,
die Aminoschutzgruppe und gegebenenfalls gleichzeitig auch die O-Schutzgruppe abspaltet.

Die Abspaltung der N-Schutzgruppe und gegebenenfalls der O-Schutzgruppe erfolgt ebenfalls nach an sich bekannten Methoden, beispielsweise in einem unter den Reaktionsbedingungen inerten organischen Lösungsmittel oder Lösungsmittelgemisch bei einer Temperatur zwischen etwa 0° und 50°C mit einer Säure, wie Chlorwasserstoffsäure, Trifluoressigsäure, und dergleichen. Geeignete Lösungsmittel sind Äther, wie Tetrahydrofuran oder Dioxan, Alkohole, wie Methanol, oder chlorierte Kohlenwasserstoffe, wie Methylenchlorid, und dergleichen. Unter diesen Bedingungen wird in einer Verbindung der Formel VIII - wie bereits erwähnt - gleichzeitig der Oxazolidinring aufgespalten. Ist allerdings ausschliesslich die Aufspaltung des Oxazolidinrings erwünscht, so sind andere Reaktionsbedingungen zu wählen: die Reaktion muss bei tieferen Temperaturen und in wäßrigen Lösungsmitteln oder beispielsweise mit Eisentrichlorid/Kieselgel durchgefünrt werden.

Die Verbindungen der Formeln VII und VIII sind ebenfalls teilweise neu und teilweise bekannt und können wie im nachstehenden Reaktionsschema I formelmäßig angegeben, hergestellt werden.

Die Verbindungen der Formel VI sind bekannt oder können in Analogie zur Herstellung der bekannten Verbindungen erhalten werden.

Die Säuren der Formel III und ihre aktivierten Derivate sind neu und ebenfalls Gegenstand vorliegender Erfindung. Im nachstehenden Reaktionsschema II ist die Herstellung der Säuren der Formel III formelmäßig dargestellt. Die aktivierten Derivate können im Hinblick auf den Stand der Technik und unter Berücksichtigung der Natur des aktivierten Derivates durch jeden Fachmann ohne weiteres hergestellt werden.

Die Säuren der Formel V und ihre aktivierten Derivate sind ebenfalls neu und Gegenstand vorliegender Erfindung. Diejenigen Säuren, worin R³ die Bedeutung von R³¹ besitzt, können leicht hergestellt werden, indem man eine entsprechende Säure der Formel III mit einer Verbindung der Formel VI umsetzt. Die Umsetzung erfolgt nach in der Peptid-Chemie bekannten Methoden, d.h. unter den Reaktionsbedingungen, wie sie weiter oben für die Acylierung einer Verbindung der Formel II beschrieben sind. Diejenigen Säuren, worin R³ Hydroxy, Alkoxy, Alkenyloxy oder Alkylthio bedeutet, können wie im nachstehenden Reaktionsschema III formelmäßig angegeben, hergestellt werden. Die aktivierten Derivate der Säuren der Formel V können wie diejenigen der Säuren der Formel III durch jeden Fachmann ohne weiteres hergestellt werden.

Bei den in den Reaktionsschemata I bis III dargestellten Schritten handelt es sich durchwegs um in der synthetischen Chemie übliche Reaktionen, die alle nach an sich bekannten Methoden durchgeführt werden. Die Umsetzung der Carbonsäureamide der Formel XXII, welche ebenfalls neu und Gegenstand vorliegender Erfindung sind, erfolgt nach dem in EPA 0 331 921 beschriebenen Verfahren. Bezüglich der genauen Reaktionsbedingungen für die in den Reaktionsschemata I bis III dargestellten Schritte wird auf den Beispielteil verwiesen. Die in den Reaktionsschemata verwendeten Symbole R¹, R⁴, R⁵, R⁶, R⁷, A, B, Y und D besitzen die oben angegebene Bedeutung, während B* ein Schwefelatom oder eine Sulfonylgruppe, R Alkyl oder Benzyl und R³² Alkoxy, Alkenyloxy oder Alkylthio bedeuten.

Die in den Reaktionsschemata verwendeten Ausgangsstoffe der Formeln IX, XIII und XIV sind bekannt oder können in Analogie zur Herstellung der bekannten Verbindungen erhalten werden.

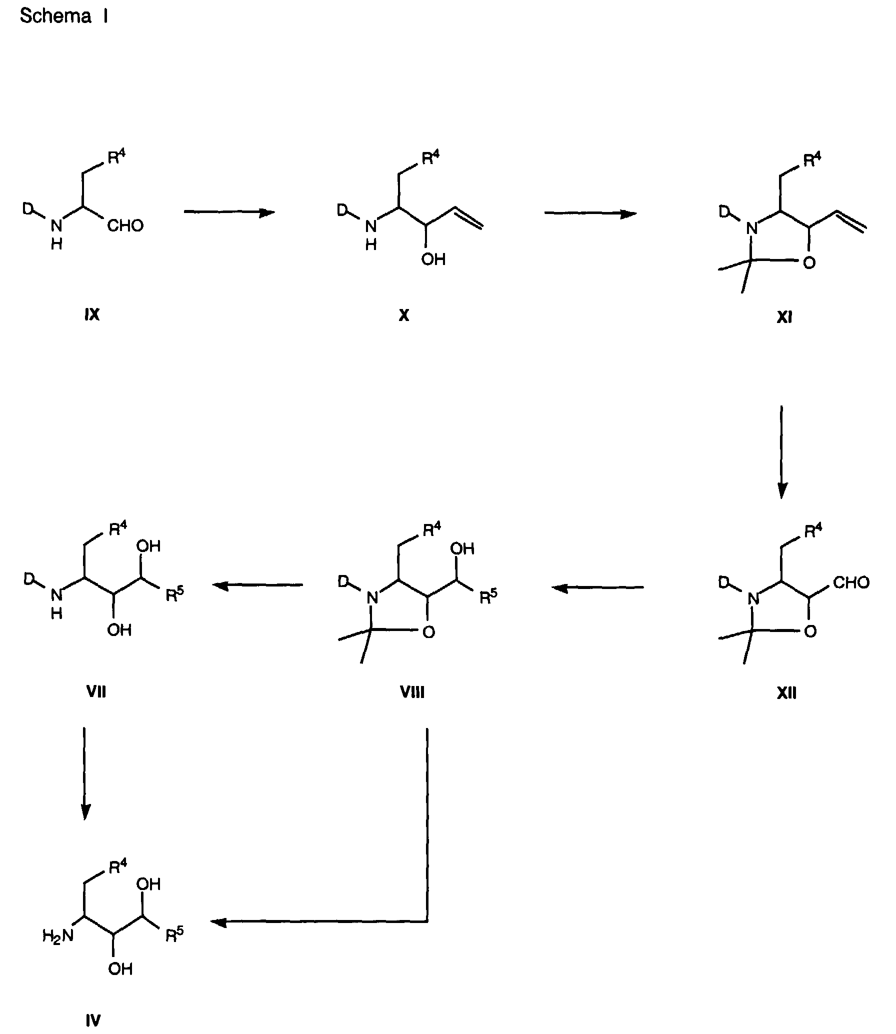

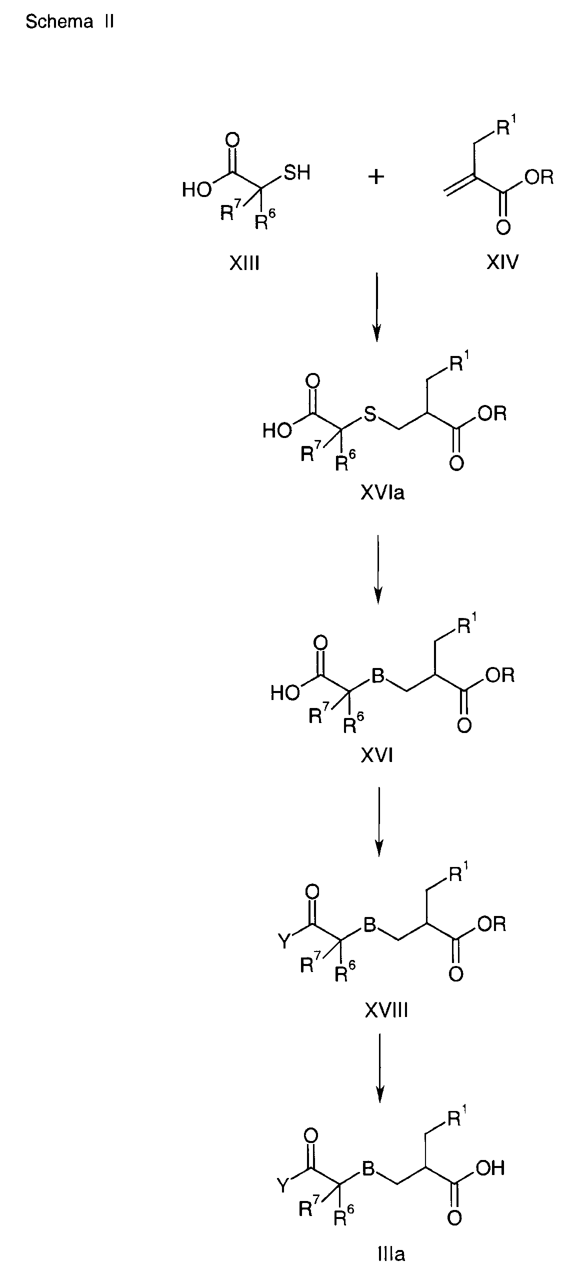

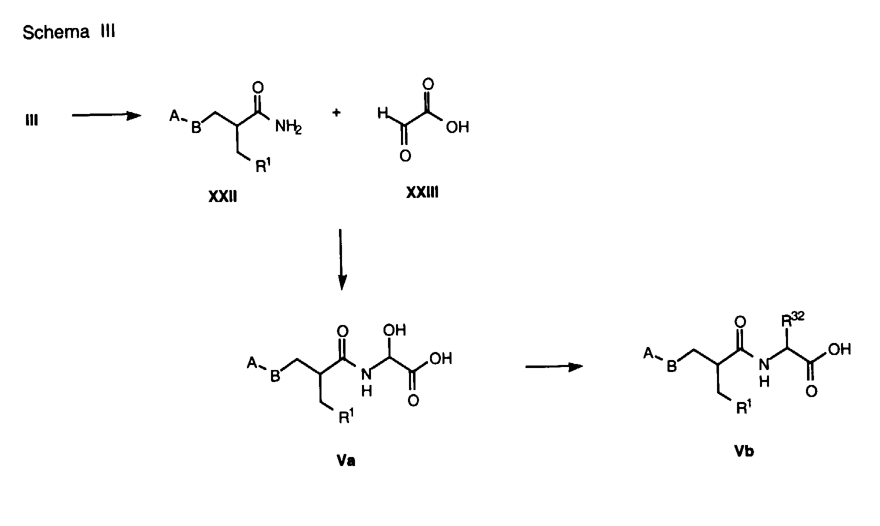

Die Verbindungen der Formel I und ihre pharmazeutisch verwendbaren Salze weisen hemmende Wirkung des natürlichen Enzyms Renin auf. Letzteres gelangt aus den Nieren in das Blut und bewirkt dort die Spaltung von Angiotensinogen unter Bildung des Dekapeptids Angiotensin I, das dann in der Lunge, den Nieren und anderen Organen zum Octapeptid Angiotensin II gespalten wird. Angiotensin II erhöht den Blutdruck sowohl direkt durch arterielle Konstriktion, als auch indirekt durch die Freisetzung des Natriumionen zurückhaltenden Hormons Aldosteron aus den Nebennieren, womit ein Anstieg des extrazellulären Flüssigkeitsvolumens verbunden ist. Dieser Anstieg ist auf die Wirkung von Angiotensin II selber oder des daraus als Spaltprodukt gebildeten Heptapeptids Angiotensin III zurückzuführen. Hemmer der enzymatischen Aktivität von Renin bewirken eine Verringerung der Bildung von Angiotensin I und als Folge davon die Bildung einer geringeren Menge von Angiotensin II. Die verminderte Konzentration dieses aktiven Peptid-Hormons ist die unmittelbare Ursache für die blutdrucksenkende Wirkung von Renin-Hemmern.

Die Wirkung von Renin-Hemmern kann experimentell mittels der nachstehend beschriebenen In vitro-Tests gezeigt werden:

### A. In vitro-Test mit reinem Human-Renin

Der Test wird in Eppendorf Röhrchen durchgeführt. Die Inkubationsmischung besteht aus (1) 100 µl Human-Renin in Puffer A (0.1M Natriumphosphatlösung, pH 7.4, enthaltend 0.1% Rinderserumalbumin, 0.1 % Natriumazid und 1 mM Aethylendiamintetraessigsäure), genügend für eine Renin-Aktivität von 2-3 ng Angiotensin I/ ml/Std.; (2) 145 µl Puffer A: (3) 30 µl von 10 µM humanem Tetradekapeptid-Reninsubstrat (hTD) in 10 mM Salzsäure: (4) 15 µl Dimethylsulfoxid mit bzw. ohne Hemmer und (5) 10 µl einer 0.03 molaren Lösung von Hydroxychinolinsulfat in Wasser.

Die Proben werden drei Stunden bei 37°C bzw. 4°C in Triplikaten inkubiert. 2 x 100 µl Proben pro Versuchsröhrchen werden dann verwendet, um die Produktion von Angiotensin I via RIA (standard radioimmunoassay; Clinical Assay solid phase kit) zu messen. Kreuzreaktivitäten der verwendeten Antikörper im RIA sind: Angiotensin I 100 %; Angiotensin II 0.0013 %; hTD (Angiotensin I-Val-Ile-His-Ser-OH) 0.09 %. Die Produktion von Angiotensin I wird durch die Differenz zwischen dem Versuch bei 37°C und demjenigen bei 4°C bestimmt.

Folgende Kontrollen werden mitgeführt:
(a) Inkubation von hTD-Proben ohne Renin und ohne Hemmer bei 37°C und 4°C. Die Differenz zwischen diesen beiden Werten ergibt den Grundwert der Angiotensin I-Produktion.
(b) Inkubation von hTD-Proben mit Renin, jedoch ohne Hemmer bei 37°C und 4°C. Die Differenz zwischen diesen Werten ergibt den Maximalwert der Angiotensin I-Produktion.

In jeder Probe wird von der ermittelten Angiotensin I-Produktion der Grundwert der Angiotensin I-Produktion abgezogen. Die Differenz zwischen dem Maximalwert und dem Grundwert ergibt den Wert der maximalen Substrathydrolyse (=100%) durch Renin.

Die Resultate werden als IC₅₀-Werte angegeben, welche diejenige Konzentration des Hemmers bezeichnen, bei welcher die enzymatische Aktivität um 50% gehemmt wird. Die IC₅₀-Werte werden aus einer linearen Regressionskurve aus einem logit-log plot ermittelt.

### B. In vitro-Test mit Human-Plasma

Die Testlösung setzt sich zusammen aus (1) 225µl Plasma, (2) 10 µl 8-Hydroxychinolinsulfat in Wasser, (3) 12 µl Natriumphosphatpuffer (0.1 M), pH 7.4, und 13 µl von verschiedenen Konzentrationen an Renin Hemmer in Dimethylsulfoxid. Der resultierende pH-Wert (pH 6) der Testlösung ist stabil für die Dauer der nachfolgenden Inkubation. Die Teströhrchen werden dann für 3 Stunden bei 37° C inkubiert. Die Enzymaktivität wird durch die Bestimmung des produzierten Angiotensin I ausgedrückt, welches mit einem kommerziell erhältlichen Radioimmunoassay Kit (Clinical Assays, Cambridge, Mass.) bestimmt wird.

Folgende Kontrollen werden mitgeführt:
- MaxP:: Maximale Produktion (maximale Enzymaktivität) in Abwesenheit eines Renin-Hemmers.
- MinP:: Minimale Produktion (Enzymaktivitäts-Blank) in Gegenwart einer supramaximalen Konzentration eines Renin-Hemmers.

Die MinP wird von der jeweiligen Angiotensin-Produktion in jedem Versuch abgezogen. Diese Werte werden in prozentuale Hemmung umgerechnet mittels Vergleich zur maximalen Enzymaktivität MaxP - MinP. Die Hemmstärke wird mit dem IC₅₀-Wert ausgedrückt, d.h. der Konzentration des Hemmers, welche die Akkumulation von Angiotensin I um 50 % reduziert.

Die in diesen Tests erhaltenen Resultate sind in der folgenden Tabelle zusammengefasst:

**Tabelle**

| Verbindung | IC₅₀-Werte in nMol/lt. | |
|---|---|---|
| | Test A | Test B |
| A | 0.03 | 0.10 |
| B | 0.30 | 0.11 |

| | | |
|---|---|---|
| A = (S)-N-[(1S,2R,3S)-1-(Cyclohexylmethyl)-3-cyclopropyl-2,3-dihydroxypropyl]-α-[(S)-α-[[[1-(morpholinocarbonyl)-1-methyläthyl]- sulfonyl]methyl]hydrocinnamamido]imidazol-4-propionamid | | |
| B = 2-[2-[[(S)-2-[[(S)-1-[[(1S,2R,3S)-1-(Cyclohexylmethyl)-3-cyclopropyl-2,3-dihydroxypropyl]carbamoyl]-2-imidazol-4-yläthyl]carbamoyl]-3- phenylpropyl]sulfonyl]-2-methylpropionamido]-2-deoxy-D-glucopyranose | | |

Die Verbindungen der Formel I sowie deren pharmazeutisch verwendbare Salze können als Heilmittel, z.B. in Form pharmazeutischer Präparate, Verwendung finden. Die pharmazeutischen Präparate können enteral, wie oral, z.B. in Form von Tabletten, Lacktabletten, Dragees, Hart- und Weichgelatinekapseln, Lösungen, Emulsionen oder Suspensionen, nasal, z.B. in Form von Nasensprays, oder rektal, z.B. in Form von Suppositorien, verabreicht werden. Die Verabreichung kann aber auch parenteral, wie intramuskulär oder intravenös, z.B. in Form von Injektionslösungen, erfolgen.

Zur Herstellung von Tabletten, Lacktabletten, Dragees und Hartgelatinekapseln können die Verbindungen der Formel I sowie ihre pharmazeutisch verwendbaren Salze mit pharmazeutisch inerten, anorganischen oder organischen Excipientien verarbeitet werden. Als solche Excipientien kann man z.B. für Tabletten, Dragees und Hartgelatinekapseln, Laktose, Maisstärke oder Derivate davon, Talk, Stearinsäure oder deren Salze etc. verwenden.

Für Weichgelatinekapseln eignen sich als Excipientien z.B. vegetabile Oele, Wachse, Fette, halbfeste und flüssige Polyole etc.

Zur Herstellung von Lösungen und Sirupen eignen sich als Excipientien z.B. Wasser. Polyole, Sacharose, Invertzucker, Glukose etc.

Für Injektionslösungen eignen sich als Excipientien z.B. Wasser, Alkohole, Polyole, Glycerin, vegetabile Oele etc.

Für Suppositorien eignen sich als Excipientien z.B. natürliche oder gehärtete Oele, Wachse, Fette, halbflüssige oder flüssige Polyole etc.

Die pharmazeutischen Präparate können daneben noch Konservierungsmittel, Lösungsvermittler, viskositätserhöhende Stoffe, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel oder Antioxidantien enthalten. Sie können auch noch andere therapeutisch wertvolle Stoffe enthalten.

Erfindungsgemäss kann man die Verbindungen der allgemeinen Formel I sowie deren pharmazeutisch verwendbare Salze bei der Bekämpfung bzw. Verhütung von Bluthochdruck und Herzinsuffizienz verwenden. Die Dosierung kann innerhalb weiter Grenzen variieren und ist natürlich in jedem einzelnen Fall den individuellen Gegebenheiten anzupassen. Im allgemeinen dürfte bei oraler Verabreichung eine Tagesdosis von etwa 3 mg bis etwa 3 g, vorzugsweise etwa 10 mg bis etwa 1 g, z.B. ungefähr 300 mg pro Person, verteilt auf vorzugsweise 1-3 Einzeldosen, die z.B. gleich gross sein können, angemessen sein, wobei aber die soeben angegebene obere Grenze auch überschritten werden kann, wenn sich dies als angezeigt erweisen sollte. Ueblicherweise erhalten Kinder die halbe Dosis von Erwachsenen.

Die folgenden Beispiele sollen die vorliegende Erfindung erläutern. Alle Temperaturen sind in Celsiusgraden angegeben. Es werden die folgenden Abkürzungen verwendet:
- His-OH: = L-Histidin
- Boc: = t-Butoxycarbonyl
- Fmoc: = 9-Fluorenylmethoxycarbonyl
- DBU: = 1,8-Diazabicyclo[5.4.0]undec-7-en[1,5-5]

### Beispiel 1

Ein Gemisch von 239 mg (0.76 mMol) rac-α-[[(1-Carbamoyl-1-methyläthyl)sulfonyl]methyl]hydrozimtsäure, 250 mg (0.69 mMol) (S)-α-Amino-N-[(1S,2R,3S)-1-(cyclohexylmethyl)-3-cyclopropyl-2,3-dihydroxypropyl]imidazol-4-propionamid, 77 mg (0.76 mMol) Triäthylamin, 118 mg (0.76 mMol) HOBT und 289 mg (0.76 mMol) HBTU in 10 ml Dimethylformamid wurde über Nacht bei Raumtemperatur gerührt. Anschließend wurde die Reaktionslösung im Hochvakuum zur Trockene eingedampft, der Rückstand in 75 ml Essigester aufgenommen und zweimal mit je 20 ml gesättigter Natriumhydrogencarbonatlösung gewaschen. Die Essigesterlösung wurde über Natriumsulfat getrocknet und anschließend unter vermindertem Druck eingedampft. Der Rückstand (450 mg) wurde zur Reinigung und Auftrennung der beiden epimeren Produkte an 30 g Kieselgel unter Verwendung eines 97:3:0.1-Gemisches von Methylenchlorid, Methanol und Ammoniak als Eluierungsmittel chromatographiert. Nach dem Lyophilisieren aus Dioxan und Wasser wurde das weniger polare Epimer (S)-α-[(R)-α-[[(1-Carbamoyl-1-methyläthyl)sulfonyl]methyl]hydrocinnamamido]-N-[1S,2R,3S)-1-(cyclohexylmethyl)-3-cyclopropyl-2,3-dihydroxypropyl]imidazol-4-propionamid als farbloses, amorphes Pulver, MS: 660 (M+H)⁺, und das polarere Epimer (S)-α-[(S)-α-[[(1-Carbamoyl-1-methyläthyl)sulfonyl]methyl]hydrocinnamamido]-N-[(1S,2R,3S)-1-(cyclohexylmethyl)-3-cyclopropyl-2,3-dihydroxypropyl]imidazol-4-propionamid ebenfalls als farbloses, amorphes Pulver, MS: 660 (M+H)⁺, erhalten.

Das als Ausgangsstoff eingesetzte (S)-α-Amino-N-[(1S,2R,3S)-1-(cyclohexylmethyl)-3-cyclopropyl-2,3-dihydroxypropyl]imidazol-4-propionamid wurde wie folgt hergestellt:
(a) Zu einer Lösung der aus 3.94 ml (49 mMol) Cyclopropylbromid und 1.2 g (0.049 Grammatom) Magnesiumspänen in 22 ml Tetrahydrofuran hergestellten Grignardverbindung wurde bei ca. 15° eine Lösung von 3.21 g tert-Butyl (4S,5R)-4-(cyclohexylmethyl)-5-formyl-2,2-dimethyl-3-oxazolidincarbonat (WO 87/05302) in 25 ml Tetrahydrofuran getropft, und das Reaktionsgemisch anschliessend 16 Stunden bei Raumtemperatur unter Argon gerührt. Danach goß man das Reaktionsgemisch auf 40 ml einer eiskalten gesättigten Ammoniumchloridlösung und extrahierte zweimal mit je 50 ml Essigester. Die Essigesterextrakte wurden mit 40 ml eiskalter gesättigter Ammoniumchloridlösung gewaschen, dann vereinigt, über Natriumsulfat getrocknet und unter vermindertem Druck eingedampft. Zur Reinigung wurde der Rückstand (4.33 g) über eine mit Toluol und 1% Triäthylamin aufgezogene Säule von 110 g Kieselgel mit einem 95:5-Gemisch von Toluol und Essigester als Eluierungsmittel chromatographiert. Man erhielt 1.9 g tert-Butyl (4S,5R)-4-(cyclohexylmethyl)-5-[(S)-cyclopropylhydroxymethyl]-2,2-dimethyl-3-oxazolidincarbonat, MS: 368 (M+H)⁺, und 0.5 g tert-Butyl (4S,5R)-4-(cyclohexylmethyl)-5-[(R)-cyclopropylhydroxymethyl]-2,2-dimethyl-3-oxazolidincarbonat, MS: 368 (M+H)⁺, jeweils als farbloses Oel.
(b) 1.42 g (3.86 mMol) tert-Butyl (4S,5R)-4-(cyclohexylmethyl)-5-[(S)-cyclopropylhydroxymethyl]-2,2-dimethyl-3-oxazolidincarbonat, gelöst in 15 ml Methanol und 10 ml Wasser, wurden mit 4 ml 7.5 N Salzsäure versetzt und 3 Stunden bei 50° gerührt. Die Reaktionslösung wurde im Eisbad auf 3° abgekühlt, mit 4 ml 7.5 N Natronlauge tropfenweise versetzt und 1 Stunde nachgerührt. Die erhaltene Suspension wurde unter vermindertem Druck eingedampft, zweimal mit 10 ml Toluol azeotrop entwässert, und der Rückstand dreimal mit 10 ml eines 95:5-Gemisches von Methylenchlorid und Methanol ausgerührt. Der unlösliche Rückstand wurde abfiltriert, und das Filtrat unter vermindertem Druck eingedampft. Das erhaltene Rohprodukt (1.14 g) wurde in 15 ml Aether suspendiert und danach abgesaugt. Man erhielt 0.58 g (1S,2R,3S)-3-Amino-4-cyclohexyl-1-cyclopropyl-1,2-butandiol als farblose Kristalle, Smp. 141-142°.
(c) Ein Gemisch von 343 mg (1.51 mMol) (1S,2R,3S)-3-Amino-4-cyclohexyl-1-cyclopropyl-1,2-butandiol, 995 mg (1.66 mMol) Fmoc-His(Fmoc)-OH, 0.21 ml (1.61 mMol) 4-Aethylmorpholin, 449 mg (3.22 mMol) HOBT und 347 mg (1.81 mMol) EDC in 20 ml Dimethylformamid wurde über Nacht bei Raumtemperatur stehengelassen. Danach wurde das Reaktionsgemisch im Hochvakuum eingedampft, der Rückstand auf ein Gemisch von Eis und 90 ml Natriumbicarbonatlösung gegossen und dreimal mit je 150 ml Essigester extrahiert. Die drei Essigesterextrakte wurden nacheinander mit 70 ml gesättigter Ammoniumchloridlösung, 70 ml 2N Natriumbicarbonatlösung und 70 ml gesättigter Natriumchloridlösung gewaschen, vereinigt, über Magnesiumsulfat getrocknet, filtriert und eingedampft. Das erhaltene Rohprodukt wurde in 60 ml Methylenchlorid und 2 ml Piperidin 3 Stunden bei Raumtemperatur gerührt. Dann wurde das Reaktionsgemisch eingedampft, und der Rückstand mit 50 ml Hexan trituriert und abfiltriert. Das Filtrat wurde mit einem 65:10:1-Gemisch von Methylenchlorid, Methanol und Ammoniak als Eluierungsmittel an 70 g Kieselgel chromatographiert, wobei man 390 mg (S)-α-Amino-N-[(1S,2R,3S)-1-(cyclohexylmethyl)-3-cyclopropyl-2,3-dihydroxypropyl]imidazol-4-propionamid als farblosen Schaum erhielt; MS: 365 (M+H)⁺.
   Die als Ausgangsstoff eingesetzte rac-α-[[(1-Carbamoyl-1-methyläthyl)sulfonyl]methyl]hydrozimtsäure wurde wie folgt hergestellt:
(d) Zu einer Lösung von 3.3 g (27.7 mMol) 2-Mercaptoisobuttersäure [Can.J. Chem. 61(8), 1872] und 6.9 g (27.7 mMol) 2-Benzylacrylsäurebenzylester (EPA 0117429) wurden 8.4 g (55.4 mMol) DBU getropft, wobei die Temperatur des Reaktionsgemisches zwischen 5 und 10° gehalten wurde. Nach beendeter Zugabe wurde weitere 5 Stunden bei 10° gerührt. Anschließend wurde das Reaktionsgemisch unter Eiskühlung mit 22.5 g (36.6 mMol) Kaliummonopersulfat-tripelsalz, suspendiert in 450 ml Wasser, tropfenweise versetzt, wobei die Temperatur unterhalb 10° gehalten wurde. Danach wurde eine Stunde bei der gleichen Temperatur weitergerührt, darin auf 0° abgekühlt und nochmals 22.5 g (36.6 mMol) Kaliummonopersulfat-tripelsalz spatelweise zugegeben. Man ließ langsam auf Raumtemperatur erwärmen und rührte 15 Stunden weiter. Zur Aufarbeitung wurde mit 200 ml Wasser verdünnt und viermal mit je 60 ml Essigester extrahiert. Die vereinigten organischen Extrakte wurden über Natriumsulfat getrocknet und unter vermindertem Druck eingedampft bis das Produkt zu kristallisieren begann. Dann wurden unter Rühren 30 ml Hexan und 20 ml Aether zugefügt, anschließend das ausgefallene Produkt abgesaugt und getrocknet. Es wurden 9.7 g (RS)-2-[[2-[(Benzyloxy)carbonyl]-3-phenylpropyl]sulfonyl]-2-methylpropionsäure als farbloser Festkörper erhalten; MS: 422 (M+NH₄)⁺.
(e) Eine Lösung von 140 g (2.5 mMol) (RS)-2-[[2-[(Benzyloxy)carbonyl]-3-phenylpropyl]sulfonyl]-2-methylpropionsäure und 1.07 ml (12.5 mMol) Oxalylchlorid in 2 ml Tetrahydrofuran wurde 18 Stunden auf 50° erwärmt. Danach wurde die Reaktionslösung unter vermindertem Druck eingedampft. Der Rückstand wurde zweimal in jeweils 30 ml Toluol aufgenommen und wiederum unter vermindertem Druck eingedampft. (RS)-2-[[2-[(Benzyloxy)carbonyl]-3-phenylpropyl]sulfonyl]-2-methylpropionsäurechlorid wurde als gelbliches Oel in quantitativer Ausbeute erhalten und ohne Reinigung und Charakterisierung in die folgende Stufe eingesetzt.
(f) Eine Lösung von 1.05 g (2.5 mMol) rohem (RS)-2-[[2-[(Benzyloxy)-carbonyl]-3-phenylpropyl]sulfonyl]-2-methylpropionsäurechlorid in 29 ml (9.3 mMol) 0.32 M Ammoniak in Tetrahydrofuran wurde 5 Stunden bei Raumtemperatur gerührt. Anschließend wurde die Reaktionslösung mit 40 ml Essigester verdünnt und mit 20 ml 1N Salzsäure extrahiert. Der Essigesterextrakt wurde in der Folge mit 20 ml gesättigter Natriumhydrogencarbonatlösung und 20 ml gesättigter Natriumchloridlösung gewaschen, über Natriumsulfat getrocknet und unter vermindertem Druck eingedampft. Es wurden 0.92 g rac-α-[[(1-Carbamoyl-1-methyläthyl)sulfonyl]methyl]hydrozimtsäurebenzylester als farbloser Festkörper erhalten, MS: 312 (M-Benzyl)⁺.
(g) Eine Suspension von 0.74 g (1.83 mMol) rac-α-[[(1-Carbamoyl-1-methyläthyl)sulfonyl]methyl]hydrozimtsäurebenzylester und 200 mg Palladium/Kohle (5%ig) in 30 ml Methanol wurde unter Normaldruck bei Raumtemperatur 2 Stunden hydriert. Anschließend wurde der Katalysator abfiltriert, und die Lösung unter vermindertem Druck eingedampft. Es wurden 0.54 g rac-α-[[(1-Carbamoyl-1-methyläthyl)sulfonyl]methyl]hydrozimtsäure als farbloser Festkörper erhalten, MS: 314 (M+H)⁺.

### Beispiel 2

In analoger Weise wie in Beispiel 1 beschrieben, wurden die folgenden Verbindungen hergestellt:
- Aus (S)-α-Amino-N-[(1S,2R,3S)-1-(cyclohexylmethyl)-3-cyclopropyl-2,3-dihydroxypropyl]imidazol-4-propionamid und rac-α-[[[1-(Morpholinocarbonyl)-1-methyläthyl]sulfonyl]methyl]hydrozimtsäure das (S)-N-[(1S,2R,3S)-1-(Cyclohexylmethyl)-3-cyclopropyl-2,3-dihydroxypropyl]-α-[(R)-α-[[[1-(morpholinocarbonyl)-1-methyläthyl]sulfonyl]methyl]hydrocinnamamido]imidazol-4-propionamid als farbloser Festkörper, MS: 730 (M+H)⁺, und das epimere (S)-N-[(1S,2R,3S)-1-(Cyclohexylmethyl)-3-cyclopropyl-2,3-dihydroxypropyl]-α-[(S)-α-[[[1-(morpholinocarbonyl)-1-methyläthyl]sulfonyl]methyl]hydrocinnamamido]imidazol-4-propionamid als farbloser Schaum, MS: 730 (M+H)⁺;
- aus (S)-α-Amino-N-[(1S,2R,3S)-1-(cyclohexylmethyl)-3-cyclopropyl-2,3-dihydroxypropyl]imidazol-4-propionamid und (RS)-α-[[[1-(Cyclopropylcarbamoyl)-1-methyläthyl]sulfonyl]methyl]hydrozimtsäure das (S)-N-[(1S,2R,3S)-1-(Cyclohexylmethyl)-3-cyclopropyl-2,3-dihydroxypropyl]-α-[(R)-α-[[[1-(cyclopropylcarbamoyl)-1-methyläthyl]sulfonyl]methyl]hydrocinnamamido]imidazol-4-propionamid, MS: 700 (M+H)⁺, und das epimere (S)-N-[(1S,2R,3S)-1-(Cyclohexylmethyl)-3-cyclopropyl-2,3-dihydroxypropyl]-α-[(S)-α-[[[1-(cyclopropylcarbamoyl)-1-methyläthyl]sulfonyl]methyl]hydrocinnamamido]imidazol-4-propionamid, MS: 700 (M+H)⁺, jeweils als farbloser, amorpher Festkörper;
- aus (S)-α-Amino-N-[(1S,2R,3S)-1-(cyclohexylmethyl)-3-cyclopropyl-2,3-dihydroxypropyl]imidazol-4-propionamid und rac-α-[[[1-Methyl-1-[(2-sulfoäthyl)carbamoyl]äthyl]sulfonyl]methyl]hydrozimtsäure die 2-[2-[[(R)-2-[[(S)-1-[[(1S,2R,3S)-1-(Cyclohexylmethyl)-3-cyclopropyl-2,3-dihydroxypropyl]carbamoyl]-2-imidazol-4-yläthyl]carbamoyl]-3-phenylpropyl]sulfonyl]-2-methylpropionamido]äthansulfonsäure, MS: 768 (M+H)⁺, und die epimere 2-[2-[[(S)-2-[[(S)-1-[[1S,2R,3S)-1-(Cyclohexylmethyl)-3-cyclopropyl-2,3-dihydroxypropyl]carbamoyl]-2-imidazol-4-yläthyl]carbamoyl]-3-phenylpropyl]sulfonyl]-2-methylpropionamido]äthansulfonsäure, MS: 768 (M+H)⁺, jeweils als amorpher Festkörper;
- aus (S)-α-Amino-N-[(1S,2R,3S)-1-(cyclohexylmethyl)-3-cyclopropyl-2,3-dihydroxypropyl]imidazol-4-propionamid und rac-α-[[[1-[(4-Hydroxypiperidino)carbonyl]-1-methyläthyl]sulfonyl]methyl]hydrozimtsäure das (S)-N-[(1S,2R,3S)-1-(Cyclohexylmethyl)-3-cyclopropyl-2,3-dihydroxypropyl]-α-[(R)-α-[[[1-[(4-hydroxypiperidino)carbonyl]-1-methyläthyl]sulfonyl]methyl]hydrocinnamamido]imidazol-4-propionamid, MS: 744 (M+H)⁺, und das epimere (S)-N-[(1S,2R,3S)-1-(Cyclohexylmethyl)-3-cyclopropyl-2,3-dihydroxypropyl]-α-[(S)-α-[[[1-[(4-hydroxypiperidino)carbonyl]-1-methyläthyl]sulfonyl]methyl]lhydrocinnamamido]imidazol-4-propionamid, MS: 744 (M+H)⁺, jeweils als amorpher Festkörper;
- aus (S)-α-Amino-N-[(1S,2R,3S)-1-(cyclohexylmethyl)-3-cyclopropyl-2,3-dihydroxypropyl]imidazol-4-propionamid und rac-α-[[[1-[(1-Benzyl-4-piperidinyl)carbamoyl]-1-methyläthyl]sulfonyl]methyl]hydrozimtsäure das (S)-α-[(S oder R)-α-[[[1-[(1-Benzyl-4-piperidinyl)carbamoyl]-1-methyläthyl]sulfonyl]methyl]hydrocinnamamido]-N-[(1S,2R,3S)-1-(cyclohexylmethyl)-3-cyclopropyl-2,3-dihydroxypropyl]imidazol-4-propionamid, MS: 833 (M+H)⁺, und das epimere (S)-α-[(R oder S)-α-[[[1-[(1-Benzyl-4-piperidinyl)carbamoyl]-1-methyläthyl]sulfonyl]methyl]hydrocinnamamido]-N-[(1S,2R,3S)-1-(cyclohexylmethyl)-3-cyclopropyl-2,3-dihydroxypropyl]imidazol-4-propionamid, MS: 833 (M+H)⁺, jeweils als farbloser Festkörper;
- aus (S)-α-Amino-N-[(1S,2R,3S)-1-(cyclohexylmethyl)-3-cyclopropyl-2,3-dihydroxypropyl]imidazol-4-propionamid und rac-α-[[(1-[[4-(2-Hydroxyäthyl)-1-piperazinyl]carbonyl]-1-methyläthyl]sulfonyl]methyl]hydrozimtsäure das (S)-N-[(1S,2R,3S)-1-(Cyclohexylmethyl)-3-cyclopropyl-2,3-dihydroxypropyl]-α-[(R oder S)-α-[[[-[4-(2-hydroxyäthyl)-1-piperazinyl]carbonyl]-1-methyläthyl]sulfonyl]methyl]hydrocinnamamido]imidazol-4-propionamid, MS: 773 (M+H)⁺, und das epimere (S)-N-[(1S,2R,3S)-1-(Cyclohexylmethyl)-3-cyclopropyl-2,3-dihydroxypropyl]-α-[(S oder R)-α-[[[1-[4-(2-hydroxyäthyl)-1-piperazinyl]carbonyl]-1-methyläthyl]sulfonyl]methyl]hydrocinnamamido]imidazol-4-propionamid, MS: 773 (M+H)⁺ jeweils als farbloser Festkörper;
- aus (S)-α-Amino-N-[(1S,2R,3S)-1-(cyclohexylmethyl)-3-cyclopropyl-2,3-dihydroxypropyl]imidazol-4-propionamid und (RS)-α-[[[1-[[(RS)-3-Hydroxy-1-pyrrolidinyl]carbonyl]-1-methyläthyl]sulfonyl]methyl]hydrozimtsäure das (S)-N-[(1S,2R,3S)-1-(Cyclohexylmethyl)-3-cyclopropyl-2,3-dihydroxypropyl]-α-[(R oder S)-α-[[[1-[[(RS)-3-hydroxy-1-pyrrolidinyl]carbonyl]-1-methyläthyl]sulfonyl]methyl]hydrocinnamamido]imidazol-4-propionamid, MS: 730 (M+H)⁺, und das epimere (S)-N-[(1S,2R,3S)-1-(Cyclohexylmethyl)-3-cyclopropyl-2,3-dihydroxypropyl]-α-[(S oder R)-α-[[[1-[[(RS)-3-hydroxy-1-pyrrolidinyl]carbonyl]-1-methyläthyl]sulfonyl]methyl]hydrocinnamamido]imidazol-4-propionamid, MS: 730 (M+H)⁺, jeweils als farbloser Festkörper;
- aus (S)-α-Amino-N-[(1S,2R,3S)-1-(cyclohexylmethyl)-3-cyclopropyl-2,3-dihydroxypropyl]imidazol-4-propionamid und (RS)-α-[[[1-[[3RS, 4RS)-3,4-Dihydroxy-1-pyrrolidinyl]carbonyl]-1-methyläthyl]sulfonyl]methyl]hydrozimtsäure das (S)-N-[(1S,2R,3S)-1-(Cyclohexylmethyl)-3-cyclopropyl-2,3-dihydroxypropyl]-α-[(RS)-α-[1-[[[[(all-RS)-3,4-dihydroxy-1-pyrrolidinyl]carbonyl]methyl]sulfonyl]-1-methyläthyl]hydrocinnamamido]imidazol-4-propionamid, MS: 746 (M+H)⁺, als amorpher Festkörper;
- aus (S)-α-Amino-N-[(1S,2R,3S)-1-(cyclohexylmethyl)-3-cyclopropyl-2,3-dihydroxypropyl]imidazol-4-propionamid und (RS)-α-[[[1-[(R)-2-(Hydroxymethyl)-1-pyrrolidinyl]carbamoyl]-1-methyläthyl]sulfonyl]methyl]hydrozimtsäure das (S)-N-[(1S,2R,3S)-1-(Cyclohexylmethyl)-3-cyclopropyl-2,3-dihydroxypropyl]-α-[(S oder R)-α-[[[1-[[(R)-2-(hydroxymethyl)-1-pyrrolidinyl]carbonyl]-1-methyläthyl]sulfonyl]methyl]hydrocinnamamido]imidazol-4-propionamid, MS: 744 (M+H)⁺, als farbloser Schaum und das epimere (S)-N-[(1S,2R,3S)-1-(Cyclohexylmethyl)-3-cyclopropyl-2,3-dihydroxypropyl]-α-[(R oder S)-α-[[[1-[[(R)-2-(hydroxymethyl)-1-pyrrolidinyl]carbonyl]-1-methyläthyl]sulfonyl]methyl]hydrocinnamamido]imidazol-4-propionamid, MS: 744 (M+H)⁺, jeweils als farbloser Festkörper;
- aus (S)-α-Amino-N-[(1S,2R,3S)-1-(cyclohexylmethyl)-3-cyclopropyl-2,3-dihydroxypropyl]imidazol-4-propionamid und rac-α-[[[1-Methyl-1-[(2-pyridylmethyl)carbamoyl]äthyl]sulfonyl]methyl]hydrozimtsäure das (S)-α-[(R oder S)-α-[[[1-[2-Pyridylmethyl)carbamoyl]-1-methyläthyl]sulfonyl]methyl]hydrocinnamamido]-N-[(1S,2R,3S)-1-(cyclohexylmethyl)-3-cyclopropyl-2,3-dihydroxypropyl]imidazol-4-propionamid, MS: 751 (M+H)⁺, und das epimere (S)-α-[(S oder R)-α-[[[1-[(2-Pyridylmethyl)carbamoyl]-1-methyläthyl]sulfonyl]methyl]hydrocinnamamido]-N-[(1S,2R,3S)-1-(cyclohexylmethyl)-3-cyclopropyl-2,3-dihydroxypropyl]imidazol-4-propionamid, MS: 751 (M+H)⁺, jeweils als farbloser Festkörper;
- aus (S)-α-Amino-N-[(1S,2R,3S)-1-(cyclohexylmethyl)-3-cyclopropyl-2,3-dihydroxypropyl]imidazol-4-propionamid und (RS)-α-[[[1-[[1-(Methoxycarbonyl)-1-methyläthyl]carbamoyl]-1-methyläthyl]sulfonyl]methyl]hydrozimtsäure das Methyl 2-[2-[[(R oder S)-2-[[(S)-1-[[(1S,2R,3S)-1-(cyclohexylmethyl)-3-cyclopropyl-2,3-dihydroxypropyl]carbamoyl]-2-imidazol-4-yläthyl]carbamoyl]-3-phenylpropyl]sulfonyl]-2-methylpropionamido]-2-methylpropionat, MS: 760 (M+H)⁺, und das epimere Methyl 2-[2-[[(S oder R)-2-[[(S)-1-[[(1S,2R,3S)-1-(cyclohexylmethyl)-3-cyclopropyl-2,3-dihydroxypropyl]carbamoyl]-2-imidazol-4-yläthyl]carbamoyl]-3-phenylpropyl]sulfonyl]-2-methylpropionamido]-2-methylpropionat. MS: 760 (M+H)⁺, jeweils als farbloser Festkörper;
- aus (S)-α-Amino-N-[(1S,2R,3S)-1-(cyclohexylmethyl)-3-cyclopropyl-2,3-dihydroxypropyl]imidazol-4-propionamid und rac-α-[[[1-[bis(2-Methoxyäthyl)carbamoyl]-1-methyläthyl]sulfonyl]methyl]hydrozimtsäure das (S)-α-[(S oder R)-α-[[[1-[bis(2-Methoxyäthyl)carbamoyl]-1-methyläthyl]sulfonyl]methyl]hydrocinnamamido]-N-[(1S,2R,3S)-1-(cyclohexylmethyl)-3-cyclopropyl-2,3-dihydroxypropyl]imidazol-4-propionamid, MS: 776 (M+H)⁺, und das epimere (S)-α-[(R oder S)-α-[[[1-[bis(2-Methoxyäthyl)cabamoyl]-1-methyläthyl]sulfonyl]methyl]hydrocinnamamido]-N-[(1S,2R,3S)-1-(cyclohexylmethyl)-3-cyclopropyl-2,3-dihydroxypropyl]imidazol-4-propionamid, MS: 776 (M+H)⁺, jeweils als farbloser Festkörper;
- aus (S)-α-Amino-N-[(1S,2R,3S)-1-(cyclohexylmethyl)-3-cyclopropyl-2,3-dihydroxypropyl]imidazol-4-propionamid und rac-α-[[[1-[[4-(tert-Butoxycarbonyl)-1-piperazinyl]carbonyl]-1-methyläthyl]sulfonyl]methyl]hydrozimtsäure das tert-Butyl 4-[2-[[(S oder R)-2-[[(S)-1-[[(1S,2R,3S)-1-(cyclohexylmethyl)-3-cyclopropyl-2,3-dihydroxypropyl]carbamoyl]-2-imidazol-4-yläthyl]carbamoyl]-3-phenylpropyl]sulfonyl]-2-methylpropionyl]-1-piperazincarbonat, MS: 829 (M+H)⁺, und das epimere tert-Butyl 4-[2-[[(S)-2-[[(R oder S)-1-[[(1S,2R,3S)-1-(cyclohexylmethyl)-3-cyclopropyl-2,3-dihydroxypropyl]carbamoyl]-2-imidazol-4-yläthyl]carbamoyl]-3-phenylpropyl]sulfonyl]-2-methylpropionyl]-1-piperazincarbonat, MS: 829 (M+H)⁺, jeweils als farbloser, amorpher Festkörper.

Die als Ausgangsstoffe eingesetzten Säuren wurden wie folgt hergestellt:

### rac-α-[[[1-(Morpholinocarbonyl)-1-methyläthyl]sulfonyl]methyl]hydrozimtsäure:

(a) Zu einer Lösung von 0.97 g (2.3 mMol) rohem (RS)-2-[[2-[(Benzyloxy)carbonyl]-3-phenylpropyl]sulfonyl]-2-methylpropionsäurechlorid in 1 ml Tetrahydrofuran wurden unter Eiskühlung 0.64 ml (7.3 mMol) Morpholin, gelöst in 1 ml Tetrahydrofuran, getropft. Man ließ das Reaktionsgemisch innerhalb 1 Stunde auf Raumtemperatur erwärmen, wobei sich ein Niederschlag bildete. Anschließend wurde mit 20 ml Essigester verdünnt, und die Lösung mit 1N Salzsäure auf pH 2 gestellt. Die Essigesterphase wurde mit 20 ml gesättigter Natriumhydrogencarbonatlösung und 20 ml gesättigter Natriumchloridlösung gewaschen, über Natriumsulfat getrocknet und unter vermindertem Druck eingedampft. Das Rohprodukt wurde zur Reinigung mit einem 3:1-Gemisch von Toluol und Essigester als Eluierungsmittel an 30 g Kieselgel chromatographiert. Es wurden 0.98 g rac-α-[[-Methyl-1-(morpholinocarbonyl)äthyl]sulfonyl]methyl]hydrozimtsäurebenzylester als farbloses Oel erhalten, MS: 473 (M)⁺.
(b) In analoger Weise wie in Beispiel 1, Absatz (g) beschrieben, wurde durch katalytische Hydrierung von rac-α-[[1-Methyl-1-(morpholinocarbonyl)äthyl]sulfonyl]methyl]hydrozimtsäurebenzylester die rac-α-[[[1-(Morpholinocarbonyl)-1-methyläthyl]sulfonyl]methyl]hydrozimtsäure als farbloser Schaum erhalten, MS: 383 (M)⁺.

### rac-α-[[[1-(Cyclopropylcarbamoyl)-1-methyläthyl]sulfonyl]methyl]hydrozimtsäure:

(c) Eine Lösung von 1.07 g (2.5 mMol) (RS)-2-[[2-[(Benzyloxy)carbonyl]-3-phenylpropyl]sulfonyl]-2-methylpropionsäurechlorid und 0.42 g (7.4 mMol) Cyclopropylamin in 20 ml Pyridin wurde 2 Stunden bei Raumtemperatur gerührt. Danach wurde die Reaktionslösung unter vermindertem Druck eingedampft, und der Rückstand unter Verwendung eines 99:1-Gemisches von Methylenchlorid und Methanol als Eluierungsmittel an 30 g Kieselgel chromatographiert. Es wurden 0.96 g rac-α-[[[1-(Cyclopropylcarbamoyl)-1-methyläthyl]sulfonyl]methyl]hydrozimtsäurebenzylester als gelblicher Festkörper erhalten, MS: 444 (M+H)⁺.
(d) In analoger Weise wie in Beispiel 1, Absatz (g) beschrieben, wurde durch katalytische Hydrierung von rac-α-[[[1-(Cyclopropylcarbamoyl)-1-methyläthyl]sulfonyl]methyl]hydrozimtsäurebenzylester die rac-α-[[[1-(Cyclopropylcarbamoyl)-1-methyläthyl]sulfonyl]methyl]hydrozimtsäure als farbloser Festkörper erhalten, MS: 354 (M+H)⁺.

In analoger Weise wie in den obigen Absätzen (c) und (d) beschrieben, wurden die folgenden Säuren hergestellt:
(e) Aus (RS)-2-[[2-[(Benzyloxy)carbonyl]-3-phenylpropyl]sulfonyl]-2-methylpropionsäurechlorid und 2-Aminoäthansulfonsäure in Pyridin der rac-α-[[[1-Methyl-1-[(2-sulfoäthyl)carbamoyl]äthyl]sulfonyl]methyl]hydrozimtsäurebenzylester, MS: 510 (M-H)⁻; als farbloser Schaum, dessen katalytische Hydrierung rac-α-[[[1-Methyl-1-[(2-sulfoäthyl)carbamoyl]äthyl]sulfonyl]methyl]hydrozimtsäure, MS: 460 (M+K)⁺, als farblosen Schaum lieferte;
(f) aus (RS)-2-[[2-[(Benzyloxy)carbonyl]-3-phenylpropyl]sulfonyl]-2-methylpropionsäurechlorid und 4-Hydroxypiperidin in Pyridin der rac-α-[[[1-[(4-Hydroxypiperidino)carbonyl]-1-methyläthyl]sulfonyl]methyl]hydrozimtsäurebenzylester, MS: 488 (M+H)⁺, als amorpher Festkörper, dessen katalytische Hydrierung rac-α-[[[1-[(4-Hydroxypiperidino)carbonyl]-1-methyläthyl]sulfonyl]methyl]hydrozimtsäure, MS: 397 (M)⁺, als farblosen Schaum lieferte.
   In analoger Weise wie in den obigen Absätzen (a) und (b) beschrieben, wurden die folgenden Säuren hergestellt:
(g) Aus (RS)-2-[[2-[(Benzyloxy)carbonyl]-3-phenylpropyl]sulfonyl]-2-methylpropionsäurechlorid und 4-Amino-N-benzylpiperidin der rac-α-[[[1-[(1-Benzyl-4-piperidinyl)carbamoyl]-1-methyläthyl]sulfonyl]methyl]hydrozimtsäurebenzylester, MS: 577 (M+H)⁺, als farbloses Oel, dessen katalytische Hydrierung rac-α-[[[1-[(1-Benzyl-4-piperidinyl)carbamoyl]-1-methyläthyl]sulfonyl]methyl]hydrozimtsäure als farblosen Schaum lieferte, der direkt in die nächste Stufe eingesetzt wurde;
(h) aus (RS)-2-[[2-[(Benzyloxy)carbonyl]-3-phenylpropyl]sulfonyl]-2-methylpropionsäurechlorid und N-(2-Hydroxyäthyl)piperazin der rac-α-[[(1-[[4-(2-Hydroxyäthyl)-1-piperazinyl]carbonyl]-1- methyläthyl]sulfonyl]methyl]hydrozimtsäurebenzylester, MS: 517 (M+H)⁺, als farbloser, amorpher Festkörper, dessen katalytische Hydrierung rac-α-[[(1-[[4-(2-Hydroxyäthyl)-1-piperazinyl]carbonyl]-1-methyläthyl]sulfonyl]methyl]hydrozimtsäure als farblosen Schaum lieferte, der direkt in die nächste Stufe eingesetzt wurde;
(i) aus (RS)-2-[[2-[(Benzyloxy)carbonyl]-3-phenylpropyl]sulfonyl]-2-methylpropionsäurechlorid und 3-Hydroxypyrrolidin der (RS)-α-[[[1-[[(RS)-3-Hydroxy-1-pyrrolidinyl]carbonyl]-1-methyläthyl]sulfonyl]methyl]hydrozimtsäurebenzylester, MS: 474 (M+H)⁺, als farbloses Oel, dessen katalytische Hydrierung (RS)-α-[[[1-[[(RS)-3-Hydroxy-1-pyrrolidinyl]carbonyl]-1-methyläthyl]sulfonyl]methyl]hydrozimtsäure als farbloses Oel lieferte, das direkt in die nächste Stufe eingesetzt wurde;
(j) aus (RS)-2-[[2-[(Benzyloxy)carbonyl]-3-phenylpropyl]sulfonyl]-2-methylpropionsäurechlorid und (R)-(-)-2-(Hydroxymethyl)pyrrolidin der (RS)-α-[[[1-[[(R)-2-(Hydroxymethyl)-1-pyrrolidinyl]carbonyl]-1-methyläthyl]sulfonyl]methyl]hydrozimtsäurebenzylester, MS: 488 (M+H)⁺, als farbloses Oel, dessen katalytische Hydrierung (RS)-α-[[[1-[(R)-2-(Hydroxymethyl)-1-pyrrolidinyl]carbamoyl]-1-methyläthyl]sulfonyl]methyl]hydrozimtsäure als farbloses Oel lieferte, das direkt in die nächste Stufe eingesetzt wurde;
(k) aus (RS)-2-[[2-[(Benzyloxy)carbonyl]-3-phenylpropyl]sulfonyl]-2-methylpropionsäurechlorid und 2-(Aminomethyl)pyridin der rac-α-[[[1-Methyl-1-[(2-pyridylmethyl)carbamoyl]äthyl]sulfonyl]methyl]hydrozimtsäurebenzylester, MS : 495 (M+H)⁺, als farbloses Oel, dessen katalytische Hydrierung rac-α-[[[1-Methyl-1-[(2-pyridylmethyl)carbamoyl]äthyl]sulfonyl]methyl]hydrozimtsäure als farblosen Schaum lieferte, der direkt in die nächste Stufe eingesetzt wurde;
(l) aus (RS)-2-[[2-[(Benzyloxy)carbonyl]-3-phenylpropyl]sulfonyl]-2-methylpropionsäurechlorid und 2-Aminoisobuttersäuremethylester der (RS)-α-[[[1-[[1-(Methoxycarbonyl)-1-methyläthyl]carbamoyl]-1-methyläthyl]sulfonyl]methyl]hydrozimtsäurebenzylester, MS: 504 (M+H)⁺, als farbloser Festkörper, dessen katalytrische Hydrierung (RS)-α-[[[1-[[1-(Methoxycarbonyl)-1-methyläthyl]carbamoyl]-1-methyläthyl]sulfonyl]methyl]hydrozimtsäure als farblosen Schaum lieferte, der direkt in die nächste Stufe eingesetzt wurde;
(m) aus (RS)-2-[[2-[(Benzyloxy)carbonyl]-3-phenylpropyl]sulfonyl]-2-methylpropionsäurechlorid und bis(2-Methoxyäthyl)amin der rac-α-[[[1-[bis(2-Methoxyäthyl)carbamoyl]-1-methyläthyl]sulfonyl]methyl]hydrozimtsäurebenzylester, MS: 520 (M+H)⁺, als farbloses Oel, dessen katalytische Hydrierung rac-α-[[[1-[bis(2-Methoxyäthyl)carbamoyl]-1-methyläthyl]sulfonyl]methyl]hydrozimtsäure als farbloses Oel lieferte, das direkt in die nächste Stufe eingesetzt wurde;
(n) aus (RS)-2-[[2-[(Benzyloxy)carbonyl]-3-phenylpropyl]sulfonyl]-2-methylpropionsäurechlorid und 1-Piperazincarbonsäure-tert-butylester [J.Org.Chem. 48(5), 661] der rac-α-[[[1-[[4-(tert-Butoxycarbonyl)-1-piperazinyl]carbonyl]-1-methyläthyl]sulfonyl]methyl]hydrozimtsäurebenzylester, MS: 595 (M+Na)⁺, als farbloser Festkörper, dessen katalytische Hydrierung rac-α-[[[1-[[4-(tert-Butoxycarbonyl)-1-piperazinyl]carbonyl]-1-methyläthyl]sulfonyl]methyl]hydrozimtsäure, MS: 426 (M-C₄H₈)⁺, als amorphen Festkörper lieferte.

### (RS)-α-[[[1-[[(3RS, 4RS)-3,4-Dihydroxy-1-pyrrolidinyl]carbonyl]-1-methyläthyl]sulfonyl]methyl]hydrozimtsäure:

(o) In analoger Weise wie im obigen Absatz (a) beschrieben, wurde aus (RS)-2-[[2-[(Benzyloxy)carbonyl]-3-phenylpropyl]sulfonyl]-2-methylpropionsäurechlorid und 3-Pyrrolin der rac-α-[[[1-Methy-1-(3-pyrrolin-1-ylcarbonyl)äthyl]sulfonyl]methyl]hydrozimtsäurebenzylester, MS: 456 (M+H)⁺, als farbloses Oel erhalten.
(p) Zu einer Lösung von 935 mg (2.05 mMol) rac-α-[[[1-Methyl-1-(3-pyrrolin-1-ylcarbonyl)äthyl]sulfonyl]methyl]hydrozimtsäurebenzylester in 30 ml Aceton und 10 ml Wasser wurden bei Raumtemperatur 832 mg (6.16 mMol) 4-Methylmorpholin-4-oxid-monohydrat und 10 ml einer Osmiumtetroxidlösung [1.0 g Osmiumtetroxid und 1 ml tert-Butylhydroperoxid (70% ig in Wasser) in 199 md tert-Butanol] gegeben. Die Reaktionsmischung wurde drei Tage bei Raumtemperatur gerührt. Anschließend wurde das Lösungsmittel unter vermindertem Druck abgedampft, der Rückstand in 80 ml eiskalte 10 %ige Zitronensäurelösung gegossen und mit 240 ml Essigester extrahiert. Die organischen Extrakte wurden zweimal mit je 80 ml Wasser gewaschen, und die vereinigten wäßrigen Phasen zweimal mit je 80 ml Essigester nachextrahiert. Die vereinigten Essigesterextrakte wurden über Magnesiumsulfat getrocknet und unter vermindertem Druck eingedampft. Das Rohprodukt (1.1 g) wurde zur Reinigung mit einem 1:4-Gemisch von Toluol und Essigester als Eluierungsmittel an 80 g Kieselgel chromatographiert. Es wurden 750 mg (RS)-α-[[[1-[[(3RS,4RS)-3,4-Dihydroxy-1-pyrrolidinyl]carbonyl]-1-methyläthyl]sulfonyl]methyl]hydrozimtsäurebenzylester als farbloses Oel erhalten, MS: 490 (M+H)⁺.
(q) Die katalytische Hydrierung von (RS)-α-[[[1-[[(3RS,4RS)-3,4-Dihydroxy-1-pyrrolidinyl]carbonyl]-1-methyläthyl]sulfonyl]methyl]hydrozimtsäurebenzylester in analoger Weise wie im obigen Absatz (b) beschrieben, ergab (RS)-α-[[[1-[[3RS, 4RS)-3,4-Dihydroxy-1-pyrrolidinyl]carbonyl]-1-methyläthyl]sulfonyl]methyl]hydrozimtsäure als farbloses Oel, das direkt in die nächste Stufe eingesetzt wurde.

### Referenzbeispiel 2a

Eine Lösung von 140 mg (0.17 mMol) tert-Butyl [(R)-2-[[(S oder R)-2-[[(S)-1-[[(1S,2R,3S)-1-(cyclohexylmethyl)-3-cyclopropyl-2,3-dihydroxypropyl]carbamoyl]-2-imidazol-4-yläthyl]carbamoyl]-3-phenylpropyl]sulfonyl]-2-(morpholinocarbonyl)äthyl]carbamat in 5 ml 2N Salzsäure und 5 ml Methanol wurde 3 Stunden bei 50° gerührt. Anschließend wurde die Reaktionslösung in 50 ml 1N Natriumhydrogencarbonatlösung gegossen und zweimal mit je 100 ml Essigester extrahiert. Die organischen Extrakte wurden mit 50 ml Wasser gewaschen, danach vereinigt, über Magnesiumsulfat getrocknet und unter vermindertem Druck eingedampft Es wurden 106 mg (S)-α-[(S oder R)-α-[[[(R)-2-Amino-2-(morpholinocarbonyl)äthyl]sulfonyl]methyl]hydrocinnamamido]-N-[(1S,2R,3S)-1-(cyclohexylmethyl)-3-cyclopropyl-2,3-dihydroxypropyl]imidazol-4-propionamid als farbloser Festkörper erhalten, MS: 731 (M+H)⁺.

### Beispiel 3

In analoger Weise wie in Beispiel 1 beschrieben, wurden die folgenden Verbindungen hergestellt:
- Aus (S)-α-Amino-N-[(1S,2R,3S)-1-(cyclohexylmethyl)-3-cyclopropyl-2,3-dihydroxypropyl]imidazol-4-propionamid und (S)-α-[[[1-[(2-Hydroxyäthyl)carbamoyl]-1-methyläthyl]sulfonyl]methyl]hydrozimtsäure das (S)-N-[(1S,2R,3S)-1-(Cyclohexylmethyl)-3-cyclopropyl-2,3-dihydroxypropyl]-α-[(S)-α-[[[1-[(2-hydroxyäthyl)carbamoyl]-1-methyläthyl]sulfonyl]methyl]hydrocinnamamido]imidazol-4-propionamid als farbloser, amorpher Festkörper, MS: 704 (M+H)⁺;
- aus (S)-α-Amino-N-[(1S,2R,3S)-1-(cyclohexylmethyl)-3-cyclopropyl-2,3-dihydroxypropyl]imidazol-4-propionamid und (S)-α-[[[1-(2-Hydroxy-1-(hydroxymethyl)-1-methyläthyl]carbamoyl]-1-methyläthyl]sulfonyl]methyl]hydrozimtsäure das (S)-N-[(1S,2R,3S)-1-(Cyclohexylmethyl)-3-cyclopropyl-2,3-dihydroxypropyl]-α-[(S)-α-[[[1-[[2-hydroxy-1-(hydroxymethyl)-1-methyläthyl]carbamoyl]-1-methyläthyl]sulfonyl]methyl]hydrocinnamamido]imidazol-4-propionamid als farbloser, amorpher Festkörper, MS: 748 (M+H)⁺;
- aus (S)-α-Amino-N-[(1S,2R,3S)-1-(cyclohexylmethyl)-3-cyclopropyl-2,3-dihydroxypropyl]imidazol-4-propionamid und 2-[2-[[(S)-2-Carboxy-3-phenylpropyl]sulfonyl]-2-methylpropionamido]-2-deoxy-D-glucopyranose die 2-[2-[[(S)-2-[[(S)-1-[[(1S,2R,3S)-1-(Cyclohexylmethyl)-3-cyclopropyl-2,3-dihydroxypropyl]carbamoyl]-2-imidazol-4-yläthyl]carbamoyl]-3-phenylpropyl]sulfonyl]-2-methylpropionamido]-2-deoxy-D-glucopyranose als farbloser, amorpher Festkörper, MS: 822 (M+H)⁺.

Die als Ausgangsstoffe eingesetzten Hydrozimtsäurederivate wurden wie folgt hergestellt:

### (S)-α-[[[1-[(2-Hydroxyäthyl)carbamoyl]-1-methyläthyl[sulfonyl]methyl]hydrozimtsäure:

(a) In analoger Weise wie in Beispiel 1, Absatz (d) beschrieben, wurde durch Addition von 2-Mercaptoisobuttersäure an 2-Benzylacrylsäureäthylester (EPA 0236734) und nachfolgende Oxidation des Thioäthers die rac-2-[[2-(Aethoxycarbonyl)-3-phenylpropyl]sulfonyl]-2-methylpropionsäure als farbloses Oel erhalten, MS: 342 (M)⁺.
(b) In analoger Weise wie in Beispiel 1 beschrieben, wurde durch Kondensation von rac-2-[[2-(Aethoxycarbonyl)-3-phenylpropyl]sulfonyl]-2-methylpropionsäure und Aethanolamin die rac-α-[[[1-[(2-Hydroxyäthyl)carbamoyl]-1-methyläthyl]sulfonyl]methyl]hydrozimtsäure als farbloses Oel erhalten, MS: 386 (M+H)⁺.
(c) Zu einem Gemisch von 0.86 g (2.23 mMol) rac-α-[[[1-[(2-Hydroxyäthyl)carbamoyl]-1-methyläthyl]sulfonyl]methyl]hydrozimtsäure und 200 mg α-Chymotrypsin in 5 ml Aethanol und 100 ml Wasser wurde unter Rühren 0.1N Natronlauge so zugetropft, daß der pH-Wert bei 7.5 gehalten wurde. Nachdem keine Natronlauge mehr verbraucht wurde, erfolgte die Aufarbeitung des Reaktionsgemisches, indem man auf pH 8 stellte und zweimal mit je 40 ml Aether extrahierte. Die vereinigten organischen Extrakte wurden mit 15 ml gesättigter Natriumhydrogencarbonatlösung gewaschen, über Natriumsulfat getrocknet und unter vermindertem Druck eingedampft. Es wurde der (R)-α-[[[1-[(2-Hydroxyäthyl)carbamoyl]-1-methyläthyl]sulfonyl]methyl]hydrozimtsäureäthylester als gelbliches Oel erhalten.
   Die wäßrige Phase und die Natriumhydrogencarbonatphase wurden vereinigt, mit 1N Salzsäure auf pH 3 gestellt und zweimal mit je 40 ml Essigester extrahiert. Die vereinigten Essigesterextrakte wurden über Natriumsulfat getrocknet und unter vermindertem Druck eingedampft. Es wurden 298 mg (S)-α-[[[1-[(2-Hydroxyäthyl)carbamoyl]-1-methyläthyl]sulfonyl]methyl]hydrozimtsäure als farbloser Festkörper erhalten, MS: 358 (M+H)⁺.

### (S)-α-[[[1-(2-Hydroxy-1-(hydroxymethyl)-1-methyläthyl]carbamoyl]-1-methyläthyl]sulfonyl]methyl]hydrozimtsäure:

(d) In analoger Weise wie in Beispiel 1 beschrieben, wurde durch Kondensation von rac-2-[[2-(Aethoxycarbonyl)-3-phenylpropyl]sulfonyl]-2-methylpropionsäure und 2-Amino-2-methyl-1,3-propandiol der (RS)-α-[[[1-(2-Hydroxy-1-(hydroxymethyl)-1-methyläthyl]carbamoyl]-1-methyläthyl]sulfonyl]methyl]hydrozimtsäureäthylester als farbloses Oel , MS: 430 (M+H)⁺, erhalten. Die nachfolgende enzymatische Hydrolyse mittels α-Chymotrypsin analog zum obigen Absatz (c) lieferte (S)-α-[[[1-(2-Hydroxy-1-(hydroxymethyl)-1-methyläthyl]carbamoyl]-1-methyläthyl]sulfonyl]methyl]hydrozimtsäure als farblosen Schaum, MS: 402 (M+H)⁺.

### 2-[2-[[(S)-2-Carboxy-3-phenylpropyl]sulfonyl]-2-methylpropionamido]-2-deoxy-D-glucopyranose:

(e) In analoger Weise wie in Beispiel 1 beschrieben, wurde durch Kondensation von rac-2-[[2-(Aethoxycarbonyl)-3-phenylpropyl]sulfonyl]-2-methylpropionsäure und Glucosamin die 2-Deoxy-2-[2-[[(RS)-2-(äthoxycarbonyl)-3-phenylpropyl]sulfonyl]-2-methylpropionamido]-D-glucopyranose als farbloser, amorpher Festkörper, MS: 504 (M+H)⁺, erhalten. Die nachfolgende enzymatische Hydrolyse analog zu Absatz (c) oben lieferte die 2-[2-[[(S)-2-Carboxy-3-phenylpropyl]sulfonyl]-2-methylpropionamido]-2-deoxy-D-glucopyranose als farblosen, amorphen Festkörper, MS: 498 (M+Na)⁺.

### Beispiel 4

In analoger Weise wie in Beispiel 1 beschrieben, wurden die folgenden Verbindungen hergestellt:
- Aus (S)-α-[[[1-(Morpholinocarbonyl)-1-methyläthyl]sulfonyl]methyl]hydrozimtsäure und (S)-2-Amino-N-[(1S,2R,3S)-1-(cyclohexylmethyl)-3-cyclopropyl-2,3-dihydroxypropyl]hexanamid das (S)-N-[(S)-1-[[(1S,2R,3S)-1-(Cyclohexylmethyl)-3-cyclopropyl-2,3-dihydroxypropyl]carbamoyl]pentyl]-α-[[[1-methyl-1-(morpholinocarbonyl)äthyl]sulfonyl]methyl]hydrozimtsäureamid als farbloser Schaum, MS: 706 (M+H)⁺;
- aus (S)-α-[[[1-(Morpholinocarbonyl)-1-methyläthyl]sulfonyl]methyl]hydrozimtsäure und (R)-2-Amino-N-[(1S,2R,3S)-1-(cyclohexylmethyl)-3-cyclopropyl-2,3-dihydroxypropyl]-3-(methylthio)propionamid das (S)-N-[(R)-[[(1S,2R,3S)-1-(Cyclohexylmethyl)-3-cyclopropyl-2,3-dihydroxypropyl]carbamoyl]-2-(methylthio)äthyl]-α-[[[1-(morpholinocarbonyl)-1-methyläthyl]sulfonyl]methyl]hydrozimtsäureamid als farbloser Festkörper, MS: 710 (M+H)⁺.

Die als Ausgangsstoff eingesetzte (S)-α-[[[1-(Morpholinocarbonyl)-1-methyläthyl]sulfonyl]methyl]hydrozimtsäure wurde wie folgt hergestellt:
(a) In analoger Weise wie in Beispiel 1, Absätze (e) und (f) beschrieben, wurde duch Kondensation von rac-2-[[2-(Aethoxycarbonyl)-3-phenylpropyl]sulfonyl]-2-methylpropionsäure und Morpholin der rac-α-[[[1-(Morpholinocarbonyl)-1-methyläthyl]sulfonyl]methyl]hydrozimtsäureäthylester als farbloser Schaum erhalten, MS: 411 (M)⁺. Dessen enzymatische Hydrolyse mittels α-Chymotrypsin, analog Beispiel 3, Absatz (c), lieferte (S)-α-[[[1-(Morpholinocarbonyl)-1-methyläthyl]sulfonyl]methyl]hydrozimtsäure als farblosen Schaum, MS: 383 (M)⁺.

Die als Ausgangsstoffe eingesetzten Amine wurden wie folgt hergestellt:

### (S)-2-Amino-N-[(1S,2R,3S)-1-(cyclohexylmethyl)-3-cyclopropyl-2,3-dihydroxypropyl]hexanamid:

(b) Analog zu dem in Beispiel 1, Absatz (c) beschriebenen Verfahren wurde durch Kondensation von (1S,2R,3S)-3-Amino-4-cyclohexyl-1-cyclopropyl-1,2-butandiol und Fmoc-Norleucin das 9H-Fluoren-9-ylmethyl [(S)-1-[[(1S,2R,3S)-1-(cyclohexylmethyl)-3-cyclopropyl-2,3- dihydroxypropyl]carbamoyl]pentyl]carbamat erhalten, das nach Umsetzung mit Piperidin in Methylenchlorid das (S)-2-Amino-N-[(1S,2R,3S)-1-(cyclohexylmethyl)-3-cyclopropyl-2,3-dihydroxypropyl]hexanamid als farblosen Schaum lieferte, MS: 322 (M-H₂O)⁺.

### (R)-2-Amino-N-[(1S,2R,3S)-1-(cyclohexylmethyl)-3-cyclopropyl-2,3-dihydroxypropyl]-3-(methylthio)propionamid:

(c) Analog zu dem in Beispiel 1, Absatz (c) beschriebenen Verfahren wurde durch Kondensation von (1S,2R,3S)-3-Amino-4-cyclohexyl-1-cyclopropyl-1,2-butandiol und Fmoc-S-Methylcystein das 9H-Fluoren-9-ylmethyl [(S)-1-[[(1S,2R,3S)-1-(cyclohexylmethyl)-3-cyclopropyl-2,3-dihydroxypropyl]carbamoyl]-2-[(methylthio)äthyl]carbamat erhalten, das nach Umsetzung mit Piperidin in Methylenchlorid das (R)-2-Amino-N-[(1S,2R,3S)-(cyclohexylmethyl)-3-cyclopropyl-2,3-dihydroxypropyl]-3-(methylthio)propionamid als farblosen Festkörper lieferte, MS: 345 (M+H)⁺.
   Das als Ausgangsstoff eingesetzte Fmoc-S-Methylcystein wurde nach dem in der Literatur allgemein bekannten Verfahren zur Herstellung von Fmoc-Aminosäuren durch Umsetzung von 9H-Fluorenylmethylsuccinimidylcarbonat und S-Methylcystein erhalten.

### Beispiel 5

In analoger Weise wie in Beispiel 1 beschrieben, wurden die folgenden Verbindungen hergestellt:
- Aus (1S,2R,3S)-3-Amino-4-cyclohexyl-1-cyclopropyl-1,2-butandiol und DL-2-Hydroxy-N-[(S)-α-[[[(1-methyl-1-(morpholinocarbonyl)äthyl]sulfonyl]methyl]hydrocinnamoyl]glycin das (S)-N-[(R oder S)-[[(1S,2R,3S)-1-(Cyclohexylmethyl)-3-cyclopropyl-2,3-dihydroxypropyl]carbamoyl]hydroxymethyl]-α-[[[1-methyl-1-(morpholinocarbonyl)äthyl]sulfonyl]methyl]hydrozimtsäureamid, MS: 666 (M+H)⁺, und das epimere (S)-N-[(S oder R)-[[(1S,2R,3S)-1-(Cyclohexylmethyl)-3-cyclopropyl-2,3-dihydroxypropyl]carbamoyl]hydroxymethyl]-α-[[[1-methyl-1-(morpholinocarbonyl)äthyl]sulfonyl]methyl]hydrozimtsäureamid, MS: 666 (M+H)⁺, jeweils als farbloser Festkörper;
- aus (1S,2R,3S)-3-Amino-4-cyclohexyl-1-cyclopropyl-1,2-butandiol und D oder L-2-Aethoxy-N-[(S)-α-[[[1-methyl-1- (morpholinocarbonyl)äthyl]sulfonyl]methyl]hydrocinnamoyl]glycin das (S)-N-[(R oder S)-[[(1S,2R,3S)-1-(Cyclohexylmethyl)-3-cyclopropyl-2,3-dihydroxypropyl]carbamoyl]äthoxymethyl]-α-[[[1-methyl-1-(morpholinocarbonyl)äthyl]sulfonyl]methyl]hydrozimtsäureamid als farbloser Festkörper, MS: 694 (M+H)⁺;
- aus (1S,2R,3S)-3-Amino-4-cyclohexyl-1-cyclopropyl-1,2-butandiol und L oder D-2-Aethoxy-N-[(S)-α-[[[1-methyl-1-(morpholinocarbonyl)äthyl]sulfonyl]methyl]hydrocinnamoyl]glycin das (S)-N-[(S oder R)-[[(1S,2R,3S)-1-(Cyclohexylmethyl)-3-cyclopropyl-2,3-dihydroxypropyl]carbamoyl]äthoxymethyl]-α-[[[1-methyl-1-(morpholinocarbonyl)äthyl]sulfonyl]methyl]hydrozimtsäureamid als farbloser Festkörper, MS: 694 (M+H)⁺;
- aus (1S,2R,3S)-3-Amino-4-cyclohexyl-1-cyclopropyl-1,2-butandiol und DL-N-[(S)-α-[[[1-Methyl-1-(morpholinocarbonyl)äthyl]sulfonyl]methyl]hydrocinnamoyl]-2-(methylthio)glycin das (S)-N-[(RS)-[[(1S,2R,3S)-1-(Cyclohexylmethyl)-3-cyclopropyl-2,3-dihydroxypropyl]carbamoyl](methylthio)methyl)]-α-[[[1-methyl-1-(morpholinocarbonyl)äthyl]sulfonyl]methyl]hydrozimtsäureamid als farbloser Festkörper, MS: 696 (M+H)⁺;
- aus (1S,2R,3S)-3-Amino-4-cyclohexyl-1-cyclopropyl-1,2-butandiol und D oder L-2-(Allyloxy)-N-[(S)-α-[[[1-methyl-1-(morpholinocarbonyl)äthyl]sulfonyl]methyl]hydrocinnamoyl]glycin das (S)-N-[(S oder R)-(Allyloxy) [[(1S,2R,3S)-1-(cyclohexylmethyl)-3-cyclopropyl-2,3-dihydroxypropyl]carbamoyl]methyl]-α-[[[1-methyl-1-(morpholinocarbonyl)äthyl]sulfonyl]methyl]hydrozimtsäureamid als farbloser Festkörper, MS: 728 (M+Na)⁺;
- aus (1S,2R,3S)-3-Amino-4-cyclohexyl-1-cyclopropyl-1,2-butandiol und L oder D-2-(Allyloxy)-N-[(S)-α-[[[1-methyl-1-(morpholinocarbonyl)äthyl]sulfonyl]methyl]hydrocinnamoyl]glycin das (S)-N-[(R oder S)-(Allyloxy) [[(1S,2R,3S)-1-(cyclohexylmethyl)-3-cyclopropyl-2,3-dihydroxypropyl]carbamoyl]methyl]-α-[[[1-methyl-1-(morpholinocarbonyl)äthyl]sulfonyl]methyl]hydrozimtsäureamid als farbloser Festkörper, MS: 728 (M+Na)⁺;
- aus (1S,2R,3S)-3-Amino-4-cyclohexyl-1-cyclopropyl-1,2-butandiol und (RS)-[(S)-α-[[[1-Methyl-1- (morpholinocarbonyl)äthyl]sulfonyl]methyl]hydrocinnamamido]malonsäuremon omethylester das Methyl (RS)-N-[(1S,2R,3S)-1-(cyclohexylmethyl)-3-cyclopropyl-2,3-dihydroxypropyl]-2-[(S)-α-[[[1-methyl-1-(morpholinocarbonyl)äthyl]sulfonyl]methyl]hydrocinnamamido]malonat, MS: 708 (M+H)⁺, und das (S)-N-[[[(1S,2R,3S)-1-(Cyclohexylmethyl)-3-cyclopropyl-2,3-dihydroxypropyl]carbamoyl]methyl]-α-[[[1-methyl-1-(morpholinocarbonyl)äthyl]sulfonyl]methyl]hydrozimtsäureamid, MS: 650 (M+H)⁺, jeweils als farbloser Festkörper.

Die als Ausgangsstoffe eingesetzten Säuren wurden wie folgt hergestellt:

### DL-2-Hydroxy-N-[(S)-α-[[[(1-methyl-1-(morpholinocarbonyl)äthyl]sulfonyl]methyl]hydrocinnamoyl]glycin:

(a) Analog zu dem von Wang et al. in Synthesis 1989, 37 beschriebenen Verfahren wurde ein Gemisch von 15.34 g (40 mMol) (S)-α-[[[1-Methyl-1-(morpholinocarbonyl)äthyl]sulfonyl]methyl]hydrozimtsäure, 9.29 g (40 mMol) Dicyclohexylcarbodiimid und 6.89 g (50 mMol) HOBT in 160 ml Methylenchlorid und 40 ml Dimethylformamid 4 Stunden bei Raumtemperatur gerührt. Danach wurde das Reaktionsgemisch filtriert, die erhaltene gelbliche Lösung mit 200 ml Methylenchlorid verdünnt und auf 0° abgekühlt. In diese Lösung wurde Ammoniak bis zur Sättigung eingeleitet. Die erhaltene Suspension wurde 1 Stunde bei Raumtemperatur nachgerührt. Anschließend wurde der gebildete Festkörper abgesaugt und mit 100 ml Methylenchlorid gewaschen. Das gelb gefärbte Filtrat wurde in der Folge zweimal mit jeweils 200 ml Wasser und zweimal mit 200 ml gesättigter Natriumchloridlösung gewaschen. Die Waschlösungen wurden mit 500 ml Methylenchlorid extrahiert. Die vereinigten organischen Phasen wurden über Natriumsulfat getrocknet und danach unter vermindertem Druck eingedampft. Das erhaltene gelbliche Rohprodukt (15.3 g) wurde zur Reinigung an 1.3 kg Kieselgel unter Verwendung eines 4:1-Gemisches von Essigester und Hexan als Eluierungsmittel chromatographiert. Man erhielt 13.6 g (S)-α-[[[(1-Methyl-1-(morpholinocarbonyl)äthyl]sulfonyl]methyl]hydrozimtsäureamid als farbloses Pulver, MS: 383 (M+H)⁺.
(b) Durch Umsetzung von Glyoxylsäure-monohydrat mit (S)-α-[[[(1-Methyl-1-(morpholinocarbonyl)äthyl]sulfonyl]methyl]hydrozimtsäureamid in Aceton unter Rückfluß analog zu dem in EPA 0343654 beschriebenen Verfahren wurde das DL-2-Hydroxy-N-[(S)-α-[[[(1-methyl-1- (morpholinocarbonyl)äthyl]sulfonyl]methyl]hydrocinnamoyl]glycin, MS: 457 (M+H)⁺, in Form farbloser Kristalle erhalten.
   Die folgenden Verbindungen wurden ebenfalls analog zu dem in EPA 0343654 beschriebenen Verfahren hergestellt:

### D oder L-2-Aethoxy-N-[(S)-α-[[[1-methyl-1-(morpholinocarbonyl)äthyl]sulfonyl]methyl]hydrocinnamoyl]glycin und L oder D-2-Aethoxy-N-[(S)-α-[[[1-methyl-1-(morpholinocarbonyl)äthyl]sulfonyl]methyl]hydrocinnamoyl]glycin:

(c) Duch Umsetzung von DL-2-Hydroxy-N-[(S)-α-[[[(1-methyl-1-(morpholinocarbonyl)äthyl]sulfonyl]methyl]hydrocinnamoyl]glycin mit Aethanol und Schwefelsäure, gefolgt von einer chromatographischen Trennung der beiden diastereomeren Ester, der L oder D-2-(Aethylthio)-N-[(S)-α-[[[1-methyl-1-(morpholinocarbonyl)äthyl]sulfonyl]methyl]hydrocinnamoyl]glycinäthylester, MS: 513 (M+H)⁺, und der D oder L-2-(Aethylthio)-N-[(S)-α-[[[1-methyl-1-(morpholinocarbonyl)äthyl]sulfonyl]methyl]hydrocinnamoyl]glycinäthylester, MS: 513 (M+H)⁺, jeweils als farbloser Festkörper.
(d) Durch Hydrolyse von L oder D-2-(Aethylthio)-N-[(S)-α-[[[1-methyl-1-(morpholinocarbonyl)äthyl]sulfonyl]methyl]hydrocinnamoyl]glycinäthylester mit 1N Natronlauge in Aethanol das D oder L-2-Aethoxy-N-[(S)-α-[[[1-methyl-1-(morpholinocarbonyl)äthyl]sulfonyl]methyl]hydrocinnamoyl]glycin, MS: 507 (M+Na)⁺, als farbloser Festkörper.
(e) Durch Hydrolyse von D oder L-2-(Aethylthio)-N-[(S)-α-[[[1-methyl-1-(morpholinocarbonyl)äthyl]sulfonyl]methyl]hydrocinnamoyl]glycinäthylester mit 1N Natronlauge in Aethanol das L oder D-2-Aethoxy-N-[(S)-α-[[[1-methyl-1-(morpholinocarbonyl)äthyl]sulfonyl]methyl]hydrocinnamoyl]glycin, MS: 507 (M+Na)⁺, als farbloser Festkörper.

### DL-N-[(S)-α-[[[1-Methyl-1-(morpholinocarbonyl)äthyl]sulfonyl]methyl]hydrocinnamoyl]-2-(methylthio)glycin:

(f) Durch Umsetzung von DL-2-Hydroxy-N-[(S)-α-[[[(1-methyl-1-(morpholinocarbonyl)äthyl]sulfonyl]methyl]hydrocinnamoyl]glycin mit Aethylmercaptan und Schwefelsäure in Eisessig das DL-N-[(S)-α-[[[1-Methyl-1-(morpholinocarbonyl)äthyl]sulfonyl]methyl]hydrocinnamoyl]-2-(methylthio)glycin, MS: 487 (M+H)⁺, als farbloser Festkörper.

### D oder L-2-(Allyloxy)-N-[(S)-α-[[[1-methyl-1-(morpholinocarbonyl)äthyl]sulfonyl]methyl]hydrocinnamoyl]glycin und L oder D-2-(Allyloxy)-N-[(S)-α-[[[1-methyl-1-(morpholinocarbonyl)äthyl]sulfonyl]methyl]hydrocinnamoyl]glycin:

(g) Durch Umsetzung von DL-2-Hydroxy-N-[(S)-α-[[[(1-methyl-1-(morpholinocarbonyl)äthyl]sulfonyl]methyl]hydrocinnamoyl]glycin mit Allylalkohol und Schwefelsäure, gefolgt von einer chromatographischen Trennung der beiden diastereomeren Ester, der L oder D-2-(Allyloxy)-N-[(S)-α-[[[1-methyl-1-(morpholinocarbonyl)äthyl]sulfonyl]methyl]hydrocinnamoyl]glycinallylester, MS: 645 [M+H+Thioglycerin (=Matrix)]⁺, und der D oder L-2-(Allyloxy)-N-[(S)-α-[[[1-methyl-1-(morpholinocarbonyl)äthyl]sulfonyl]methyl]hydrocinnamoyl]glycinallylester, MS: 645 [M+H+Thioglycerin (=Matrix)]⁺, jeweils als farbloser Festkörper.
(h) Durch Hydrolyse von L oder D-2-(Allyloxy)-N-[(S)-α-[[[1-methyl-1-(morpholinocarbonyl)äthyl]sulfonyl]methyl]hydrocinnamoyl]glycinallylester mit 1N Natronlauge in Dioxan das D oder L-2-(Allyloxy)-N-[(S)-α-[[[1-methyl-1-(morpholinocarbonyl)äthyl]sulfonyl]methyl]hydrocinnamoyl]glycin, MS: 627 [M+Na+Thioglycerin (=Matrix)]⁺, als farbloser Festkörper.
(i) Durch Hydrolyse von D oder L-2-(Allyloxy)-N-[(S)-α-[[[1-methyl-1-(morpholinocarbonyl)äthyl]sulfonyl]methyl]hydrocinnamoyl]glycinallylester mit 1N Natronlauge in Dioxan das L oder D-2-(Allyloxy)-N-[(S)-α-[[[1-methyl-1-(morpholinocarbonyl)äthyl]sulfonyl]methyl]hydrocinnamoyl]glycin, MS: 627 [M+Na+Thioglycerin (=Matrix)]⁺, als farbloser Festkörper.

### (RS)-[(S)-α-[[[1-Methyl-1-(morpholinocarbonyl)äthyl]sulfonyl]methyl]hydrocinnamamido]malonsäuremon omethylester:

(j) Durch Kondensation von (S)-α-[[[1-Methyl-1-(morpholinocarbonyl)äthyl]sulfonyl]methyl]hydrozimtsäureäthylester und Aminomalonsäuredimethylester der [(S)-α-[[[(1-Methyl-1-(morpholinocarbonyl)äthyl]sulfonyl]methyl]hydrocinnamamido]malonsäuredime thylester, MS: 513 (M+H)⁺, als farbloser Festkörper.
(k) Durch Hydrolyse von [(S)-α-[[[1-Methyl-1-(morpholinocarbonyl)äthyl]sulfonyl]methyl]hydrocinnamamido]malonsäuredime thylester mit 1N Natronlauge in Methanol der (RS)-[(S)-α-[[[1-Methyl-1-(morpholinocarbonyl)äthyl]sulfonyl]methyl]hydrocinnamamido]malonsäuremon omethylester, MS: 499 (M+H)⁺, als farbloser Festkörper.

### Beispiel 6

In analoger Weise wie in Beispiel 1 beschrieben, wurde durch Kondensation von (S)-α-[[[1-Methyl-1-(morpholinocarbonyl)äthyl]sulfonyl]methyl]hydrozimtsäure und (S)-α-Amino-N-[(1S,2R,3S)-1-(cyclohexylmethyl)-2,3-dihydroxy-5-methylhexyl]imidazol-4-propionamid (EPA 0189203) das (S)-N-[(1S,2R,3S)-1-(Cyclohexylmethyl)-2,3-dihydroxy-5-methylhexyl]-α-[(S)-α-[[[(1-methyl-1-(morpholinocarbonyl)äthyl]sulfonyl]methyl]hydrocinnamamido]imidazol-4-propionamid als farbloses Harz erhalten, MS: 746 (M+H)⁺.

### Beispiel 7

In analoger Weise wie in Beispiel 1 beschrieben, wurde durch Kondensation von (S)-α-[[[1-Methyl-1-(morpholinocarbonyl)äthyl]sulfonyl]methyl]hydrozimtsäure und (S)-α-Amino-N-[(1S,2R,3S)-1-(cyclohexylmethyl)-2,3-dihydroxy-4-methylpentyl]imidazol-4-propionamid (EPA 0332008) das (S)-N-[(1S,2R,3S)-1-(Cyclohexylmethyl)-2,3-dihydroxy-4-methylpentyl]-α-[(S)-α-[[[1-methyl-1-(morpholinocarbonyl)äthyl]sulfonyl]methyl]hydrocinnamamido]imidazol-4-propionamid als farbloses Harz erhalten, MS: 732 (M+H)⁺.

### Beispiel 8

In analoger Weise wie in Beispiel 1 beschrieben, wurden die folgenden Verbindungen hergestellt:
- Aus (S)-α-Amino-N-[(1S,2R,3S)-1-(cyclohexylmethyl)-3-cyclopropyl-2,3-dihydroxypropyl]imidazol-4-propionamid und (S)-α-[[[1-Methyl-1-(morpholinocarbonyl)äthyl]sulfonyl]methyl]-3-(naphthalin-1-yl)propionsäure das (S)-N-[(1S,2R,3S)-1-(Cyclohexylmethyl)-3-cyclopropyl-2,3-dihydroxypropyl]-α-[(S)-α-[[[1-methyl-1-(morpholinocarbonyl)äthyl]sulfonyl]methyl]-3-(naphthalin-1-yl)propionamido]imidazol-4-propionamid als farbloser Schaum, MS: 780 (M+H)⁺;
- aus (S)-α-Amino-N-[(1S,2R,3S)-1-(cyclohexylmethyl)-3-cyclopropyl-2,3-dihydroxypropyl]imidazol-4-propionamid und (S)-p-Fluor-α-[[[1-methyl-1-(morpholinocarbonyl)äthyl]sulfonyl]methyl]hydrozimtsäure das (S)-N-[(1S,2R,3S)-1-(Cyclohexylmethyl)-3-cyclopropyl-2,3-dihydroxypropyl]-α-[(S)-p-fluor-α-[[[1-methyl-1-(morpholinocarbonyl)äthyl]sulfonyl]methyl]hydrocinnamamido]imidazol-4-propionamid als farbloser Schaum, MS: 748 (M+H)⁺;
- aus (S)-α-Amino-N-[(1S,2R,3S)-1-(cyclohexylmethyl)-3-cyclopropyl-2,3-dihydroxypropyl]imidazol-4-propionamid und (S)-α-[[[1-Methyl-1-(morpholinocarbonyl)äthyl]sulfonyl]methyl]-3-(thiophen-2-yl)propionsäure das (S)-N-[(1S,2R,3S)-1-(Cyclohexylmethyl)-3-cyclopropyl-2,3-dihydroxypropyl]-α-[(S)-α-[[[1-methyl-1-(morpholinocarbonyl)äthyl]sulfonyl]methyl]-3-(thiophen-2-yl)propionamido]imidazol-4-propionamid als farbloser Schaum, MS: 736 (M+H)⁺;
- aus (S)-α-Amino-N-[(1S,2R,3S)-1-(cyclohexylmethyl)-3-cyclopropyl-2,3-dihydroxypropyl]imidazol-4-propionamid und (S)-α-[[[1-Methyl-1-(morpholinocarbonyl)äthyl]sulfonyl]methyl]cyclohexanpropionsäure das (S)-N-[(1S,2R,3S)-1-(Cyclohexylmethyl)-3-cyclopropyl-2,3-dihydroxypropyl]-α-[(S)-α-[[[1-methyl-1-(morpholinocarbonyl)äthyl]sulfonyl]methyl]-3-cyclohexylpropionamido]imidazol-4-propionamid als farbloser Schaum, MS: 736 (M+H)⁺.

Die als Ausgangsstoffe eingesetzten Säuren wurden wie folgt hergestellt:

### (S)-α-[[[1-Methyl-1-(morpholinocarbonyl)äthyl]sulfonyl]methyl]-3-(naphthalin-1-yl)propionsäure:

In analoger Weise wie in Beispiel 1, Absätze (d) und (e) und Beispiel 2, Absatz (a) beschrieben, erhielt man, ausgehend von 2-Mercaptoisobuttersäure und 2-Methylen-3-(naphthalin-1-yl)propionsäureäthylester, den (R,S)-α-[[[1-Methyl-1-(morpholinocarbonyl)äthyl]sulfonyl]methyl]-3-(naphthalin-1-yl)propionsäureäthylester, welcher, in analoger Weise wie in Beispiel 3, Absatz (c) beschrieben, enzymatisch verseift wurde und so die (S)-α-[[[1-Methyl-1-(morpholinocarbonyl)äthyl]sulfonyl]methyl]-3-(naphthalin-1-yl)propionsäure, MS: 456 (M+H)⁺, lieferte.

### (S)-p-Fluor-α-[[[1-methyl-1-(morpholinocarbonyl)äthyl]sulfonyl]methyl]hydrozimtsäure:

In analoger Weise wie in Beispiel 1, Absätze (d) und (e) und Beispiel 2, Absatz (a) beschrieben, erhielt man, ausgehend von 2-Mercaptoisobuttersäure und 3-(4-Fluorphenyl)-2-methylenpropionsäureäthylester den (R,S)-p-Fluor-α-[[[-methyl-1-(morpholinocarbonyl)äthyl]sulfonyl] methyl]hydrozimtsäureäthylester, welcher, in analoger Weise wie in Beispiel 3, Absatz (c) beschrieben, enzymatisch verseift wurde und so die (S)-p-Fluor-α-[[[1-methyl-1-(morpholinocarbonyl)äthyl]sulfonyl]methyl]hydrozimtsäure, MS: 402 (M+H)⁺, lieferte.

### (S)-α-[[[1-Methyl-1-(morpholinocarbonyl)äthyl]sulfonyl]methyl]-3-(thiophen-2-yl)propionsäure:

In analoger Weise wie in Beispiel 1, Absätze (d) und (e) und Beispiel 2, Absatz (a) beschrieben, erhielt man, ausgehend von 2-Mercaptoisobuttersäure und 2-Methylen-3-(thiophen-2-yl)propionsäureäthylester den (R,S)-α-[[[1-Methyl-1-(morpholinocarbonyl)äthyl]sulfonyl]methyl]-3-(thiophen-2-yl)propionsäureäthylester, welcher, in analoger Weise wie in Beispiel 3, Absatz (c) beschrieben, enzymatisch verseift wurde und so die (S)-α-[[[1-Methyl-1-(morpholinocarbonyl)äthyl]sulfonyl]methyl]-3-(thiophen-2-yl)propionsäure, MS: 390 (M+H)⁺, lieferte.

### (S)-α-[[[1-Methyl-1-(morpholinocarbonyl)äthyl]sulfonyl]methyl]cyclohexanpropionsäure:

In analoger Weise wie in Beispiel 1, Absätze (d) und (e) beschrieben, erhielt man, ausgehend von 2-Mercaptoisobuttersäure und 2-Benzylacrylsäureäthylester, das (RS)-2-[[2-[(Äthoxy)carbonyl]-3-phenylpropyl]sulfonyl]-2-methylpropionsäurechlorid, dessen Umsetzung mit Morpholin, analog Beispiel 2, Absatz (a), und nachfolgende enzymatische Hydrolyse, analog Beispiel 3, Absatz (c), die (S)-α-[[[1-Methyl-1-(morpholinocarbonyl]äthyl]sulfonyl]methyl]hydrozimtsäure, MS: 383 (M)⁺, als farblosen Festkörper lieferte.

Eine Lösung von 610 mg (1.59 mMol) (S)-α-[[[1-Methyl-1-(morpholinocarbonyl]äthyl]sulfonyl]methyl]hydrozimtsäure in 20 ml Methanol wurde in Gegenwart von 60 mg Rhodium auf Aluminiumoxid bei 50° bei einem Druck von 10⁶Pa Wasserstoff 4 Stunden hydriert. Anschließend wurde der Katalysator abfiltriert und mit Methanol nachgewaschen. Die methanolische Lösung wurde unter vermindertem Druck eingedampft, wobei 580 mg (S)-α-[[[1-Methyl-1-(morpholinocarbonyl)äthyl]sulfonyl]methyl]cyclohexanpropionsäure, MS : 346 (M-C₃H₇)⁺, als farbloser, kristalliner Festkörper erhalten wurden.

### Beispiel 9

In analoger Weise wie in Beispiel 1 beschrieben, wurden folgende Verbindungen hergestellt:
- Aus (S oder R)-α-Amino-N-[(1S,2R,3S)-1-(cyclohexylmethyl)-3-cyclopropyl-2,3-dihydroxypropyl]-3-(pyridin-3-yl)propionamid und (S)-α-[[[1-Methyl-1-(morpholinocarbonyl)äthyl]sulfonyl]methyl]hydrozimtsäure das (S oder R)-N-[(1S,2R,3S)-1-(Cyclohexylmethyl)-3-cyclopropyl-2,3-dihydroxypropyl]-α-[(S)-α-[[[1-methyl-1-(morpholinocarbonyl)äthyl]sulfonyl]methyl]hydrocinnamamido]-3-(pyridin-3-yl)propionamid als farbloser Schaum, MS: 741 (M+H))⁺;
- aus (R oder S)-α-Amino-N-[(1S,2R,3S)-1-(cyclohexylmethyl)-3-cyclopropyl-2,3-dihydroxypropyl]-3-(pyridin-3-yl)propionamid und (S)-α-[[[1-Methyl-1-(morpholinocarbonyl)äthyl]sulfonyl]methyl]hydrozimtsäure das (R oder S)-N-[(1S,2R,3S)-1-(Cyclohexylmethyl)-3-cyclopropyl-2,3-dihydroxypropyl]-α-[(S)-α-[[[1-methyl-1-(morpholinocarbonyl)äthyl]sulfonyl]methyl]hydrocinnamamido]-3-(pyridin-3-yl)propionamid als farbloser Schaum, MS: 741 (M+H)⁺;
- aus (S oder R)-α-Amino-N-[(1S,2R,3S)-1-(cyclohexylmethyl)-3-cyclopropyl-2,3-dihydroxypropyl]-3-(pyridin-2-yl)propionamid und (S)-α-[[[1-Methyl-1-(morpholinocarbonyl)äthyl]sulfonyl]methyl]hydrozimtsäure das (S oder R)-N-[(1S,2R,3S)-1-(Cyclohexylmethyl)-3-cyclopropyl-2,3-dihydroxypropyl]-α-[(S)-α-[[[1-methyl-1-(morpholinocarbonyl)äthyl]sulfonyl]methyl]hydrocinnamamido]-3-(pyridin-2-yl)propionamid als farbloser Schaum, MS: 741 (M+H))⁺;
- aus (R oder S)-α-Amino-N-[(1S,2R,3S)-1-(cyclohexylmethyl)-3-cyclopropyl-2,3-dihydroxypropyl]-3-(pyridin-2-yl)propionamid und (S)-α-[[[1-Methyl-1-(morpholinocarbonyl)äthyl]sulfonyl]methyl]hydrozimtsäure das (R oder S)-N-[(1S,2R,3S)-1-(Cyclohexylmethyl)-3-cyclopropyl-2,3-dihydroxypropyl]-α-[(S)-α- [[[1-methyl-1-(morpholinocarbonyl)äthyl]sulfonyl]methyl]hydrocinnamamido]-3-(pyridin-2-yl)propionamid als farbloser Schaum, MS: 741 (M+H))⁺;
- aus (S oder R)-α-Amino-N-[(1S,2R,3S)-1-(cyclohexylmethyl)-3-cyclopropyl-2,3-dihydroxypropyl]-3-(pyridin-4-yl)propionamid und (S)-α-[[[1-Methyl-1-(morpholinocarbonyl)äthyl]sulfonyl]methyl]hydrozimtsäure das (S oder R)-N-[(1S,2R,3S)-1-(Cyclohexylmethyl)-3-cyclopropyl-2,3-dihydroxypropyl]-α-[(S)-α-[[[1-methyl-1-(morpholinocarbonyl)äthyl]sulfonyl]methyl]hydrocinnamamidol]-3-(pyridin-4-yl)propionamid als farbloser Schaum, MS: 741 (M+H))⁺;
- aus (R oder S)-α-Amino-N-[(1S,2R,3S)-1-(cyclohexylmethyl)-3-cyclopropyl-2,3-dihydroxypropyl]-3-(pyridin-4-yl)propionamid und (S)-α-[[[1-Methyl-1-(morpholinocarbonyl)äthyl]sulfonyl]methyl]hydrozimtsäure das (R oder S)-N-[(1S,2R,3S)-1-(Cyclohexylmethyl)-3-cyclopropyl-2,3-dihydroxypropyl]-α-[(S)-α-[[[1-methyl-1-(morpholinocarbonyl)äthyl]sulfonyl]methyl]hydrocinnamamido]-3-(pyridin-4-yl)propionamid als farbloser Schaum, MS: 741 (M+H))⁺;
- aus (S)-α-Amino-N-[(1S,2R,3S)-1-(cyclohexylmethyl)-3-cyclopropyl-2,3-dihydroxypropyl]-3-(pyrazol-1-yl)propionamid und (S)-α-[[[1-Methyl-1-(morpholinocarbonyl)äthyl]sulfonyl]methyl]hydrozimtsäure das (S)-N-[(1S,2R,3S)-1-(Cyclohexylmethyl)-3-cyclopropyl-2,3-dihydroxypropyl]-α-[(S)-α-[[[1-methyl-1-(morpholinocarbonyl)äthyl]sulfonyl]methyl]hydrocinnamamido]-3-(pyrazol-1-yl)propionamid als farbloser Schaum, MS: 730 (M+H))⁺;
- aus (S)-α-Amino-N-[(1S,2R,3S)-1-(cyclohexylmethyl)-3-cyclopropyl-2,3-dihydroxypropyl]-3-(imidazol-1-yl)propionamid und (S)-α-[[[1-Methyl-1-(morpholinocarbonyl)äthyl]sulfonyl]methyl]hydrozimtsäure das (S)-N-[(1S,2R,3S)-1-(Cyclohexylmethyl)-3-cyclopropyl-2,3-dihydroxypropyl]-α-[(S)-α-[[[1-methyl-1-(morpholinocarbonyl)äthyl]sulfonyl]methyl]hydrocinnamamido]-3-(imidazol-1-yl)propionamid als farbloser Schaum, MS: 730 (M+H))⁺;
- aus (RS)-α-Amino-N-[(1S,2R,3S)-1-(cyclohexylmethyl)-3-cyclopropyl-2,3-dihydroxypropyl]-3-(thiophen-2-yl)propionamid und (S)-α-[[[1-Methyl-1-(morpholinocarbonyl)äthyl]sulfonyl]methyl]hydrozimtsäure das weniger polare (S oder R)-N-[(1S,2R,3S)-1-(Cyclohexylmethyl)-3-cyclopropyl-2,3-dihydroxypropyl]-α-[(S)-α-[[[1-methyl-1-(morpholinocarbonyl)äthyl]sulfonyl]methyl]hydrocinnamamido]-3-(thiophen-2-yl)propionamid, MS: 746 (M+H)⁺, und das polarere Epimer (R oder S)-N-[(1S,2R,3S)-1-(Cyclohexylmethyl)-3-cyclopropyl-2,3-dihydroxypropyl]-α-[(S)-α-[[[1-methyl-1-(morpholinocarbonyl)äthyl]sulfonyl]methyl]hydrocinnamamido]-3-(thiophen-2-yl)propionamid , MS: 746 (M+H)⁺, jeweils als farbloser Schaum;
- aus (RS)-α-Amino-N-[(1S,2R,3S)-1-(cyclohexylmethyl)-3-cyclopropyl-2,3-dihydroxypropyl]-3-(furan-2-yl)propionamid und (S)-α-[[[1-Methyl-1-(morpholinocarbonyl)äthyl]sulfonyl]methyl]hydrozimtsäure das weniger polare (S oder R)-N-[(1S,2R,3S)-1-(Cyclohexylmethyl)-3-cyclopropyl-2,3-dihydroxypropyl]-α-[(S)-α-[[[1-methyl-1-(morpholinocarbonyl)äthyl]sulfonyl]methyl]hydrocinnamamido]-3-(furan-2-yl)propionamid, MS: 730 (M+H)⁺, und das polarere Epimer (R oder S)-N-[(1S,2R,3S)-1-(Cyclohexylmethyl)-3-cyclopropyl-2,3-dihydroxypropyl]-α-[(S)-α-[[[1-methyl-1-(morpholinocarbonyl)äthyl]sulfonyl]methyl]hydrocinnamamido]-3-(furan-2-yl)propionamid, MS: 730 (M+H)⁺, jeweils als farbloser Schaum.

Die als Ausgangsstoffe eingesetzten Amine wurden wie folgt hergestellt:

### (S oder R)- und (R oder S)-α-Amino-N-[(1S,2R,3S)-1-(cyclohexylmethyl)-3-cyclopropyl-2,3-dihydroxypropyl]-3-(pyridin-3-yl)propionamid:

Zu einer Lösung von 878 mg (3.3 mMol) N-(tert-Butoxycarbonyl)-3-(pyridin-3-yl)-D,L-alanin [J. Gen. Chem. USSR, 40, 2488 (1970)] und 750 mg (3.3 mMol) (1S,2R,3S)-3-Amino-4-cyclohexyl-1-cyclopropyl-1,2-butandiol in 33 ml Acetonitril wurden 1.27 g (3.3 mMol) HBTU und 0.45 ml (3.3 mMol) Triäthylamin gegeben und über Nacht bei Raumtemperatur gerührt. Die nach Filtration erhaltenen farblosen Kristalle löste man in 30 ml Äthanol und gab 23.3 ml 1N Salzsäure dazu. Nach 24 Stunden bei 50° wurde die Lösung mit 2N Natronlauge basisch gestellt, unter vermindertem Druck eingedampft, und der Rückstand zwischen Wasser und Essigsäureäthylester verteilt. Die wäßrige Phase wurde dreimal mit Essigsäureäthylester extrahiert, die vereinigten organischen Phasen mit Magnesiumsulfat getrocknet, filtriert und unter vermindertem Druck eingedampft. Chromatographie des Rückstandes (Kieselgel, Methylenchlorid/Methanol/Ammoniak 140/10/1) lieferte 315 mg (S oder R)-α-Amino-N-[(1S,2R,3S)-1-(cyclohexylmethyl)-3-cyclopropyl-2,3-dihydroxypropyl]-3-(pyridin-3-yl)propionamid, MS: 267 (M+H)⁺, als farblosen Schaum, sowie 315 mg (R oder S)-α-Amino-N-[(1S,2R,3S)-1-(cyclohexylmethyl)-3-cyclopropyl-2,3-dihydroxypropyl]-3-(pyridin-3-yl)propionamid, MS: 267 (M+H)⁺, als farblosen Schaum.

### (S oder R)- und (R oder S)-α-Amino-N-[(1S,2R,3S)-1-(cyclohexylmethyl)-3-cyclopropyl-2,3-dihydroxypropyl]-3-(pyridin-2-yl)propionamid:

In analoger Weise wie oben beschrieben, erhielt man durch Kondensation von N-(tert-Butoxycarbonyl)-3-(pyridin-2-yl)-D,L-alanin [J. Gen. Chem. USSR, 40, 2488 (1970)] und (1S,2R,3S)-3-Amino-4-cyclohexyl-1-cyclopropyl-1,2-butandiol, gefolgt von einer sauren Hydrolyse, das (S oder R)-α-Amino-N-[(1S,2R,3S)-1-(cyclohexylmethyl)-3-cyclopropyl-2,3-dihydroxypropyl]-3-(pyridin-2-yl)propionamid, MS: 267 (M+H)⁺, und das (R oder S)-α-Amino-N-[(1S,2R,3S)-1-(cyclohexylmethyl)-3-cyclopropyl-2,3-dihydroxypropyl]-3-(pyridin-2-yl)propionamid, MS: 267 (M+H)⁺, jeweils als farblosen Schaum.

### (S oder R)- und (R oder S)-α-Amino-N-[(1S,2R,3S)-1-(cyclohexylmethyl)-3-cyclopropyl-2,3-dihydroxypropyl]-3-(pyridin-4-yl)propionamid:

In analoger Weise wie oben beschrieben, erhielt man durch Kondensation von N-(tert-Butoxycarbonyl)-3-(pyridin-4-yl)-D,L-alanin [J. Gen. Chem. USSR, 40, 2488 (1970)] und (1S,2R,3S)-3-Amino-4-cyclohexyl-1-cydopropyl-1,2-butandiol, gefolgt von einer sauren Hydrolyse, das (S oder R)-α-Amino-N-[(1S,2R,3S)-1-(cyclohexylmethyl)-3-cyclopropyl-2,3-dihydroxypropyl]-3-(pyridin-4-yl)propionamid, MS: 267 (M+H)⁺, und (R oder S)-α-Amino-N-[(1S,2R,3S)-1-(cyclohexylmethyl)-3-cyclopropyl-2,3-dihydroxypropyl]-3-(pyridin-4-yl)propionamid, MS: 267 (M+H)⁺, jeweils als farblosen Schaum.

### (S)-α-Amino-N-[(1S,2R,3S)-1-(cyclohexylmethyl)-3-cyclopropyl-2,3-dihydroxypropyl]-3-(pyrazol-1-yl)propionamid:

Zu einer Lösung von 820 mg (2.83 mMol) N-[(Benzyloxy)carbonyl]-3-(pyrazol-1-yl)-L-alanin [J. Am. Chem . Soc. 107, 7105 (1985)] und 644 mg (2.83 mMol) (1S,2R,3S)-3-Amino-4-cyclohexyl-1-cyclopropyl-1,2-butandiol in 28 ml Acetonitril wurden 1.03 g (2.83 mMol) HBTU und 0.39 ml (2.83 mMol) Triäthylamin gegeben und über Nacht bei Raumtemperatur gerührt. Die nach Filtration erhaltenen Kristalle wurden in 30 ml Äthanol gelöst und bei 40° zwei Stunden hydriert. Filtration über Diatomeenerde und Eindampfen unter vermindertem Druck lieferte 670 mg (S)-α-Amino-N-[(1S,2R,3S)-1-(cyclohexylmethyl)-3-cyclopropyl-2,3-dihydroxypropyl]-3-(pyrazol-1-yl)propionamid, MS: 365 (M+H)⁺, als farblosen Schaum.

### (S)-α-Amino-N-[(1S,2R,3S)-1-(cyclohexylmethyl)-3-cyclopropyl-2,3-dihydroxypropyl]-3-(imidazol-1-yl)propionamid:

In analoger Weise wie oben beschrieben, erhielt man durch Kondensation von N-[(Benzyloxy)carbonyl]-3-(imidazol-1-yl)-L-alanin und (1S,2R,3S)-3-Amino-4-cyclohexyl-1-cyclopropyl-1,2-butandiol, gefolgt von einer katalytischen Hydrierung, das (S)-α-Amino-N-[(1S,2R,3S)-1-(cydohexylmethyl)-3-cyclopropyl-2,3-dihydroxypropyl]-3-(imidazol-1-yl)propionamid, MS: 365 (M+H)⁺, als farblosen Schaum.

Das verwendete N-[(Benzyloxy)carbonyl]-3-(imidazol-1-yl)-L-alanin, MS: 290 (M+H)⁺, wurde in Analogie zur der in J. Am. Chem. Soc. 107, 7105 (1985) beschriebenen Synthese von N-[(Benzyloxy)carbonyl]-3-(pyrazolyl-1-yl)-L-alanin hergestellt, indem man das Pyrazol durch Imidazol ersetzte.

### (RS)-α-Amino-N-[(1S,2R,3S)-1-(cyclohexylmethyl)-3-cyclopropyl-2,3-dihydroxypropyl]-3-(thiophen-2-yl)propionamid:

In analoger Weise wie oben beschrieben, erhielt man durch Kondensation von rac-α-(tert-Butoxyformamido)-3-(thiophen-2-yl)propionsäure [Pol. J. Chem. 54(11-12), 2225 (1980)] und (1S,2R,3S)-3-Amino-4-cyclohexyl-1-cyclopropyl-1,2-butandiol, gefolgt von einer sauren Hydrolyse, das (RS)-α-Amino-N-[(1S,2R,3S)-1-(cyclohexylmethyl)-3-cyclopropyl-2,3-dihydroxypropyl]-3-(thiophen-2-yl)propionamid, MS: 381 (M+H)⁺ als farblosen Schaum.

### (RS)-α-Amino-N-[(1S,2R,3S)-1-(cyclohexylmethyl)-3-cyclopropyl-2,3-dihydroxypropyl]-3-(furan-2-yl)propionamid:

In analoger Weise wie oben beschrieben, erhielt man durch Kondensation von (RS)-α-(1-Benzyloxyformamido)-3-(füran-2-yl)propionsäure [J. Biol. Chem. 171, 383 (1947)] und (1S,2R,3S)-3-Amino-4-cyclohexyl-1-cyclopropyl-1,2-butandiol, gefolgt von einer katalytischen Hydrierung, das rac-α-Amino-N-[(1S,2R,3S)-1-(cyclohexylmethyl)-3-cyclopropyl-2,3-dihydroxypropyl]-3-(furan-2-yl)propionamid, MS: 365 (M+H)⁺, als farblosen Schaum.

### Beispiel 10

In analoger Weise wie in Beispiel 1 beschrieben, wurden folgende Verbindungen hergestellt:
- Aus (S)-α-[[[1-Methyl-1-[methyl-[2-(2-pyridyl)äthyl]carbamoyl]äthyl]sulfonyl]methyl]hydrozimtsäure und (S)-Amino-N-[(1S,2R,3S)-1-(cyclohexylmethyl)-3-cyclopropyl-2,3-dihydroxypropyl]imidazol-4-propionamid das (S)-N-[(1S,2R,3S)-1-(Cyclohexylmethyl)-3-cyclopropyl-2,3-dihydroxypropyl]-α-[(S)-α-[[[1-methyl-1-[methyl-[2-(2-pyridyl)äthyl]carbamoyl]äthyl]sulfonyl]methyl]hydrocinnamamido]imidazol-4-propionamid als farbloser Schaum, MS: 779 (M+H))⁺;
- aus (S)-α-[[[1-Methyl-1-[methyl-[2-(2-pyridyl)äthyl]carbamoyl]äthyl]sulfonyl]methyl]hydrozimtsäure und (S)-Amino-N-[(1S,2R,3S)-1-(cyclohexylmethyl)-3-cyclopropyl-2,3-dihydroxypropyl]imidazol-4-propionamid das (S)-N-[(S)-1-[[(1S,2R,3S)-1-(Cyclohexylmethyl)-3-cyclopropyl-2,3-dihydroxypropyl]carbamoyl]pentyl]-α-[[1-methyl-1-[methyl-[2-(2-pyridyl)äthyl]äthyl]sulfonyl]methyl]hydrocinnamamid als farbloser Schaum, MS: 755 (M+H))⁺;
- aus (S)-α-[[[(Morpholinocarbonyl)methyl]sulfonyl]methyl]hydrozimtsäure und (S)-Amino-N-[(1S,2R,3S)-1-(cyclohexylmethyl)-3-cyclopropyl-2,3-dihydroxypropyl]imidazol-4-propionamid das (S)-N-[(1S,2R,3S)-1-(Cyclohexylmethyl)-3-cyclopropyl-2,3-dihydroxypropyl]-α-[(S)-α-[[[(morpholinocarbonyl)methyl]sulfonyl]methyl]hydrocinnamamido]imidazol-4-propionamid als farbloser Festkörper, MS : 702 (M+H))⁺;
- aus (S)-α-[[[(RS)-1-(Morpholinocarbonyl)äthyl]sulfonyl]methyl]hydrozimtsäure und (S)-Amino-N-[(1S,2R,3S)-1-(cyclohexylmethyl)-3-cyclopropyl-2,3-dihydroxypropyl]imidazol-4-propionamid das (S)-N-[(1S,2R,3S)-1-(Cyclohexylmethyl)-3-cyclopropyl-2,3-dihydroxypropyl]-α-[(S)-α-[[[(RS)-1-(morpholinocarbonyl)äthyl]sulfonyl]methyl]hydrocinnamamido]imidazol-4-propionamid als farbloser, amorpher Festkörper, MS: 716 (M+))⁺;
- aus (S)-α-[[[1-Methyl-1-[(tetrahydro-4H-1,4-thiazin-4-yl)carbonyl]äthyl]sulfonyl]methyl]hydrozimtsäure und (S)-Amino-N-[(1S,2R,3S)-1-(cyclohexylmethyl)-3-cyclopropyl-2,3-dihydroxypropyl]imidazol-4-propionamid das (S)-N-[(1S,2R,3S)-1-(Cyclohexylmethyl)-3-cyclopropyl-2,3-dihydroxypropyl]-α-[(S)-α-[[[1-methyl-1-[(tetrahydro-4H-1,4-thiazin-4- yl)carbonyl]äthyl]sulfonyl]methyl]hydrocinnamamido]imidazol-4-propionanamid als farbloser Festkörper, MS: 746 (M+H))⁺;
- aus (S)-α-[[[1-Methyl-1-[(tetrahydro-4'H-1,4-thiazin-4'-yl)carbonyl]äthyl]sulfonyl]methyl]hydrozimtsäure-1',1'-dioxid und (S)-Amino-N-[(1S,2R,3S)-1-(cyclohexylmethyl)-3-cyclopropyl-2,3-dihydroxypropyl]imidazol-4-propionamid das (S)-N-[(1S,2R,3S)-1-(Cyclohexylmethyl)-3-cyclopropyl-2,3-dihydroxypropyl]-α-[(S)-α-[[[1-methyl-1-[(tetrahydro-4'H-1,4-thiazin-4'-yl)carbonyl]äthyl]sulfonyl]methyl]hydrocinnamamido]imidazol-4-propionamid-1',1'-dioxid als farbloser Festkörper, MS: 778 (M+H))⁺;
- aus (S)-α-[[[1-Methyl-1-[(4-methyl-1-piperazinyl)carbonyl]äthyl]sulfonyl]methyl]hydrozimtsäure und (S)-Amino-N-[(1S,2R,3S)-1-(cyclohexylmethyl)-3-cyclopropyl-2,3-dihydroxypropyl]imidazol-4-propionamid das (S)-N-[(1S,2R,3S)-1-(Cyclohexylmethyl)-3-cyclopropyl-2,3-dihydroxypropyl]-α-[(S)-α-[[[1-methyl-1-[(4-methyl-1-piperazinyl)carbonyl]äthyl]sulfonyl]methyl]hydrocinnamamido]imidazol-4-propionamid als farbloser Festkörper, MS: 743 (M+H))⁺;
- aus (S/R=9/1)-α-[[[1-[[2-(2-Hydroxyäthoxy)äthyl]carbamoyl]-1-methyläthyl]sulfonyl]methyl]hydrozimtsäure und (S)-Amino-N-[(1S,2R,3S)-1-(cyclohexylmethyl)-3-cyclopropyl-2,3-dihydroxypropyl]imidazol-4-propionamid das (S)-N-[(1S,2R,3S)-1-(Cyclohexylmethyl)-3-cyclopropyl-2,3-dihydroxypropyl]-α-[(S/R=9/1)-α-[[[1-[2-(2-hydroxyäthoxy)carbamoyl]-1-methyläthyl]sulfonyl]methyl]hydrocinnamamido]imidazol-4-propionamid als farbloses Öl, MS: 748.3 (M+H)⁺.

Die als Ausgangsstoffe eingesetzten Säuren wurden wie folgt hergestellt:

### (S)-α-[[[1-Methyl-1-[methyl-[2-(2-pyridyl)äthyl]carbamoyl]äthyl]sulfonyl]methyl] hydrozimtsäure:

In analoger Weise wie in Beispiel 1 beschrieben, erhielt man durch Kondensation von rac-2-[[2-(Äthoxycarbonyl)-3-phenylpropyl]sulfonyl]-2-methylpropionsäure und 2-(2-Methylaminoäthyl)pyridin den (S)-α-[[[1-Methyl-1-[methyl-[2-(2-pyridyl)äthyl]carbamoyl]äthyl]sulfonyl]methyl]hydrozimtsäureäthylester. Die nachfolgende enzymatische Verseifung, analog Beispiel 3, Absatz (c), lieferte so die (S)-α-[[[1-Methyl-1-[methyl-[2-(2-pyridyl)äthyl]carbamoyl]äthyl]sulfonyl]methyl] hydrozimtsäure als farblosen Festkörper, MS: 470 (M+H)⁺.

### (S)-α-[[[(Morpholinocarbonyl)methyl]sulfonyl]methyl]hydrozimtsäure:

In analoger Weise wie in Beispiel 1, Absätze (d) und (e) und Beispiel 2, Absatz (a) beschrieben, erhielt man, ausgehend von 2-Mercaptoessigsäure und 2-Benzylacrylsäureäthylester, den (RS)-α-[[[(Morpholinocarbonyl)methyl]sulfonyl]methyl]hydrozimtsäureäthylester als farbloses Öl, MS : 383 (M)⁺, dessen enzymatische Verseifung, analog Beispiel 3, Absatz (c), die (S)-α-[[[(Morpholinocarbonyl)methyl]sulfonyl]methyl]hydrozimtsäure, MS: 355 (M)⁺, als farblosen Schaum lieferte.

### (S)-α-[[[(RS)-1-(Morpholinocarbonyl)äthyl]sulfonyl]methyl]hydrozimtsäure:

In analoger Weise wie in Beispiel 1, Absätze (d) und (e) und Beispiel 2, Absatz (a) beschrieben, erhielt man, ausgehend von Thiomilchsäure und 2-Benzylacrylsäureäthylester, den (RS)-α-[[[(RS)-1-(Morpholinocarbonyl)äthyl]sulfonyl]methyl]hydrozimtsäureäthylester, MS: 397 (M)⁺, als farbloses Öl, dessen enzymatische Verseifung, analog Beispiel 3, Absatz (c), die (S)-α-[[[(RS)-1-(Morpholinocarbonyl)äthyl]sulfonyl]methyl]hydrozimtsäure, MS: 369 (M)⁺, als farblosen Schaum lieferte.

### (S)-α-[[[1-Methyl-1-[(tetrahydro-4H-1,4-thiazin-4-yl)carbonyl]äthyl]sulfonyl]methyl]hydrozimtsäure:

In analoger Weise wie in Beispiel 1, Absätze (d) und (e) beschrieben, erhielt man, ausgehend von 2-Mercaptoisobuttersäure und 2-Benzylacrylsäureäthylester, das (RS)-2-[[2-[(Äthoxy)carbonyl]-3-phenylpropyl]sulfonyl]-2-methylpropionsäurechlorid, dessen Umsetzung mit Thiomorpholin, analog Beispiel 2, Absatz (a), und nachfolgende enzymatische Hydrolyse, analog Beispiel 3, Absatz (c), die (S)-α-[[[1-Methyl-1-[(tetrahydro-4H-1,4-thiazin-4-yl)carbonyl]äthyl]sulfonyl]methyl]hydrozimtsäure, MS: 399 (M)⁺, als farblosen Festkörper lieferte.

### (S)-α-[[[1-Methyl-1-[(tetrahydro-4'H-1,4-thiazin-4'-yl)carbonyl]äthyl]sulfonyl]methyl]hydrozimtsäure-1'1'-dioxid:

In analoger Weise wie in Beispiel 1, Absatz (d) beschrieben, wurde durch Oxidation von (S)-α-[[[1-Methyl-1-[(tetrahydro-4H-1,4-thiazin-4-yl)carbonyl]äthyl]sulfonyl]methyl]hydrozimtsäure mit Kaliummonopersulfattripelsalz das (S)-α-[[[1-Methyl-1-[(tetrahydro-4'H-1,4-thiazin-4'-yl)carbonyl]äthyl]sulfonyl]methyl]hydrozimtsäure-1',1'-dioxid, MS: 431 (M)⁺, als farbloser Festkörper erhalten.

### (S)-α-[[[1-Methyl-1-[(4-methyl-1-piperazinyl)carbonyl]äthyl]sulfonyl]methyl]hydrozimtsäure:

In analoger Weise wie in Beispiel 1, Absätze (d) und (e) beschrieben, erhielt man, ausgehend von 2-Mercaptoisobuttersäure und 2-Benzylacrylsäureäthylester, das (RS)-2-[[2-[(Äthoxy)carbonyl]-3-phenylpropyl]sulfonyl]-2-methylpropionsäurechlorid, dessen Umsetzung mit 1-Methylpiperazin, analog Beispiel 2, Absatz (a), und nachfolgende enzymatische Hydrolyse, analog Beispiel 3, Absatz (c), die (S)-α-[[[1-Methyl-1-[(4-methyl-1-piperazinyl)carbonyl]äthyl]sulfonyl]methyl]hydrozimtsäure, MS: 396 (M)⁺, als farblosen Festkörper lieferte.

### (S/R=9/1)-α-[[[1-[[2-(2-Hydroxyäthoxy)äthyl]carbamoyl]-1-methyläthyl]sulfonyl]methyl]hydrozimtsäure:

In analoger Weise wie in Beispiel 3, Absätze (a) bis (c) beschrieben, erhielt man, ausgehend von 2-Mercaptoisobuttersäure und 2-Benzylacrylsäureäthylester, die (RS)-2-[[2-[(Äthoxy)carbonyl]-3-phenylpropyl]sulfonyl]-2-methylpropionsäure, deren Umsetzung mit 2-(2-Aminoäthoxy)äthanol und nachfolgende enzymatische Hydrolyse des resultierenden Äthylesters die (S/R=9/1)-α-[[[1-[[2-(2-Hydroxyäthoxy)äthyl]carbamoyl]-1-methyläthyl]sulfonyl]methyl]hydrozimtsäure. MS: 401.9 (M+H)⁺, als farbloses Öl lieferte.

### Beispiel 11

Zu einer Lösung von 365 mg (0.5 mMol) N-[(S)-α-[[[1-Methyl-1-[methyl-[2-(2-pyridyl)äthyl]carbamoyl]äthyl]sulfonyl]methyl]hydrocinnamoyl]-3-(thiazol-4-yl)-D,L-alanin und 115 mg (0.5 mMol) (1S,2R,3S)-3-Amino-4-cyclohexyl-1-cyclopropyl-1,2-butandiol in 5 ml Acetonitril wurden 0.14 ml (1 mMol) Triäthylamin und 191 mg (0.5 mMol) HBTU gegeben und über Nacht bei Raumtemperatur gerührt. Die Reaktionslösung wurde unter vermindertem Druck eingedampft ,und der erhaltene Rückstand zwischen Essigsäureäthylester und gesättigter Natriumhydrogencarbonatlösung verteilt. Die wäßrige Phase wurde dreimal mit Essigsäureäthylester extrahiert. Die organischen Phasen wurden mit Wasser und gesättigter Natriumchloridlösung gewaschen, über Magnesiumsulfat getrocknet, filtriert und unter vermindertem Druck eingedampft. Chromatographie des Rückstandes an Kieselgel unter Verwendung eines 95:5-Gemisches von Methylenchlorid und Methanol lieferte 152 mg des weniger polaren (S oder R)-N-[(1S,2R,3S)-1-(Cyclohexylmethyl)-3-cyclopropyl-2,3-dihydroxypropyl]-α-[(S)-α-[[[1-methyl-1-[methyl-[2-(2-pyridyl)äthyl]carbamoyl]äthyl]sulfonyl]methyl]hydrocinnamamido]-3-(thiazol-4-yl)propionamids, MS: 797 (M+H)⁺ und 114 mg des polareren (R oder S)-N-[(1S,2R,3S)-1-(Cyclohexylmethyl)-3-cyclopropyl-2,3-dihydroxypropyl]-a-[(S)-a-[[[1-methyl-1-[methyl-[2-(2-pyridyl)äthyl]carbamoyl]äthyl]sulfonyl]methyl]hydrocinnamamido]-3-(thiazol-4-yl)propionamids, MS: 797 (M+H)⁺, jeweils als farbloser Schaum.

Das als Ausgangsstoff eingesetzte N-[(S)-α-[[[1-Methyl-1-[methyl-[2-(2-pyridyl)äthyl]carbamoyl]äthyl]sulfonyl]methyl]hydrocinnamoyl]-3-(thiazol-4-yl)-D,L-alanin wurde wie folgt hergestellt:

### (a) N-[(S)-α-[[[1-Methyl-1-[methyl-[2-(2-pyridyl)äthyl]carbamoyl]äthyl]sulfonyl]methyl]hydrocinnamoyl]-3-(thiazol-4-yl)-D,L-alaninmethylester:

Zu einer Lösung von 328 mg (0.7 mMol) (S)-α-[[[1-Methyl-1-[methyl-[2-(2-pyridyl)äthyl]carbamoyl]äthyl]sulfonyl]lmethyl]hydrozimtsäure und 181 mg (0.7 mMol) 3-(Thiazol-4-yl)-D,L-alaninmethylester-dihydrochlorid in 7 ml Acetonitril wurden 0.28 ml (2.1 mMol) Triäthylamin und 265 mg (0.7 mMol) HBTU gegeben und über Nacht bei Raumtemperatur gerührt. Eindampfen der Lösung unter vermindertem Druck und Chromatographie des Rückstandes an Kieselgel unter Verwendung eines 200:10:1-Gemisches von Methylenchlorid, Methanol und Ammoniak als Eluierungsmittel lieferte 309 mg N-[(S)-α-[[[1-Methyl-1-[methyl-[2-(2-pyridyl)äthyl]carbamoyl]äthyl]sulfonyl]methyl]hydrocinnamoyl]-3-(thiazol-4-yl)-D,L-alaninmethylester als farblosen Schaum, MS: 601 (M+H)⁺.

### (b) N-[(S)-α-[[[1-Methyl-1-[methyl-[2-(2-pyridyl)äthyl]carbamoyl]äthyl]sulfonyl]methyl]hydrocinnamoyl]-3-(thiazol-4-yl)-D,L-alanin:

Eine Lösung von 300 mg N-[(S)-α-[[[1-Methyl-1-[methyl-[2-(2-pyridyl)äthyl] carbamoyl]äthyl]sulfonyl]methyl]hydrocinnamoyl]-3-(thiazol-4-yl)-D,L-alaninmethylester in 2.5 ml Dioxan wurde mit einer Lösung von 24.3 mg Lithiumhydroxid-hydrat in 1 ml Wasser versetzt und 3 Stunden bei 5° gerührt. Anschliessend wurde die Lösung zwischen Essigsäureäthylester und Wasser verteilt, und die organische Phase dreimal mit Wasser extrahiert. Die wässerigen Extrakte wurden mit Essigsäureäthylester und mit 2N Salzsäure auf pH 2 gestellt und unter vermindertem Druck eingedampft. Man erhielt 365 mg N-[(S)-α-[[[1-Methyl-1-[methyl-[2-(2-pyridyl)äthyl]carbamoyl]äthyl]-sulfonyl]methyl]hydrocinnamoyl]-3-(thiazol-4-yl)-D,L-alanin als farblosen Schaum, welcher direkt in die nächste Stufe eingesetzt wurde.

### Beispiel 12

In analoger Weise wie in Beispiel 11, Absätze (a) und (b) beschrieben, erhielt man aus α-[[[1-Methyl-1-(morpholinocarbonyl)äthyl]sulfonyl]methyl]hydrozimtsäure und 3-(Thiazol-4-yl)-D,L-alaninmethylester-dihydrochlorid den N-[(S)-α-[[[1-Methyl-1-(morpholinocarbonyl)äthyl]sulfonyl]methyl]hydrocinnamoyl]-3-(thiazol-4-yl)-D,L-alaninmethylester, welcher mit Lithiumhydroxid zur entsprechenden Säure verseift wurde. Die Kopplung mit (1S,2R,3S)-3-Amino-4-cyclohexyl-1-cyclopropyl-1,2-butandiol und anschließende Chromatographie lieferte das (R oder S)-N-[(1S,2R,3S)-1-(Cyclohexylmethyl)-3-cyclopropyl-2,3-dihydroxypropyl]-α-[(S)-α-[[[1-methyl-1-(morpholinocarbonyl)äthyl]sulfonyl]methyl]hydrocinnamamido]-3-(thiazol-4-yl)propionamid, MS 747 (M+H)⁺ und das (S oder R)-N-[(1S,2R,3S)-1-(Cyclohexylmethyl)-3-cyclopropyl-2,3-dihydroxypropyl]-α-[(S)-α-[[[1-methyl-1-(morpholinocarbonyl)äthyl]sulfonyl]methyl]hydrocinnamamido]-3-(thiazol-4-yl)propionamid, MS 747 (M+H)⁺, jeweils als farblosen Schaum.

### Beispiel 13

In analoger Weise wie in Beispiel 1 beschrieben, wurde die folgende Verbindung hergestellt:
- Aus (S)-α-Amino-N-[(1S,2R,3S)-1-benzyl-3-cyclopropyl-2,3-dihydroxypropyl]-4-imidazolpropionamid und α-[[[1-Methyl-1-(morpholinocarbonyl)äthyl]sulfonyl]methyl]hydrozimtsäure das (S)-N-[(1S,2R,3S)-1-Benzyl-3-cyclopropyl-2,3-dihydroxypropyl]-α-[(S)-α-[[[1-methyl-1-(morpholinocarbonyl)äthyl]sulfonyl]methyl]hydrocinnamamido]imidazol-4-propionamid, MS 724 (M+H)⁺, als farbloser Schaum.

Das als Ausgangsstoff verwendete (S)-α-Amino-N-[(1S,2R,3S)-1-benzyl-3-cyclopropyl-2,3-dihydroxypropyl]imidazol-4-propionamid wurde wie folgt hergestellt:

### (a) tert-Butyl [(1S,2R)-1-benzyl-2-(cyclopropylcarbonyl)-2-hydroxyäthyl]carbamat:

Zu 3.03 g (124.5 mMol) Magnesium in 10 ml abs. Äther tropfte man unter leichtem Rückfluß 9.96 ml (124.5 mMol) Cyclopropylbromid in 100 ml Äther innert 30 Minuten und erhitzte anschließend 2 1/2 Stunden zum Rückfluß. Anschließend fügte man innerhalb von 45 Minuten 6.25 g (22.63 mMol) tert-Butyl [(1S,2R)-1-benzyl-2-cyano-2-hydroxyäthyl]carbamat (EPA 0.266.950) unter Rückfluß tropfenweise zu und erhitzte noch weitere 2 1/2 Stunden zum Rückfluß. Schließlich ließ man abkühlen (10° ), tropfte 10%ige Zitronensäure zu und extrahierte zweimal mit Äther. Die organische Phase wurde mit Wasser und gesättigter Kochsalzlösung gewaschen, über Magnesiumsulfat getrocknet, filtriert und unter vermindertem Druck eingedampft. Chromatographie an Kieselgel unter Verwendung eines 9:1-Gemisches von Toluol und Essigester lieferte das tert-Butyl [(1S,2R)-1-benzyl-2-(cyclopropylcarbonyl)-2-hydroxyäthyl]carbamat als hellgelben Feststoff, MS: 246 (M-C₃H₉O)⁺.

### (b) tert-Butyl [(1S,2R,3S)-1-benzyl-3-cyclopropyl-2,3-dihydroxypropyl]carbamat:

Eine Lösung von 2.83 g (8.77 mMol) tert-Butyl [(1S,2R)-1-benzyl-2-(cyclopropylcarbonyl)-2-hydroxyäthyl]carbamat löste man in 130 ml Methylenchlorid, fügte 3 ml Essigsäure hinzu und versetzte schließlich portionsweise bei 0 - 10° mit 0.332 g (8.78 mMol) Natriumborhydrid. Man rührte weitere 2 Stunden bei 5° und verteilte dann die Reaktionslösung zwischen 2N Natriumhydrogencarbonatlösung und Methylenchlorid. Die organische Phase wurde abgetrennt, mit Wasser und mit gesättigter Kochsalzlösung gewaschen, über Magnesiumsulfat getrocknet, filtiert und unter vermindertem Druck eingedampft. Kristallisation des Rückstandes aus Äther/Hexan lieferte 2.1 g tert-Butyl [(1S,2R,3S)-1-benzyl-3-cyclopropyl-2,3-dihydroxypropyl]carbamat als farblose Nadeln, Smp. 83-85°.

### (c) (1S,2R,3S)-3-Amino-1-cyclopropyl-4-phenyl-1,2-butandiol:

Eine Lösung von 2.0 g (6.23 mMol) tert-Butyl [(1S,2R,3S)-1-benzyl-3-cyclopropyl-2,3-dihydroxypropyl]carbamat in 20 ml Methanol versetzte man mit 20 ml 2N Salzsäure und erhitzte die Lösung 90 Minuten auf 50°. Nach dem Abkühlen neutralisierte man durch Zugabe von 40 ml 1N Natronlauge und dampfte am Rotationsverdampfer unter vermindertem Druck zur Trockene ein. Den Rückstand verteilte man zwischen Wasser und Methylenchlorid. Die organische Phase wurde mit Wasser und gesättigter Kochsalzlösung gewaschen, über Magnesiumsulfat getrocknet, filtriert und unter vermindertem Druck eingedampft. Man erhielt 0.7 g (1S,2R,3S)-3-Amino-1-cyclopropyl-4-phenyl-1,2-butandiol als weißen, kristallinen Feststoff, MS: 150 (M-C₄H₈O)⁺.

### (d) (S)-α-Amino-N-[(1S,2R,3S)-1-benzyl-3-cyclopropyl-2,3-dihydroxypropyl]imidazol-4-propionamid:

In analoger Weise wie in Beispiel 1, Absatz (c) beschrieben, erhielt man durch Kondensation von FMOC-His(Fmoc)-OH und (1S,2R,3S)-3-Amino-1-cyclopropyl-4-phenyl-1,2-butandiol, gefolgt von der Abspaltung der Schutzgruppen mittels Piperidin in Methylenchlorid, das (S)-α-Amino-N-[(1S,2R,3S)-1-benzyl-3-cyclopropyl-2,3-dihydroxypropyl]imidazol-4-propionamid als farblosen Schaum, MS: 359 (M+H)⁺.

### Beispiel 14

In analoger Weiser wie in Beispiel 1 beschrieben, wurde duch Kondensation von (RS)-α-[[[1-[(cis-2,6-Dimethylmorpholino)carbonyl]-1-methyläthyl]sulfonyl]methyl]hydrozimtsäure und (S)-α-Amino-N-[(1S,2R,3S)-1-(cyclohexylmethyl)-3-cyclopropyl-2,3-dihydroxypropyl]imidazol-4-propionamid das weniger polare (S)-N-[(1S,2R,3S)-1-(Cyclohexylmethyl)-3-cyclopropyl-2,3-dihydroxypropyl]-α-[(R oder S)-α-[[[1-[(cis-2,6-dimethylmorpholino)carbonyl]-1-methyläthyl]sulfonyl]methyl]hydrocinnamamido]imidazol-4-propionamid, MS: 758 (M+H)⁺, und das polarere (S)-N-[(1S,2R,3S)-1-(Cyclohexylmethyl)-3-cyclopropyl-2,3-dihydroxypropyl]-α-[(S oder R)-α-[[[1-[(cis-2,6-dimethylmorpholino)carbonyl]-1-methyläthyl]sulfonyl]methyl]hydrocinnamamido]imidazol-4-propionamid, MS: 758 (M+H)⁺, jeweils als farbloser Schaum erhalten.

Die als Ausgangsstoff eingesetzte (RS)-α-[[[1-[(cis-2,6-Dimethylmorpholino)carbonyl]-1-methyläthyl]sulfonyl]methyl]hydrozimtsäure wurde wie folgt hergestellt:

In analoger Weise wie in Beispiel 2, Absätze (c) und (d) beschrieben, wurde durch Umsetzung von (RS)-2-[[2-[(Benzyloxy)carbonyl]-3-phenylpropyl]sulfonyl]-2-methylpropionsäurechlorid mit 2,6-Dimethylmorpholin in Pyridin, gefolgt von einer chromatographischen Trennung der erhaltenen Diastereomeren, der (RS)-α-[[[1-[(cis-2,6-Dimethylmorpholino)carbonyl]-1-methyläthyl]sulfonyl]methyl]hydrozimtsäurebenzylester, MS: 501 (M)⁺, als farbloses Öl erhalten.

Die anschließende katalytische Hydrierung lieferte die (RS)-α-[[[1-[(cis-2,6-Dimethylmorpholino)carbonyl]-1-methyläthyl]sulfonyl]methyl]hydrozimtsäure, MS: 411 (M)⁺, als farblosen Schaum.

### Beispiel 15

In analoger Weise wie in Referenzbeispiel 2a beschrieben, wurden durch Abspaltung der Boc-Schutzgruppe die folgenden Verbindungen erhalten, wobei auf eine basische Aufarbeitung verzichtet wurde:
- Aus 4-[2-[[(S oder R)-2-[[(S)-1-[[(1S,2R,3S)-1-(Cyclohexylmethyl)-3-cyclopropyl-2,3-dihydroxypropyl]carbamoyl]-2-imidazol-4-yläthyl]carbamoyl]-3-phenylpropyl]sulfonyl]-2-methylpropionyl]-1-piperazincarbonsäure-tert-butylester (siehe Beispiel 2) das (S)-N-[(1S,2R,3S)-1-(Cyclohexylmethyl)-3-cyclopropyl-2,3-dihydroxypropyl]-α-[(S oder R)-α-[[[1-methyl-1-(1-piperazinylcarbonyl)äthyl]sulfonyl]methyl]hydrocinnamamido]imidazol-4-propionamid-dihydrochlorid als farbloser Festkörper; MS: 729 (M+H)⁺ (Base);
- aus 4-[2-[[(R oder S)-2-[[(S)-1-[[1S,2R,3S)-1-(Cyclohexylmethyl)-3-cyclopropyl-2,3-dihydroxypropyl]carbamoyl]-2-imidazol-4-yläthyl]carbamoyl]-3-phenylpropyl]sulfonyl]-2-methylpropionyl]-1-piperazincarbonsäure-tert-butylester (siehe Beispiel 2) das (S)-N-[(1S,2R,3S)-1-(Cyclohexylmethyl)-3-cyclopropyl-2,3-dihydroxypropyl]-α-[(R oder S)-α-[[[1-methyl-1-(1-piperazinylcarbonyl)äthyl]sulfonyl]methyl]hydrocinnamamido]imidazol-4-propionamid-dihydrochlorid als farbloser Festkörper; MS: 729 (M+H)⁺ (Base).

### Beispiel A

### Orale, wässrige Suspension

| Zusammensetzung: | |
|---|---|
| 2-[2-[[(S)-2-[[(S)-1-[[(1S,2R,3S)-1-(Cyclohexylmethyl)-3-cyclopropyl-2,3-dihydroxypropyl]carbamoyl]-2-imidazol-4-yläthyl]carbamoyl]-3-phenylpropyl]sulfonyl]-2-methylpropionamido]-2-deoxy-D-glucopyranose mikronisiert | 5.0 g |
| Polysorbat 80 | 0.3 g |
| Hydroxypropylmethylcellulose | 1.0g |
| Geschmackstoff | q.s. |
| Methylparaben | 0.2 g |
| Propylenparaben | 0.04 g |
| Wasser | ad 100.0 ml |

### Beispiel B

### Orale, wässrige Lösung

| Zusammensetzung: | |
|---|---|
| 2-[2-[[(S)-2-[[(S)-1-[[(1S,2R,3S)-1-(Cyclohexylmethyl)-3-cyclopropyl-2,3-dihydroxypropyl]carbamoyl]-2-imidazol-4-yläthyl]carbamoyl]-3-phenylpropyl]sulfonyl]-2-methylpropionamido]-2-deoxy-D-glucopyranosemethansulfonat | 1.0 g |
| Methylparaben | 0.2 g |
| Propylparaben | 0.04 g |
| Geschmackstoff | q.s. |
| Wasser | ad 100.0 ml |

### Beispiel C

### Tabletten

| Zusammensetzung: | |
|---|---|
| 1) 2-[2-[[(S)-2-[[(S)-1-[[(1S,2R,3S)-1-(Cyclohexylmethyl)-3-cyclopropyl-2,3-dihydroxypropyl]carbamoyl]-2-imidazol-4-yläthyl]carbamoyl]-3-phenylpropyl]sulfonyl]-2-methylpropionamido]-2-deoxy-D-glucopyranosemethansulfonat | 200 mg |
| 2) Milchzucker wasserfrei | 160 mg |
| 3) Hydroxypropylmethylcellulose | 18 mg |
| 4) Natrium-carboxymethylcellulose | 20 mg |
| 5) Magnesiumstearat | 2 mg |
| | Tablettengewicht 400 mg |

### Herstellungsverfahren:

1) und 2) werden intensiv gemischt. Die Mischung wird danach mit einer wäßrigen Lösung von 3) befeuchtet und geknetet, und die resultierende Masse granuliert, getrocknet und gesiebt. Das Granulat wird mit 4) und 5) gemischt und zu Tabletten geeigneter Größe verpreßt.

### Beispiel D

### Kapseln

| Zusammensetzung: | |
|---|---|
| 1) 2-[2-[[(S)-2-[[(S)-1-[[(1S,2R,3S)-1-(Cyclohexylmethyl)-3-cyclopropyl-2,3-dihydroxypropyl]carbamoyl]-2-imidazol-4-yläthyl]carbamoyl]-3-phenylpropyl]sulfonyl]-2-methylpropionamido]-2-deoxy-D-glucopyranosemethansulfonat | 200 mg |
| 2) Milchzucker wasserfrei | 160 mg |
| 3) Hydroxypropylmethylcellulose | 18 mg |
| 4) Natrium-carboxymethylcellulose | 20 mg |
| 5) Magnesiumstearat | 2 mg |
| | Kapselfüllgewicht 400 mg |

### Herstellungsverfahren:

1) und 2) werden intensiv gemischt. Die Mischung wird danach mit einer wäßrigen Lösung von 3) befeuchtet und geknetet, und die resultierende Masse granuliert, getrocknet und gesiebt. Das Granulat wird mit 4) und 5) gemischt, das Gemisch in Kapseln geeigneter Größe abgefüllt.

### Beispiel E

### Injektionslösung

| Zusammensetzung: | 1 ml |
|---|---|
| 2-[2-[[(S)-2-[[(S)-1-[[(1S,2R,3S)-1-(Cyclohexylmethyl)-3-cyclopropyl-2,3-dihydroxypropyl]carbamoyl]-2-imidazol-4-yläthyl]carbamoyl]-3-phenylpropyl]sulfonyl]-2-methylpropionamido]-2-deoxy-D-glucopyranosemethansulfonat | 20 mg |
| D-Mannit pyrogenfrei | 10 mg |
| Wasser zu Injektionszwecken | ad 1.0 ml |

### Herstellungsverfahren:

Der Wirkstoff und das Mannit werden in Stickstoff-begastem Wasser gelöst und anschließend nach einem üblichen Verfahren lyophilisiert.

### Beispiel F

Wenn man nach den in den Beispielen A-E beschriebenen Verfahren arbeitet, können aus den folgenden, ebenfalls bevorzugten Verbindungen und ihren pharmazeutisch verwendbaren Salzen entsprechende galenische Präparate hergestellt werden:
(S)-N-[(1S,2R,3S)-1-(Cyclohexylmethyl)-3-cyclopropyl-2,3-dihydroxypropyl]-α-[(S)-α-[[[1-(morpholinocarbonyl)-1-methyläthyl]sulfonyl]methyl]hydrocinnamamido]imidazol-4-propionamid,
(S)-N-[(1S,2R,3S)-1-(Cyclohexylmethyl)-3-cyclopropyl-2,3-dihydroxypropyl]-α-[(S)-α-[[[1-[(4-hydroxypiperidino)carbonyl]-1-methyläthyl]sulfonyl]methyl]hydrocinnamamido]imidazol-4-propionamid,
(S oder R)-N-[(1S,2R,3S)-1-(Cyclohexylmethyl)-3-cyclopropyl-2,3-dihydroxypropyl]-α-[(S)-α-[[[1-methyl-1-[morpholinocarbonyl]äthyl]sulfonyl]methyl]hydrocinnamamido]-3-(thiazolyl-4-yl)propionamid,

## Patentansprüche

1. Aminosäurederivate der allgemeinen Formel 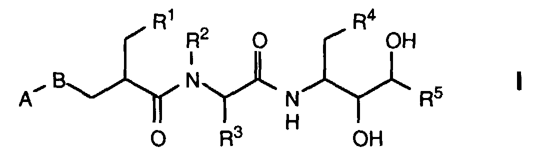
worin R¹ C₃-C₆-Cycloalkyl, Phenyl, gegebenenfalls ein- oder mehrfach substituiert durch C₁-C₆-Alkyl, C₁-C₆-Alkoxy, C₁-C₆-Alkylcarbonyloxy, Hydroxy, Halogen oder Trifluormethyl, R¹ auch Naphthyl, Thienyl, Pyridyl, Chinolyl, Isochinolyl oder Benzyl, R² Wasserstoff oder Methyl, R³ Wasserstoff, Hydroxy, C₁-C₆-Alkyl, C₁-C₆-Alkoxy, C₃-C₆-Alkenyloxy, C₁-C₆-Alkylthio, C₁-C₆-Alkylthio-C₁-C₆-alkyl, C₁-C₆-Alkoxycarbonyl, Imidazol-1-ylmethyl, Imidazol-2-ylmethyl oder Imidazol-4-ylmethyl, welche an einem Kohlenstoff- und/oder Stickstoffatom methyliert sein können, 2-Aminoimidazol-4-yl-methyl, 5-Jodimidazol-4-ylmethyl, Pyrazol-1-ylmethyl, Pyrazol-3-ylmethyl, Thiazol-2-ylmethyl, Thiazol-4-ylmethyl, Thiazol-5-ylmethyl, 2-Aminothiazol-4-ylmethyl, Thienylmethyl, Furanylmethyl, Pyridylmethyl, Aminocarbonyl, Aminocarbonyl-C₁-C₆-alkyl oder C₁-C₆-Alkoxycarbonyl-C₁-C₆-alkyl, R⁴ C₃-C₆-Cycloalkyl, gegebenenfalls substituiert durch C₁-C₆-Alkyl, Hydroxy oder Halogen, R⁴ weiterhin Phenyl oder Halophenyl, R⁵ C₃-C₆-Cycloalkyl, C₃-C₆-Cycloalkyl-C₁-C₆-alkyl oder C₁-C₆-Alkyl, B ein Schwefelatom oder eine Sulfinyl- oder Sulfonylgruppe und A die Gruppe 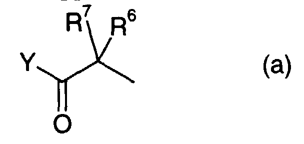 bedeuten, in welcher R⁶ und R⁷ unabhängig voneinander je Wasserstoff oder C₁-C₆-Alkyl und Y C₃-C₆-Cycloalkylamino, Sulfo-C₁-C₆-alkylamino, Bis-C₁-C₆-alkoxy-C₁-C₆-alkylamino, oder Pyridyl-C₁-C₆-alkylamino oder Morpholino-C₁-C₆-alkylamino, welche jeweils an der Aminogruppe durch C₁-C₆-Alkyl substituiert sein können, Pyrazinyl-C₁-C₆-alkylamino, welches am Stickstoffatom und/oder an der Aminogruppe durch C₁-C₆-Alkyl substituiert sein kann, 4-Piperidinyl-C₁-C₆-alkylamino, welches am Stickstoffatom durch C₁-C₆-Alkyl, Benzyl, Benzyloxycarbonyl, C₁-C₆-Alkoxycarbonyl oder Hydroxy-C₁-C₆-alkyl und/oder an der Aminogruppe durch C₁-C₆-Alkyl substituiert sein kann, C₁-C₆-Alkoxycarbonyl-C₁-C₆-alkylamino, Hydroxy-C₁-C₆-alkoxy-C₁-C₆-alkylamino, Hydroxy-C₁-C₆-alkylamino, Bishydroxy-C₁-C₆-alkylamino, den Rest eines natürlichen Aminodesoxyzuckers, oder die Gruppe (R^{a})(R^{b})N- bedeutet, in welcher R^{a} und R^{b} unabhängig voneinander je Wasserstoff oder C₁-C₆-Alkyl oder zusammen mit dem Stickstoffatom, an welches sie gebunden sind, einen gesättigten 5- oder 6-gliedrigen heterocyclischen Ring bedeuten, welcher ein zusätzliches Stickstoffatom oder ein Sauerstoff- oder Schwefelatom als weiteres Heteroatom enthalten oder eine Oxogruppe aufweisen kann, wobei das Schwefelatom auch in Form einer Sulfinyl- oder Sulfonylgruppe vorliegen kann, oder an einem oder zwei Kohlenstoffatomen durch C₁-C₆-Alkyl, Hydroxy, Hydroxy-C₁-C₆-alkyl oder C₁-C₆-Alkoxy-C₁-C₆-alkoxy oder am allfällig vorhandenen zweiten Stickstoffatom durch C₁-C₆-Alkyl, Benzyl, Benzyloxycarbonyl, C₁-C₆-Alkoxycarbonyl oder Hydroxy-C₁-C₆-alkyl substituiert sein kann, oder in welchem das allfällig vorhandene zweite Stickstoffatom ein Sauerstoffatom tragen kann,
in Form von optisch reinen Diastereomeren, Diastereomerengemische, diastereomeren Racematen oder Gemischen von diastereomeren Racematen sowie pharmazeutisch verwendbare Salze dieser Verbindungen.

2. Verbindungen gemäss Anspruch 1, worin R¹ C₃-C₆-Cycloalkyl, Phenyl, gegebenenfalls ein- oder mehrfach substituiert durch C₁-C₆-Alkyl, C₁-C₆-Alkoxy, C₁-C₆-Alkylcarbonyloxy, Hydroxy, Halogen oder Trifluormethyl, R¹ auch Naphthyl, Thienyl, Pyridyl, Chinolyl, Isochinolyl oder Benzyl, R² Wasserstoff oder Methyl, R³ Wasserstoff, Hydroxy, C₁-C₆-Alkyl, C₁-C₆-Alkoxy, C₃-C₆-Alkenyloxy, C₁-C₆-Alkylthio, C₁-C₆-Alkylthio C₁-C₆-alkyl, C₁-C₆-Alkoxycarbonyl, Imidazol-2-ylmethyl oder Imidazol-4-ylmethyl, welche an einem Kohlenstoff- und/oder Stickstoffatom methyliert sein können, 2-Aminoimidazol-4-yl-methyl, 5-Jodimidazol-4-ylmethyl, Thiazol-4-ylmethyl, Thienylmethyl, Furanylmethyl, Pyridylmethyl, Aminocarbonyl, Aminocarbonyl-C₁-C₆-Alkyl oder C₁-C₆-Alkoxycarbonyl-C₁-C₆-alk yl, R⁴ C₃-C₆- Cycloalkyl, gegebenenfalls substituiert durch C₁-C₆-Alkyl, Hydroxy oder Halogen, R⁴ weiterhin Phenyl oder Halophenyl, R⁵ C₃-C₆-Cycloalkyl, C₃-C₆-Cycloalkyl-C₁-C₆-alkyl oder C₁-C₆-Alkyl, B ein Schwefelatom oder eine Sulfinyl- oder Sulfonylgruppe, R⁶ und R⁷ unabhängig voneinander je Wasserstoff oder C₁-C₆-Alkyl und Y C₃-C₆-Cycloalkylamino, Sulfo-C₁-C₆-alkylamino, Bis-C₁-C₆-alkoxy-C₁-C₆-alkylamino, Pyridyl-C₁-C₆-alkylamino, 4-Piperidinyl-C₁-C₆-alkylamino, welches am Stickstoffatom durch C₁-C₆-Alkyl, Benzyl, Benzyloxycarbonyl, C₁-C₆-Alkoxycarbonyl oder Hydroxy-C₁-C₆-alkyl substituiert sein kann, C₁-C₆-Alkoxycarbonyl-C₁-C₆-alkylamino, Hydroxy-C₁-C₆-alk ylamino, Bishydroxy-C₁-C₆-alkylamino, den Rest eines natürlichen Aminodesoxyzuckers oder die Gruppe (R^{a})(R^{b})N- bedeutet, in welcher R^{a} und R^{b} unabhängig voneinander je Wasserstoff oder C₁-C₆-Alkyl oder zusammen mit dem Stickstoffatom, an welches sie gebunden sind, einen gesättigten 5- oder 6-gliedrigen heterocyclischen Ring bedeuten, welcher ein zusätzliches Stickstoffatom oder ein Sauerstoff- oder Schwefelatom als weiteres Heteroatom enthalten kann, wobei das Schwefelatom auch in Form einer Sulfinyl- oder Sulfonylgruppe vorliegen kann, an einem oder zwei Kohlenstoffatomen durch C₁-C₆-Alkyl, Hydroxy, Hydroxy-C₁-C₆-alkyl oder C₁-C₆-Alkoxy-C₁-C₆-alkoxy, am allfällig vorhandenen zweiten Stickstoffatom durch C₁-C₆-Alkyl, Benzyl, Benzyloxycarbonyl, C₁-C₆-Alkoxycarbonyl oder Hydroxy-C₁-C₆-alkyl substituiert sein kann, oder eine Oxogruppe aufweisen kann.

3. Verbindungen gemäss Anspruch 1 oder 2, worin R¹ C₃-C₆-Cycloalkyl, Phenyl, gegebenenfalls ein- oder mehrfach substituiert durch C₁-C₆-Alkyl, C₁-C₆-Alkoxy, C₁-C₆-Alkylcarbonyloxy, Hydroxy, Halogen oder Trifluormethyl, R¹ auch Naphthyl, Thienyl oder Pyridyl bedeutet.

4. Verbindungen gemäss Anspruch 3, worin R¹ Cyclohexyl, Phenyl, Naphthyl oder Thienyl bedeutet.

5. Verbindungen gemäss Anspruch 3, worin R¹ Phenyl bedeutet.

6. Verbindungen gemäss einem der Ansprüche 1-5, worin R² Wasserstoff bedeutet.

7. Verbindungen gemäss einem der Ansprüche 1-6, worin R³ Wasserstoff, Hydroxy, C₁-C₆-Alkyl, C₁-C₆-Alkoxy, Allyloxy, C₁-C₆-Alkylthio, C₁-C₆-Alkylthiomethyl, C₁-C₆-Alkoxycarbonyl, Imidazol-1-ylmethyl, Imidazol-4-ylmethyl, 2-Aminoimidazol-4-ylmethyl, 5-Jodimidazol-4-ylmethyl, Pyrazol-1-ylmethyl, Thiazol-4-ylmethyl, Thien-2-ylmethyl, Furan-2-ylmethyl, Pyridylmethyl oder Aminocarbonyl bedeutet.

8. Verbindungen gemäss Anspruch 7, worin R³ Wasserstoff, Hydroxy, C₁-C₄-Alkyl, C₁-C₄-Alkoxy, Allyloxy, C₁-C₄-Alkylthio, C₁-C₄-Alkylthiomethyl, C₁-C₄-Alkoxycarbonyl, Imidazol-4-ylmethyl, Thiazol-4-ylmethyl oder Pyridylmethyl bedeutet.

9. Verbindungen gemäss Anspruch 7, worin R³ Imidazol-4-ylmethyl oder 3-Pyridylmethyl bedeutet.

10. Verbindungen gemäss einem der Ansprüche 1-9, worin R⁴ C₃-C₆-Cycloalkyl oder Phenyl bedeutet.

11. Verbindungen gemäss Anspruch 10, worin R⁴ Cyclohexyl bedeutet.

12. Verbindungen gemäss einem der Ansprüche 1-11, worin R⁵ C₃-C₆-Cycloalkyl oder C₁-C₆-Alkyl bedeutet.

13. Verbindungen gemäss Anspruch 12, worin R⁵ Cyclopropyl, Isopropyl oder Isobutyl bedeutet.

14. Verbindungen gemäss Anspruch 12, worin R⁵ Cyclopropyl bedeutet.

15. Verbindungen gemäss einem der Ansprüche 1-14, worin B Sulfonyl bedeutet.

16. Verbindungen gemäss einem der Ansprüche 1-15, worin R⁶ und R⁷ je Wasserstoff oder C₁-C₄-Alkyl bedeuten.

17. Verbindungen gemäss Anspruch 16, worin R⁶ und R⁷ je Methyl bedeuten.

18. Verbindungen gemäss einem der Ansprüche 1-17, worin Y C₃-C₆-Cycloalkylamino, Sulfo-C₁-C₆-alkylamino, Bis-C₁-C₆-alkoxy-C₁-C₆-alkylamino, Pyridyl-C₁-C₆-alkylamino, welches an der Aminogruppe durch C₁-C₆-Alkyl substituiert sein kann, 4-Piperidinyl-C₁-C₆-alkylamino, welches am Stickstoffatom durch C₁-C₆-Alkyl, Benzyl, Benzyloxycarbonyl, C₁-C₆-Alkoxycarbonyl oder Hydroxy-C₁-C₆-alkyl substituiert sein kann, C₁-C₆-Alkoxycarbonyl-C₁-C₆-alkylamino, Hydroxy-C₁-C₆-alkoxy-C₁-C₆-alkylamino, Hydroxy-C₁-C₆-alkylamino, Bishydroxy-C₁-C₆-alkylamino oder die Gruppe (R^{a})(R^{b})N- bedeutet, in welcher R^{a} und R^{b} unabhängig voneinander je Wasserstoff oder C₁-C₆-Alkyl oder zusammen mit dem Stickstoffatom, an welches sie gebunden sind, einen gesättigten 5- oder 6-gliedrigen heterocyclischen Ring bedeuten, welcher ein zusätzliches Stickstoffatom oder ein Sauerstoff- oder Schwefelatom als weiteres Heteroatom enthalten oder eine Oxogruppe aufweisen kann, wobei das Schwefelatom auch in Form einer Sulfinyl- oder Sulfonylgruppe vorliegen kann, oder an einem oder zwei Kohlenstoffatomen durch C₁-C₆-Alkyl, Hydroxy, Hydroxy-C₁-C₆-alkyl oder C₁-C₆-Alkoxy-C₁-C₆-alkoxy, oder am allfällig vorhandenen zweiten Stickstoffatom durch C₁-C₆-Alkyl, Benzyl, Benzyloxycarbonyl, C₁-C₆-Alkoxycarbonyl oder Hydroxy-C₁-C₆-Alkyl substituiert sein kann bedeutet.

19. Verbindungen gemäss Anspruch 18, worin Y die Gruppe (R^{a})(R^{b})N- bedeutet, wobei R^{a} und R^{b} die in Anspruch 18 angegebene Bedeutung besitzen.

20. Verbindungen gemäss Anspruch 18, worin Y Pyrrolidinyl, Piperidinyl, Piperazinyl, Morpholinyl, Thiomorpholinyl, 2-Hydroxymethylpyrrolidinyl, 3-Hydroxypyrrolidinyl, 3,4-Dihydroxypyrrolidinyl, 4-Hydroxypiperidinyl, 4-Oxopiperidinyl, 3,5-Dimethylmorpholinyl, 4,4-Dioxothiomorpholinyl, 4-Oxothiomorpholinyl oder 2,6-Dimethylmorpholinyl bedeutet.

21. Verbindungen gemäss Anspruch 18, worin Y Morpholinyl bedeutet.

22. Verbindungen gemäss einem der Ansprüche 1-4, worin R¹ Cyclohexyl, Phenyl, Naphthyl oder Thienyl, R² Wasserstoff, R³ Wasserstoff, Hydroxy, C₁-C₄-Alkyl, C₁-C₄-Alkoxy, Allyloxy, C₁-C₄-Alkylthio, C₁-C₄-Alkylthiomethyl, C₁-C₄-Alkoxycarbonyl, Imidazol-4-ylmethyl, Thiazol-4-ylmethyl oder Pyridylmethyl, R⁴ Cyclohexyl, R⁵ Cyclopropyl, Isopropyl oder Isobutyl, B Sulfonyl, R⁶ und R⁷ je Methyl und Y Pyrrolidinyl, Piperidinyl, Piperazinyl, Morpholinyl, Thiomorpholinyl, 2-Hydroxymethylpyrrolidinyl, 3-Hydroxypyrrolidinyl, 3,4-Dihydroxypyrrolidinyl, 4-Hydroxypiperidinyl, 4-Oxopiperidinyl, 3,5-Dimethylmorpholinyl, 4,4-Dioxothiomorpholinyl, 4-Oxothiomorpholinyl oder 2,6-Dimethylmorpholinyl bedeuten.

23. Verbindungen gemäss Anspruch 1, ausgewählt aus (S)-N-[(1S,2R,3S)-1-(Cyclohexylmethyl)-3-cyclopropyl-2,3-dihydroxypropyl]-α-[(S)-α-[[[1-[[2-hydroxy-1-(hydroxymethyl)-1-methyläthyl]carbamoyl-1-methyläthyl]sulfonyl]methyl]hydrocinnamamido]imidazol-4-propionamid, (S)-α-[(R oder S)-α-[[[1-[(2-Pyridylmethyl)carbamoyl]-1-methyläthyl]sulfonyl]methyl]hydrocinnamamido]-N-1(1S,2R,3S)-1-(cyclohexylmethyl)-3-cyclopropyl-2,3-dihydroxypropyl]imidazol-4-propionamid oder (S)-α-[(R oder S)-α-[[[1-[(1-Benzyl-4-piperidinyl)carbamoyl]-1-methyläthyl]sulfonyl]methyl]hydrocinnamamido]-N-[(1S,2R,3S)-1-(cyclohexylmethyl)-3-cyclopropyl-2,3-dihydroxypropyl]imidazol-4-propionamid.

24. Verbindungen gemäss Anspruch 1, ausgewählt aus 2-[2-[[(S)-2-[[(S)-1-[[(1S,2R,3S)-1-(Cyclohexylmethyl)-3-cyclopropyl-2,3-dihydroxypropyl]carbamoyl]-2-imidazol-4-yläthyl]carbamoyl]-3-phenylpropyl]sulfonyl]-2-methylpropionamido]-2-deoxy-D-glucopyranose oder (S)-α-[(R oder S)-α-[[[1-[bis(2-Methoxyäthyl)carbamoyl]-1-methyläthyl]sulfonyl]methyl]hydrocinnamamido]-N-[(1S,2R,3S)-1-(cyclohexylmethyl)-3-cyclopropyl-2,3-dihydroxypropyl]imidazol-4-propionamid.

25. Verbindungen gemäss Anspruch 1, ausgewählt aus (S oder R)-N-[(1S,2R,3S)-1-(Cyclohexylmethyl)-3-cyclopropyl-2,3-dihydroxypropyl]-α-[(S)-α-[[[1-methyl-1-[morpholinocarbonyl]äthyl]sulfonyl]methyl]hydrocinnamamido]-3-(thiazolyl-4-yl)propionamid,

26. Verbindung gemäss Anspruch 1 mit der Bezeichnung (S)-N-[(1S,2R,3S)-1-(Cyclohexylmethyl)-3-cyclopropyl-2,3-dihydroxypropyl]-α-[(S)-α-[[[1-(morpholinocarbonyl)-1-methyläthyl]sulfonyl]methyl]hydrocinnamamido]imidazol-4-propionamid.

27. Verbindung gemäss Anspruch 1 mit der Bezeichnung (S)-N-[(1S,2R,3S)-1-(Cyclohexylmethyl)-3-cyclopropyl-2,3-dihydroxypropyl]-α-[(S)-α-[[[1-[(4-hydroxypiperidino)carbonyl]-1-methyläthyl]sulfonyl]methyl]hydrocinnamamido]imidazol-4-propionamid.

28. Verbindungen gemäss Anspruch 1, ausgewählt aus (S oder R)-N-[(1S,2R,3S)-1-(Cyclohexylmethyl)-3-cyclopropyl-2,3-dihydroxypropyl]-α-[(S)-α-[[[1-methyl-1-[morpholinocarbonyl]äthyl]sulfonyl]methyl]hydrocinnamamido]-3-(pyridin-3-yl)propionamid.

29. Verbindungen der allgemeinen Formeln 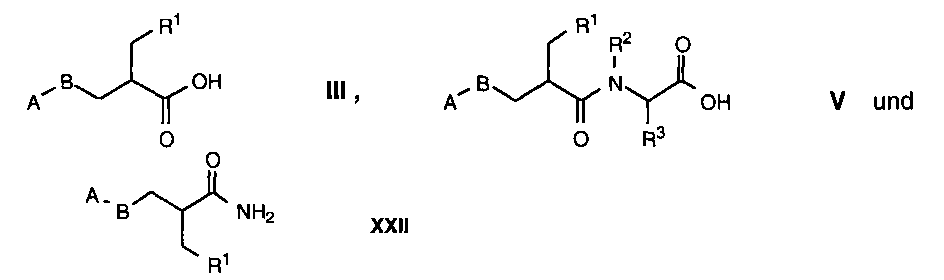 worin A die Gruppe 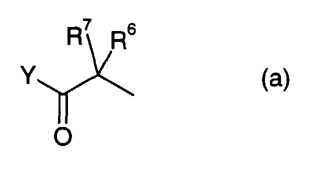 und B, Y, R¹, R², R³, R⁶ und R⁷ die in Anspruch 1 angegebene Bedeutung besitzen.

30. Aminosäurederivate gemäss einem der Ansprüche 1-27 zur Anwendung als therapeutische Wirkstoffe.

31. Aminosäurederivate gemäss einem der Ansprüche 1-27 zur Anwendung bei der Bekämpfung bzw. Verhütung von Bluthochdruck und Herzinsuffizienz.

32. Verfahren zur Herstellung einer Verbindung gemäss einem der Ansprüche 1-27, dadurch gekennzeichnet, dass man
a) eine Verbindung der allgemeinen Formel 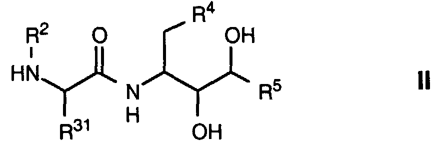
worin R³¹ Wasserstoff, C₁-C₆-Alkyl, C₁-C₆-Alkylthio-C₁-C₆-alkyl, C₁-C₆-Alkoxycarbonyl, Imidazol-1-ylmethyl, Imidazol-2-ylmethyl oder Imidazol-4-ylmethyl, welche an einem Kohlenstoff- und/oder Stickstoffatom methyliert sein können, 2-Aminoimidazol-4-yl-methyl, 5-Jodimidazol-4-ylmethyl, Pyrazol-1-ylmethyl, Pyrazol-3-ylmethyl, Thiazol-2-ylmethyl, Thiazol-4-ylmethyl, Thiazol-5-ylmethyl, 2-Aminothiazol-4-ylmethyl, Thienylmethyl, Furanylmethyl, Pyridylmethyl, Aminocarbonyl, Aminocarbonyl-C₁-C₆-alkyl oder C₁-C₆-Alkoxycarbonyl-C₁-C₆-alkyl bedeutet und R², R⁴ und R⁵ die in Anspruch 1 angegebene Bedeutung besitzen,
mit einer Säure der allgemeinen Formel 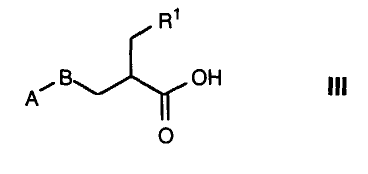
worin A, B und R¹ die in Anspruch 1 angegebene Bedeutung besitzen, oder einem aktivierten Derivat davon umsetzt, oder
b) eine Verbindung der allgemeinen Formel 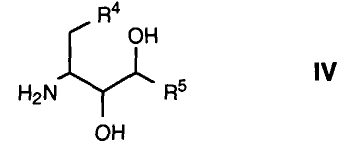
worin R⁴ und R⁵ die in Anspruch 1 angegebene Bedeutung besitzen, mit einer Säure der allgemeinen Formel 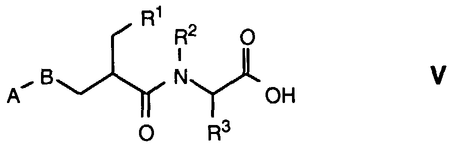
worin A, B, R¹, R² und R³ die in Anspruch 1 angegebene Bedeutung besitzen, oder einem aktivierten Derivat davon umsetzt, oder
c) zur Herstellung einer Verbindung der Formel I, worin A freie primäre oder sekundäre Aminogruppen und/oder freie primäre oder sekundäre Hydroxygruppen enthält, aus einer Verbindung der allgemeinen Formel 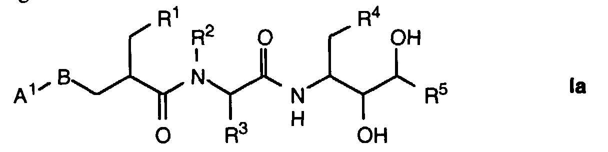
worin A¹ die gleiche Bedeutung wie A hat, mit der Maßgabe, daß A eine N- oder O-Schutzgruppe enthält,
die N- oder O-Schutzgruppe(n) abspaltet, und
d) erwünschtenfalls ein Gemisch diastereomerer Racemate in die diastereomeren Racemate oder optisch reinen Diastereomeren auftrennt, und/oder
e) erwünschtenfalls ein Diastereomerengemisch in die optisch reinen Diastereomeren auftrennt, und/oder
f) erwünschtenfalls eine erhaltene Verbindung in ein pharmazeutisch verwendbares Salz überführt.

33. Arzneimittel, enthaltend ein Aminosäurederivat gemäss einem der Ansprüche 1-27 und ein therapeutisch inertes Excipiens.

34. Mittel zur Bekämpfung bzw. Verhütung von Bluthochdruck und Herzinsuffizienz, enthaltend ein Aminosäurederivat gemäss einem der Ansprüche 1-27 und ein therapeutisch inertes Excipiens.

35. Verwendung eines Aminosäurederivates gemäss einem der Ansprüche 1-27 zur Herstellung von Mitteln gegen Bluthochdruck und/oder Herzinsuffizienz.

## Claims

1. Amino acid derivatives of the general formula 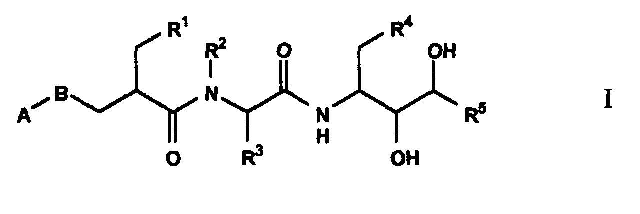
wherein R¹ signifies C₃-C₆-cycloalkyl, phenyl optionally mono- or multiply-substituted by C₁-C₆-alkyl, C₁-C₆-alkoxy, C₁-C₆-alkylcarbonyloxy, hydroxy, halogen or trifluoromethyl, R¹ also signifies naphthyl, thienyl, pyridyl, quinolyl, isoquinolyl or benzyl, R² signifies hydrogen or methyl, R³ signifies hydrogen, hydroxy, C₁-C₆-alkyl, C₁-C₆-alkoxy, C₃-C₆-alkenyloxy, C₁-C₆-alkylthio, C₁-C₆-alkylthio-C₁-C₆-alkyl, C₁-C₆-alkoxycarbonyl, imidazol-1-ylmethyl, imidazol-2-ylmethyl or imidazol-4-ylmethyl, which can be methylated on a carbon atom and/or nitrogen atom, 2-aminoimidazol-4-yl-methyl, 5-iodoimidazol-4-ylmethyl, pyrazol-1-ylmethyl, pyrazol-3-ylmethyl, thiazol-2-ylmethyl, thiazol-4-ylmethyl, thiazol-5-ylmethyl, 2-aminothiazol-4-ylmethyl, thienylmethyl, furanylmethyl, pyridylmethyl, aminocarbonyl, aminocarbonyl-C₁-C₆-alkyl or C₁-C₆-alkoxycarbonyl-C₁-C₆-alkyl, R⁴ signifies C₃-C₆-cycloalkyl optionally substituted by C₁-C₆-alkyl, hydroxy or halogen, R⁴ furthermore signifies phenyl or halophenyl, R⁵ signifies C₃-C₆-cycloalkyl, C₃-C₆-cycloalkyl-C₁-C₆-alkyl or C₁-C₆-alkyl, B signifies a sulphur atom or a sulphinyl or sulphonyl group and A signifies the group 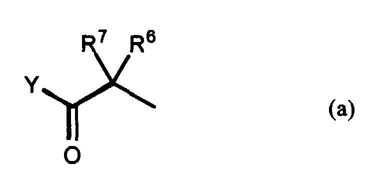 in which R⁶ and R⁷ each independently signify hydrogen or C₁-C₆-alkyl and Y signifies C₃-C₆-cycloalkylamino, sulpho-C₁-C₆-alkylamino, bis-C₁-C₆-alkoxy-C₁-C₆-alkylamino, or pyridyl-C₁-C₆-alkylamino or morpholino-C₁-C₆-alkylamino, each of which can be substituted on the amino group by C₁-C₆-alkyl, pyrazinyl-C₁-C₆-alkylamino, which can be substituted on the nitrogen atom and/or on the amino group by C₁-C₆-alkyl, 4-piperidinyl-C₁-C₆-alkylamino, which can be substituted on the nitrogen atom by C₁-C₆-alkyl, benzyl, benzyloxycarbonyl, C₁-C₆-alkoxycarbonyl or hydroxy-C₁-C₆-alkyl and/or on the amino group by C₁-C₆-alkyl, C₁-C₆-alkoxycarbonyl-C₁-C₆-alkylamino, hydroxy-C₁-C₆-alkoxy-C₁-C₆-alkylamino, hydroxy-C₁-C₆-alkylamino, bishydroxy-C₁-C₆-alkylamino, the residue of a natural aminodesoxy sugar, or the group (R^{a})(R^{b})N- in which R^{a} and R^{b} each independently signify hydrogen or C₁-C₆-alkyl or together with the nitrogen atom to which they are attached signify a saturated 5- or 6-membered heterocyclic ring which can contain an additional nitrogen atom or an oxygen or sulphur atom as a further hetero atom or which can carry an oxo group, whereby the sulphur atom can also be present in the form of a sulphinyl or sulphonyl group, or which can be substituted on one or two carbon atoms by C₁-C₆-alkyl, hydroxy, hydroxy-C₁-C₆-alkyl or C₁-C₆-alkoxy-C₁-C₆-alkoxy or on a second nitrogen atom which may be present by C₁-C₆-alkyl, benzyl, benzyloxycarbonyl, C₁-C₆-alkoxycarbonyl or hydroxy-C₁-C₆-alkyl, or in which the second nitrogen atom which may be present can carry an oxygen atom,
in the form of optically pure diastereomers, mixtures of diastereomers, diastereomeric racemates or mixtures of diastereomeric racemates as well as pharmaceutically usable salts of these compounds.

2. Compounds in accordance with claim 1, wherein R¹ signifies C₃-C₆-cycloalkyl, phenyl optionally mono- or multiply-substituted by C₁-C₆-alkyl, C₁-C₆-alkoxy, C₁-C₆-alkylcarbonyloxy, hydroxy, halogen or trifluoromethyl, R¹ also signifies naphthyl, thienyl, pyridyl, quinolyl, isoquinolyl or benzyl, R² signifies hydrogen or methyl, R³ signifies hydrogen, hydroxy, C₁-C₆-alkyl, C₁-C₆-alkoxy, C₃-C₆-alkenyloxy, C₁-C₆-alkylthio, C₁-C₆-alkylthio-C₁-C₆-alkyl, C₁-C₆-alkoxycarbonyl, imidazol-2-ylmethyl or imidazol-4-ylmethyl, which can be methylated on a carbon atom and/or nitrogen atom, 2-aminoimidazol-4-yl-methyl, 5-iodoimidazol-4-ylmethyl, thiazol-4-ylmethyl, thienylmethyl, furanylmethyl, pyridylmethyl, aminocarbonyl, aminocarbonyl-C₁-C₆-alkyl or C₁-C₆-alkoxycarbonyl-C₁-C₆-alkyl, R⁴ signifies C₃-C₆-cycloalkyl optionally substituted by C₁-C₆-alkyl, hydroxy or halogen, R⁴ furthermore signifies phenyl or halophenyl, R⁵ signifies C₃-C₆-cycloalkyl, C₃-C₆-cycloalkyl-C₁-C₆-alkyl or C₁-C₆-alkyl, B signifies a sulphur atom or a sulphinyl or sulphonyl group, R⁶ and R⁷ each independently signify hydrogen or C₁-C₆-alkyl and Y signifies C₃-C₆-cycloalkyl-amino, sulpho-C₁-C₆-alkylamino, bis-C₁-C₆-alkoxy-C₁-C₆-alkylamino, pyridyl-C₁-C₆-alkylamino, 4-piperidinyl-C₁-C₆-alkylamino, which can be substituted on the nitrogen atom by C₁-C₆-alkyl, benzyl, benzyloxycarbonyl, C₁-C₆-alkoxycarbonyl or hydroxy-C₁-C₆-alkyl, C₁-C₆-alkoxycarbonyl-C₁-C₆-alkylamino, hydroxy-C₁-C₆-alkylamino, bishydroxy-C₁-C₆-alkylamino, the residue of a natural aminodesoxy sugar, or the group (R^{a})(R^{b})N- in which R^{a} and R^{b} each independently signify hydrogen or C₁-C₆-alkyl or together with the nitrogen atom to which they are attached signify a saturated 5- or 6-membered heterocyclic ring which can contain an additional nitrogen atom or an oxygen or sulphur atom as a further hetero atom, whereby the sulphur atom can also be present in the form of a sulphinyl or sulphonyl group, which can be substituted on one or two carbon atoms by C₁-C₆-alkyl, hydroxy, hydroxy-C₁-C₆-alkyl or C₁-C₆-alkoxy-C₁-C₆-alkoxy, which can be substituted on a second nitrogen atom which may be present by C₁-C₆-alkyl, benzyl, benzyloxycarbonyl, C₁-C₆-alkoxycarbonyl or hydroxy-C₁-C₆-alkyl, or which can carry an oxo group.

3. Compounds in accordance with claim 1 or 2, wherein R¹ signifies C₃-C₆-cycloalkyl, phenyl optionally mono- or multiply-substituted by C₁-C₆-alkyl, C₁-C₆-alkoxy, C₁-C₆-alkylcarbonyloxy, hydroxy, halogen or trifluoromethyl, R¹ also signifies naphthyl, thienyl or pyridyl.

4. Compounds in accordance with claim 3, wherein R¹ signifies cyclohexyl, phenyl, naphthyl or thienyl.

5. Compounds in accordance with claim 4, wherein R¹ signifies phenyl.

6. Compounds in accordance with any one of claims 1-5, wherein R² signifies hydrogen.

7. Compounds in accordance with any one of claims 1-6, wherein R³ signifies hydrogen, hydroxy, C₁-C₆-alkyl, C₁-C₆-alkoxy, allyloxy, C₁-C₆-alkylthio, C₁-C₆-alkylthiomethyl, C₁-C₆-alkoxycarbonyl, imidazolyl-1-ylmethyl, imidazol-4-ylmethyl, 2-aminoimidazol-4-ylmethyl, 5-iodoimidazol-4-ylmethyl, pyrazol-1-ylmethyl, thiazol-4-ylmethyl, thien-2-ylmethyl, furan-2-ylmethyl, pyridylmethyl or aminocarbonyl.

8. Compounds in accordance with claim 7, wherein R³ signifies hydrogen, hydroxy, C₁-C₄-alkyl, C₁-C₄-alkoxy, allyloxy, C₁-C₄-alkylthio, C₁-C₄-alkylthiomethyl, C₁-C₄-alkoxycarbonyl, imidazol-4-ylmethyl, thiazol-4-ylmethyl or pyridylmethyl.

9. Compounds in accordance with claim 7, wherein R³ signifies imidazol-4-ylmethyl or 3-pyridylmethyl.

10. Compounds in accordance with any one of claims 1-9, wherein R⁴ signifies C₃-C₆-cycloalkyl or phenyl.

11. Compounds in accordance with claim 10, wherein R⁴ signifies cyclohexyl.

12. Compounds in accordance with any one of claims 1-11, wherein R⁵ signifies C₃-C₆-cycloalkyl or C₁-C₆-alkyl.

13. Compounds in accordance with claim 12, wherein R⁵ signifies cyclopropyl, isopropyl or isobutyl.

14. Compounds in accordance with claim 12, wherein R⁵ signifies cyclopropyl.

15. Compounds in accordance with any one of claims 1-14, wherein B signifies sulphonyl.

16. Compounds in accordance with any one of claims 1-15, wherein R⁶ and R⁷ each signify hydrogen or C₁-C₄-alkyl.

17. Compounds in accordance with claim 16, wherein R⁶ and R⁷ each signify methyl.

18. Compounds in accordance with any one of claims 1-17, wherein Y signifies C₃-C₆-cycloalkylamino, sulpho-C₁-C₆-alkylamino, bis-C₁-C₆-alkoxy-C₁-C₆-alkylamino, pyridyl-C₁-C₆-alkylamino, which can be substituted on the amino group by C₁-C₆-alkyl, 4-piperidinyl-C₁-C₆-alkylamino, which can be substituted on the nitrogen atom by C₁-C₆-alkyl, benzyl, benzyloxycarbonyl, C₁-C₆-alkoxycarbonyl or hydroxy-C₁-C₆-alkyl, C₁-C₆-alkoxycarbonyl-C₁-C₆-alkylamino, hydroxy-C₁-C₆-alkoxy-C₁-C₆-alkylamino, hydroxy-C₁-C₆-alkylamino, bishydroxy-C₁-C₆-alkylamino or the group (R^{a}) (R^{b})N- in which R^{a} and R^{b} each independently signify hydrogen or C₁-C₆-alkyl or together with the nitrogen atom to which they are attached signify a saturated 5- or 6-membered heterocyclic ring which can contain an additional nitrogen atom or an oxygen or sulphur atom as a further hetero atom or which can carry an oxo group, whereby the sulphur atom can also be present in the form of a sulphinyl or sulphonyl group, or which can be substituted on one or two carbon atoms by C₁-C₆-alkyl, hydroxy, hydroxy-C₁-C₆-alkyl or C₁-C₆-alkoxy-C₁-C₆-alkyl or on a second nitrogen atom which may be present by C₁-C₆-alkyl, benzyl, benzyloxycarbonyl, C₁-C₆-alkoxycarbonyl or hydroxy-C₁-C₆-alkyl.

19. Compounds in accordance with claim 18, wherein Y signifies the group (R^{a})(R^{b})N- in which R^{a} and R^{b} have the significance given in claim 18.

20. Compounds in accordance with claim 18, wherein Y signifies pyrrolidinyl, piperidinyl, piperazinyl, morpholinyl, thiomorpholinyl, 2-hydroxymethylpyrrolidinyl, 3-hydroxypyrrolidinyl, 3,4-dihydroxypyrrolidinyl, 4-hydroxypiperidinyl, 4-oxopiperidinyl, 3,5-dimethylmorpholinyl, 4,4-dioxothiomorpholinyl, 4-oxothiomorpholinyl or 2,6-dimethylmorpholinyl.

21. Compounds in accordance with claim 18, wherein Y signifies morpholinyl.

22. Compounds in accordance with any one of claims 1-4, wherein R¹ signifies cyclohexyl, phenyl, naphthyl or thienyl, R² signifies hydrogen, R³ signifies hydrogen, hydroxy, C₁-C₄-alkyl, C₁-C₄-alkoxy, allyloxy, C₁-C₄-alkylthio, C₁-C₄-alkylthiomethyl, C₁-C₄-alkoxycarbonyl, imidazol-4-ylmethyl, thiazol-4-ylmethyl or pyridylmethyl, R⁴ signifies cyclohexyl, R⁵ signifies cyclopropyl, isopropyl or isobutyl, B signifies sulphonyl, R⁶ and R⁷ each signify methyl and Y signifies pyrrolidinyl, piperidinyl, piperazinyl, morpholinyl, thiomorpholinyl, 2-hydroxymethylpyrrolidinyl, 3-hydroxypyrrolidinyl, 3,4-dihydroxypyrrolidinyl, 4-hydroxypiperidinyl, 4-oxopiperidinyl, 3,5-dimethylmorpholinyl, 4,4-dioxothiomorpholinyl, 4-oxothiomorpholinyl or 2,6-dimethylmorpholinyl.

23. Compounds in accordance with claim 1, selected from (S)-N-[(1S,2R,3S)-1-(cyclohexylmethyl)-3-cyclopropyl-2,3-dihydroxypropyl]-α-[(S)-α-[[[1-[[2-hydroxy-1-(hydroxymethyl)-1-methylethyl]carbamoyl]-1-methylethyl]sulphonyl]methy]hydrocinnamamido]imidazole-4-propionamide, (S)-α-[(R or S)-α-[[[1-(2-pyridylmethyl)-carbamoyl]-1-methylethyl]sulphonyl]methyl]hydrocinnamamido]-N-[(1S,2R,3S)-1-(cyclohexylmethyl)-3-cyclopropyl-2,3-dihydroxypropyl]imidazole-4-propionamide or (S)-α-[(R or S)-α-[[[1-[(1-benzyl-4-piperidinyl)carbamoyl]-1-methylethyl]sulphonyl]methyl]-hydrocinnamamido]-N-[(1S,2R,3S)-1-(cyclohexylmethyl)-3-cyclopropyl-2,3-dihydroxypropyl]imidazole-4-propionamide.

24. Compounds in accordance with claim 1, selected from 2-[2-[[(S)-2-[[(S)-1-[[(1S,2R,3S)-1-(cyclohexylmethyl)-3-cyclopropyl-2,3-dihydroxypropyl]carbamoyl]-2-imidazol-4-ylethyl]carbamoyl]-3-phenylpropyl]sulphonyl]-2-methylpropionamido-2-deoxy-D-glucopyranose or (S)-α-[(R or S)-α-[[[1-[bis(2-methoxyethyl)carbamoyl]-1-methylethyl]sulphonyl]methyl]hydrocinnamamido]-N-[(1S,2R,3S)-1-(cyclohexylmethyl)-3-cyclopropyl-2,3-dihydroxypropyl]imidazole-4-propionamide.

25. Compounds in accordance with claim 1, selected from (S or R)-N-[(1S,2R,3S)-1-(cyclohexylmethyl)-3-cyclopropyl-2,3-dihydroxypropyl]-α-[(S)-α-[[[1-methyl-1- [morpholinocarbonyl]ethyl]sulphonyl]methyl]hydrocinnamamido]-3-(thiazol-4-yl)propionamide.

26. The compound in accordance with claim 1 having the name (S)-N-[(1S,2R,3S)-1-(cyclohexylmethyl)-3-cyclopropyl-2,3-dihydroxypropyl]-α-[(S)-α-[[[1-(morpholinocarbonyl)-1-methylethyl]sulphonyl]methyl]hydrocinnamamido]imidazole-4-propionamide.

27. The compound in accordance with claim 1 having the name (S)-N-[(1S,2R,3S)-1-(cyclohexylmethyl)-3-cyclopropyl-2,3-dihydroxypropyl]-α-[(S)-α-[[[1-[(4-hydroxypiperidino)carbonyl]-1-methylethyl]sulphonyl]methyl]hydrocinnamamido]-imidazole-4-propionamide.

28. Compounds in accordance with claim 1, selected from (S or R)-N-[(1S,2R,3S)-1-(cyclohexylmethyl)-3-cyclopropyl-2,3-dihydroxypropyl]-α-[(S)-α-[[[1-methyl-1-[morpholinocarbonyl]ethyl]sulphonyl]methyl]hydrocinnamamido]-3-(pyridin-3-yl)-propionamide.

29. Compounds of the general formulae 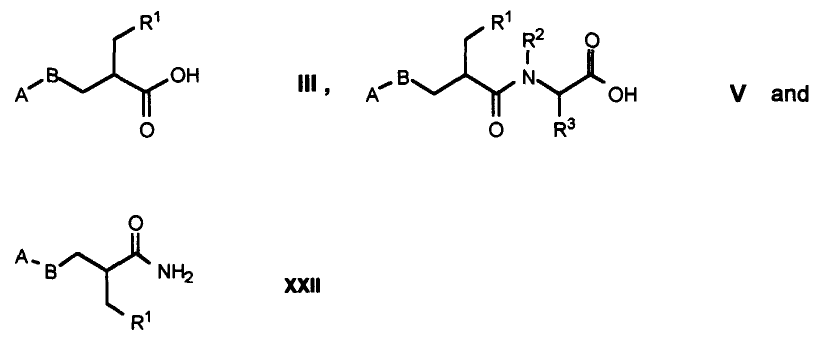 wherein A [signifies] the group 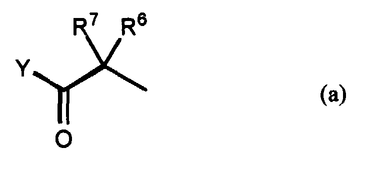 and B, Y, R¹, R², R³, R⁶, and R⁷ have the significance given in claim 1.

30. Amino acid derivatives in accordance with any one of claims 1-28 for use as therapeutically active substances.

31. Amino acid derivatives in accordance with any one of claims 1-28 for use in the control or prevention of high blood pressure and cardiac insufficiency.

32. A process for the manufacture of a compound in accordance with any one of claims 1-28, characterized by
a) reacting a compound of the general formula 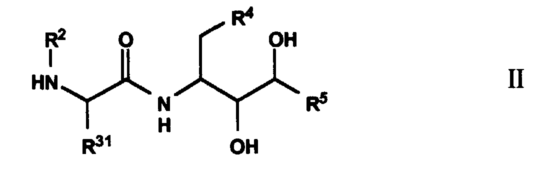
wherein R³¹ signifies hydrogen, C₁-C₆-alkyl, C₁-C₆-alkylthio-C₁-C₆-alkyl, C₁-C₆-alkoxycarbonyl, imidazolyl-1-ylmethyl, imidazol-2-ylmethyl or imidazol-4-ylmethyl, which can be methylated on a carbon atom and/or nitrogen atom, 2-aminoimidazol-4-yl-methyl, 5-iodoimidazol-4-ylmethyl, pyrazol-1-ylmethyl, pyrazol-3-ylmethyl, thiazol-2-ylmethyl, thiazol-4-ylmethyl, thiazol-5-ylmethyl, 2-aminothiazol-4-ylmethyl, thienylmethyl, furanylmethyl, pyridylmethyl, aminocarbonyl, aminocarbonyl-C₁-C₆-alkyl or C₁-C₆-alkoxycarbonyl-C₁-C₆-alkyl and R², R⁴ and R⁵ have the significance given in claim 1,
with an acid of the general formula 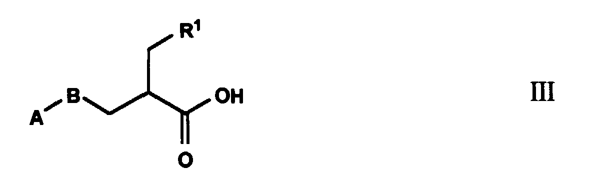
wherein A,B and R¹ have the significance given in claim 1, or an activated derivative thereof, or
b) reacting a compound of the general formula 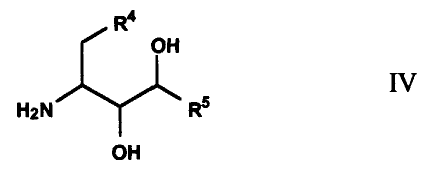
wherein R⁴ and R⁵ have the significance given in claim 1, with an acid of the general formula 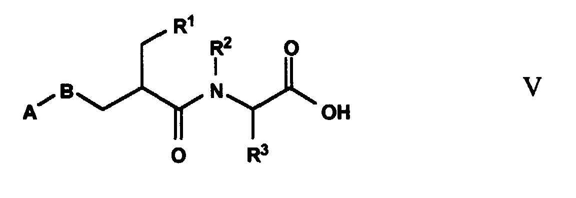
wherein A, B, R¹, R² and R³ have the significance given in claim 1, or an activated derivative thereof, or
c) for the manufacture of a compound of formula I in which A contains free primary or secondary amino groups and/or free primary or secondary hydroxy groups, cleaving off the N- or O-protecting group(s) from a compound of the general formula 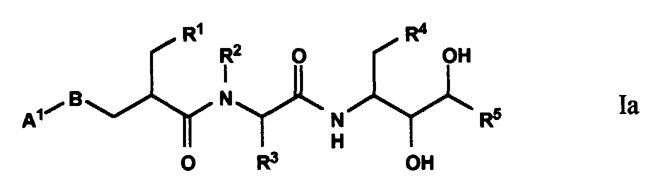
wherein A¹ has the same significance as A, with the proviso that A contains a N- or O-protecting group,
and
d) if desired, separating a mixture of diastereomeric racemates into the diastereomeric racemates or optically pure diastereomers, and/or
e) if desired, separating a mixture of diastereomers into the optically pure diastereomers, and/or
f) if desired, converting a compound obtained into a pharmaceutically usable salt

33. A medicament containing an amine acid derivative in accordance with any one of claims 1-28 and a therapeutically inert excipient.

34. A medicaments for the control or prevention of high blood pressure and cardiac insufficiency, containing an amino add derivative in accordance with any one of claims 1-28 and a therapeutically inert excipient.

35. The use of an amino acid derivative in accordance with any one of claims 1-28 for the production of medicaments against high blood pressure and/or cardiac insufficiency.

## Revendications

1. Dérivés d'acides aminés de formule 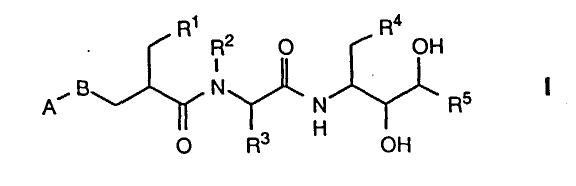
dans laquelle R¹ représente un cycloalkyle en C₃ à C₆, un phényle, éventuellement mono- ou polysubstitué par un alkyle en C₁ à C₆, un alcoxy en à C₆, un alkyle en C₁ à C₆-carbonyloxy, un hydroxy, un halogène ou un trifluorométhyle, R¹ représente également un naphtyle, un thiényle, un pyridyle, un quinoléyle, un isoquinoléyle ou un benzyle, R² représente un hydrogène ou un méthyle, R³ représente un hydrogène, un hydroxy, un alkyle en C₁ à C₆, un alcoxy en C₁ à C₆, un alcényloxy en C₃ à C₆, un alkylthio en C₁ à C₆, un alkylthio en C₁ à C₆-alkyle en C₁ à C₆, un alcoxy en C₁ à C₆-carbonyle, un imidazol-1-yl-méthyle, un imidazol-2-yl-méthyle ou un imidazol-4-yl-méthyle, qui peut être méthylé sur un atome de carbone et/ou un atome d'azote, un 2-aminoimidazol-4-yl-méthyle, un 5-iodoimidazol-4-yl-méthyle, un pyrazol-1-yl-méthyle, un pyrazol-3-yl-méthyle, un thiazol-2-yl-méthyle, un thiazol-4-yl-méthyle, un thiazol-5-yl-méthyle, un 2-amino-thiazol-4-yl-méthyle, un thiénylméthyle, un furanylméthyle, un pyridylméthyle, un aminocarbonyle, un aminocarbonylalkyle en C₁ à C₆ ou un alcoxy en C₁ à C₆-carbonylalkyle en C₁ à C₆, R⁴ représente un cycloalkyle en C₃ à C₆, éventuellement substitué par un alkyle en C₁ à C₆, un hydroxy ou un halogène, R⁴ représente en outre un phényle ou un halophényle, R⁵ représente un cycloalkyle en C₃ à C₆, un cycloalkyle en C₃ à C₆-alkyle en C₁ à C₆ ou un alkyle en C₁ à C₆, B représente un atome de soufre ou un groupe sulfinyle ou sulfonyle et A représente le groupe 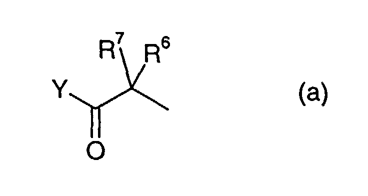 dans lequel R⁶ et R⁷ représentent indépendamment l'un de l'autre chacun un hydrogène ou un alkyle en C₁ à C₆, et Y représente un cycloalkylamino en C₃ à C₆, un sulfo-alkylamino en C₁ à C₆, un bis-alcoxy en C₁ à C₆-alkylamino en C₁ à C₆, ou un pyridyl-alkylamino en C₁ à C₆ ou un morpholino-alkyle en C₁ à C₆, qui peut à chaque fois être susbtitué sur le groupe amino par un alkyle en C₁ à C₆, un pyrazinyl-alkylamino en C₁ à C₆, qui peut être substitué sur l'atome d'azote et/ou sur le groupe amino par un alkyle en C₁ à C₆, un 4-pipéridinylalkylamino en C₁ à C₆, qui peut être substitué sur l'atome d'azote par un alkyle en C₁ à C₆, un benzyle, un benzyloxy carbonyle, un alcoxy en C₁ à C₆-carbonyle ou un hydroxy-alkyle en C₁ à C₆ et/ou sur le groupe amino par un alkyle en C₁ à C₆, un alcoxy en C₁ à C₆ carbonyl-alkylamino en C₁ à C₆, un hydroxy-alcoxy en C₁ à C₆-alkylamino en C₁ à C₆, un hydroxy-alkylamino en C₁ à C₆, un bis hydroxy-alkylamino en C₁ à C₆, le radical d'un sucre amino désoxy naturel, ou le groupe (R^{a})(R^{b})N-, dans lequel R^{a} et R^{b} représentent indépendamment l'un de l'autre chacun un hydrogène ou un alkyle en C₁ à C₆ ou forment ensemble avec l'atome d'azote auxquels ils sont liés un noyau hétérocyclique saturé à 5 ou 6 chaînons qui peut contenir un atome d'azote supplémentaire ou un atome d'oxygène ou de soufre comme autre hétéroatome, ou présenter un groupe oxo, l'atome de soufre pouvant également se présenter sous la forme d'un groupe sulfinyle ou sulfonyle, ou bien pouvant être substitué sur un ou deux atomes de carbone par un alkyle en C₁ à C₆, un hydroxy, un hydroxy-alkyle en C₁ à C₆ ou un alcoxy en C₁ à C₆-alcoxy en C₁ à C₆ ou sur le second atome d'azote éventuellement présent par un alkyle en C₁ à C₆, un benzyle, un benzyloxycarbonyle, un alcoxy en C₁ à C₆ carbonyle ou un hydroxy-alkyle en C₁ à C₆, ou dans lequel le second atome d'azote éventuellement présent peut porter un atome d'oxygène,
sous forme de diastéréomères optiquement purs, de mélanges de diastéréomères, de racémates diastéréomères ou de mélanges de racémates diastéreomères, ainsi que les sels pharmaceutiquement acceptables de ces composés.

2. Composés selon la revendication 1, dans lesquels R¹ représente un cycloalkyle en C₃ à C₆, un phényle, éventuellement mono- ou polysubstitué par un alkyle en C₁ à C₆, un alcoxy en C₁ à C₆, un alkyle en C₁ à C₆-carbonyloxy, un hydroxy, un halogène ou un trifluorométhyle, R¹ représente également un naphtyle, un thiényle, un pyridyle, un quinoléyle, un isoquinoléyle ou un benzyle, R² représente un hydrogène ou un méthyle, R³ représente un hydrogène, un hydroxy, un alkyle en C₁ à C₆, un alcoxy en C₁ à C₆, un alcényloxy en C₃ à C₆, un alkylthio en C₁ à C₆, un alkylthio en C₁ à C₆-alkyle en C₁ à C₆, un alcoxy en C₁ à C₆-carbonyle, un imidazol-2-yl-méthyle ou un imidazol-4-yl-méthyle, qui peut être méthylé sur un atome de carbone et/ou un atome d'azote, un 2-aminoimidazol-4-yl-méthyle, un 5-iodoimidazol-4-yl-méthyle, un thiazol-4-yl-méthyle, un thiénylméthyle, un furanylméthyle, un pyridylméthyle, un aminocarbonyle, un aminocarbonyl-alkyle en C₁ à C₆ ou un alcoxy en C₁ à C₆-carbonyl-alkyle en C₁ à C₆, R⁴ représente un cycloalkyle en C₃ à C₆, éventuellement substitué par un alkyle en C₁ à C₆, un hydroxy ou un halogène, R⁴ représente en outre un phényle ou un halophényle, R⁵ représente un cycloalkyle en C₃ à C₆, un cycloalkyle en C₃ à C₆-alkyle en C₁ à C₆ ou un alkyle en C₁ à C₆, B représente un atome de soufre ou un groupe sulfinyle ou sulfonyle, R⁶ et R⁷ représentent chacun indépendamment l'un de l'autre un hydrogène ou un alkyle en C₁ à C₆, et Y représente un cycloalkylamino en C₃ à C₆, un sulfo-alkylamino en C₁ à C₆, un bis-alcoxy en C₁ à C₆-alkylamino en C₁ à C₆, un pyridyl-alkylamino en C₁ à C₆, un 4-pipéridinyl-alkylamino en C₁ à C₆, qui peut être substitué sur l'atome d'azote par un alkyle en C₁ à C₆, un benzyle, un benzyloxy cabonyle, un alcoxy en C₁ à C₆-carbonyle ou un hydroxy-alkyle en C₁ à C₆, un alcoxy en C₁ à C₆ carbonyl-alkylamino en C₁ à C₆, un hydroxy-alkylamino en C₁ à C₆, un bis hydroxy-alkylamino en C₁ à C₆, le radical d'un sucre amino désoxy naturel, ou le groupe (R^{a})(R^{b})N-, dans lequel R^{a} et R^{b} représentent indépendamment l'un de l'autre chacun un hydrogène ou un alkyle en C₁ à C₆ ou forment ensemble avec l'atome d'azote auxquels ils sont liés un noyau hétérocyclique saturé à 5 ou 6 chaînons qui peut contenir un atome d'azote supplémentaire ou un atome d'oxygène ou de soufre comme autre hétéroatome, ou présenter un groupe oxo, l'atome de soufre pouvant également se présenter sous la forme d'un groupe sulfinyle ou sulfonyle, ou bien peut être substitué sur un ou deux atomes de carbone par un alkyle en C₁ à C₆, un hydroxy, un hydroxy-alkyle en C₁ à C₆ ou un alcoxy en C₁ à C₆-alcoxy en C₁ à C₆ ou sur le second atome d'azote éventuellement présent par un alkyle en C₁ à C₆, un benzyle, un benzyloxycarbonyle, un alcoxy en C₁ à C₆ carbonyle ou un hydroxy-alkyle en C₁ à C₆, ou présenter un groupe oxo.

3. Composés selon la revendication 1 ou 2 dans lesquels R¹ représente un cycloalkyle en C₃ à C₆, un phényle, éventuellement mono- ou polysubstitué par un alkyle en C₁ à C₆, un alcoxy en C₁ à C₆, un alkyle en C₁ à C₆ carbonyloxy, un hydroxy, un halogène ou un trifluorométhyle, R¹ représente également un naphtyle, un thiényle ou un pyridyle.

4. Composés selon la revendication 3, dans lesquels R¹ représente un cyclohexyle, un phényle, un naphtyle ou un thiényle.

5. Composés selon la revendication 3, dans lesquels R¹ représente un phényle.

6. Composés selon l'une des revendications 1 à 5 dans lesquels R² représente un hydrogène.

7. Composés selon l'une des revendications 1 à 6, dans lesquels R³ représente un hydrogène, un hydroxy, un alkyle en C₁ à C₆, un alcoxy en C₁ à C₆, un allyloxy, un alkylthio en C₁ à C₆, un alkyle en C₁ à C₆ thiométhyle, un alcoxy en en C₁ à C₆ carbonyle, un imidazol-1-yl-méthyle, un imidazol-4-yl-méthyle, un 2-aminoimidazol-4-yl-méthyle, un 5-iodoimidazol-4-yl-méthyle, un pyrazol-1-yl-méthyle, un thiazol-4-yl-méthyle, un thién-2-yl-méthyle, un furan-2-yl-méthyle, un pyridylméthyle ou un aminocarbonyle.

8. Composés selon la revendication 7, dans lesquels R³ représente un hydrogène, un hydroxy, un alkyle en C₁ à C₄, un alcoxy en C₁ à C₄, un allyloxy, un alkylthio en C₁ à C₄, un alkyle en C₁ à C₄-thiométhyle, un alcoxy en C₁ à C₄-carbonyle, un imidazol-4-yl-méthyle, un thiazol-4-yl-méthyle ou un pyridyl-méthyle.

9. Composés selon la revendication 7, dans lesquels R³ représente un imidazol-4-yl-méthyle, ou un 3-pyridylméthyle.

10. Composés selon l'une des revendications 1 à 9, dans lesquels R⁴ représente un cycloalkyle en C₃ à C₆ ou un phényle.

11. Composés selon la revendication 10, dans lesquels R⁴ représente un cyclohexyle.

12. Composés selon l'une des revendications 1 à 11, dans lesquels R⁵ représente un cycloalkyle en C₃ à C₆ ou un alkyle en C₁ à C₆.

13. Composés selon la revendication 12, dans lesquels R⁵ représente un cyclopropyle, un isopropyle ou un isobutyle.

14. Composés selon la revendication 12, dans lesquels R⁵ représente un cyclopropyle.

15. Composés selon l'une des revendications 1 à 14, dans lesquels B représente un sulfonyle.

16. Composés selon l'une des revendications 1 à 15, dans lesquels R⁶ et R⁷ représentent chacun un hydrogène ou un alkyle en C₁ à C₄.

17. Composés selon la revendication 16, dans lesquels R⁶ et R⁷ représentent chacun un méthyle.

18. Composés selon l'une des revendications 1 à 17, dans lesquels Y représente un cycloalkylamino en C₃ à C₆, un sulfo-alkylamino en C₁ à C₆, un bis-alcoxy en C₁ à C₆-alkylamino en C₁ à C₆, un pyridyl-alkylamino en C₁ à C₆, qui peut être substitué sur le groupe amino par un alkyle en C₁ à C₆, un 4-pipéridinyl-alkylamino en C₁ à C₆, qui peut être substitué sur l'atome d'azote par un alkyle en C₁ à C₆, un benzyle, un benzyloxy carbonyle, un alcoxy en C₁ à C₆-carbonyle ou un hydroxy-alkyle en C₁ à C₆, un alcoxy en C₁ à C₆ carbonyl-alkylamino en C₁ à C₆, un hydroxy-alcoxy en C₁ à C₆-alkylamino en C₁ à C₆, un hydroxy-alkylamino en C₁ à C₆, un bis hydroxy-alkylamino en C₁ à C₆, ou le groupe (R^{a})(R^{b})N-, dans lequel R^{a} et R^{b} représentent indépendamment l'un de l'autre chacun un hydrogène ou un alkyle en C₁ à C₆ ou forment ensemble avec l'atome d'azote auxquels ils sont liés un noyau hétérocyclique saturé à 5 ou 6 chaînons qui peut contenir un atome d'azote supplémentaire ou un atome d'oxygène ou de soufre comme autre hétéroatome, ou présenter un groupe oxo, l'atome d'oxygène pouvant également se présenter sous la forme d'un groupe sulfinyle ou sulfonyle, ou bien pouvant être substitué sur un ou deux atomes de carbone par un alkyle en C₁ à C₆, un hydroxy, un hydroxy-alkyle en C₁ à C₆ ou un alcoxy en C₁ à C₆-alcoxy en C₁ à C₆ ou, sur le second atome d'azote éventuellement présent, par un alkyle en C₁ à C₆, un benzyle, un benzyloxycarbonyle, un alcoxy en C₁ à C₆ carbonyle ou un hydroxy-alkyle en C₁ à C₆.

19. Composés selon la revendication 18, dans lesquels Y représente le groupe (R^{a})(R^{b})N-, dans lequel R^{a} et R^{b} ont la signification indiquée dans la revendication 18.

20. Composés selon la revendication 18, dans lesquels Y représente un pyrrolidinyle, pipéridinyle, pipérazinyle, morpholinyle, thiomorpholinyle, 2-hydroxyméthylpyrrolidinyle, 3-hydroxypyrrolidinyle, 3,4-dihydroxypyrrolidinyle, 4-hydroxypipéridinyle, 4-oxopipéridinyle, 3,5-diméthylmorpholinyle, 4,4-dioxothiomorpholinyle, 4-oxothiomorpholinyle ou 2,6-diméthylmorpholinyle.

21. Composés selon la revendication 18, dans lesquels Y représente un morpholinyle.

22. Composés selon l'une des revendications 1 à 4, dans lesquels R¹ représente un cyclohexyle, un phényle, un naphtyle ou un thiényle, R² représente un hydrogène, R³ représente un hydrogène, un hydroxy, un alkyle en C₁ à C₄, un alcoxy en C₁ à C₄, un allyloxy, un alkylthio en C₁ à C₄, un alkyle en C₁ à C₄ thiométhyle, un alcoxy en C₁ à C₄ carbonyle, un imidazol-4-yl-méthyle, un thiazol-4-ylméthyle ou un pyridylméthyle, R⁴ représente un cyclohexyle, R⁵ représente un cyclopropyle, un isopropyle ou un isobutyle, B représente un sulfonyle, R⁶ et R⁷ représentent chacun un méthyle et Y un pyrrolidinyle, un pipéridinyle, un pipérazinyle, un morpholinyle, un thimorpholinyle, un 2-hydroxyméthylpyrrolidinyle, un 3-hydroxypyrrolidinyle, un 3,4-dihydroxypyrrolidinyle, un 4-hydroxypipéridinyle, un 4-oxopipéridinyle, un 3,5-diméthylmorpholinyle, un 4,4-dioxothiomorpholinyle, un 4-oxothiomorpholinyle ou un 2,6-diméthylmorpholinyle.

23. Composés selon la revendication 1, choisis parmi le (S)-N-[(1S,2R,3S)-1-(cyclohexylméthyl)-3-cyclopropyl-2,3-dihydroxypropyl]-α-[(S)-α-[[[1-[[2-hydroxy-1-(hydroxyméthyl)-1-méthyléthyl]carbamoyl]-1-méthyléthyl]sulfonyl]méthyl]hydrocinnamamido]imidazol-4-propionamide, le (S)-α-[(R ou S)-α-[[[1-[(2-pyridylméthyl)carbamoyl]-1-méthyléthyl]sulfonyl]méthyl]hydroxycinnamamido]-N-[(1S,2R,3S)-1-(cyclohexylméthyl)-3-cyclopropyl-2,3-dihydroxypropyl]imidazole-4-propionamide ou le (S)-α-[(R ou S)-α-[[[1-[(1-benzyl-4-pipéridinyl)carbamoyl]-1-méthyléthyl]sulfonyl]méthyl]hydroxycinnamamido]-N-[(1S,2R,3S)-1-(cyclohexylméthyl)-3-cyclopropyl-2,3-dihydroxypropyl]imidazole-4-propionamide.

24. Composés selon la revendication 1 choisis parmi le 2-[2-[[(S)-2-[[(S)-1-[[(1S,2R,3S)-1-(cyclohexylméthyl)-3-cyclopropyl-2,3-dihydroxypropyl]carbamoyl]-2-imidazol-4-yléthyl]carbamoyl]-3-phénylpropyl]sulfonyl]-2-méthylpropionamido]-2-désoxy-D-glucopyranose ou le (S)-α-[(R ou S)-α-[[[1-[bis(2-méthoxyéthyl)carbamoyl]-1-méthyléthyl]sulfonyl]méthyl]hydroxycinnamamido]-N- [(1S,2R,3S)-1-(cyclohexylméthyl)-3-cyclopropyl-2,3-dihydroxypropyl]imidazole-4-propionamide.

25. Composés selon la revendication 1, choisis parmi le (S ou R)-N-[(1S,2R,3S)-1-(cyclohexylméthyl)-3-cyclopropyl-2,3-dihydroxypropyl]-α-[(S)-α-[[[1-méthyl-1-[morpholinocarbonyl]éthyl]sulfonyl]méthyl]-hydrocinnamido]-3-(thiazol-4-yl)propionamide.

26. Composé selon la revendication 1 sous la désignation de (S)-N-[(1S,2R,3S)-1-(cyclohexylméthyl)-3-cyclopropyl-2,3-dihydroxypropyl]-α-[(S)-α-[[[1-(morpholinocarbonyl)-1-méthyléthyl]sulfonyl]méthyl]-hydrocinnamamido]imidazole-4-propionamide.

27. Composé selon la revendication 1 désigné comme (S)-N-[(1S,2R,3S)-1-(cyclohexylméthyl)-3-cyclopropyl-2,3-dihydroxypropyl]-α-[(S)-α-[[[1-[(4-hydroxypipéridino)carbonyl]-1-méthyléthyl]sulfonyl]-méthyl]hydrocinnamamido]imidazole-4-propionamide.

28. Composés selon la revendication 1, choisis parmi le (S ou R)-N-[(1S,2R,3S)-1-(cyclohexylméthyl)-3-cyclopropyl-2,3-dihydroxypropyl]-α-[(S)-α-[[[1-méthyl-1-[morpholinocarbonyl]éthyl]sulfonyl]méthyl]-hydrocinnamamido]-3-(pyridin-3-yl)propionamide.

29. Composés de formules générales 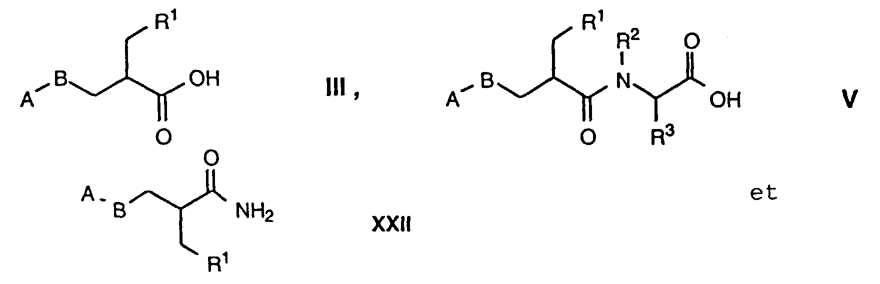 dans lesquelles A représente le groupe 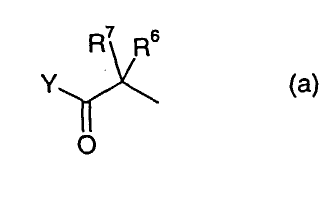 et B, Y, R¹, R², R³, R⁶ et R⁷ ont la signification indiquée dans la revendication 1.

30. Dérivés d'acides aminés selon l'une des revendications 1 à 28 aux fins d'application comme substances actives thérapeutiques.

31. Dérivés d'acides aminés des revendications 1 à 28 aux fins d'application pour combattre ou selon les cas prévenir l'hypertension artérielle et l'insuffisance cardiaque.

32. Procédé de préparation d'un composé selon l'une des revendications 1 à 28, caractérisé en ce que
a) on fait réagir un composé de formule générale 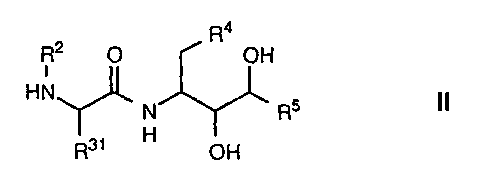 dans laquelle R³¹ représente un hydrogène, un alkyle en C₁ à C₆, un alkylthio en C₁ à C₆-alkyle en C₁ à C₆, un alcoxy en C₁ à C₆-carbonyle, un imidazol-1-yl-méthyle, un imidazol-2-yl-méthyle ou un imidazol-4-yl-méthyle, qui peuvent être méthylés sur l'atome de carbone et/ou l'atome d'azote, un 2-aminoimidazol-4-yl-méthyle, un 5-iodoimidazol-4-yl-méthyle, un pyrazol-1-ylméthyle, un pyrazol-3-ylméthyle, un thiazol-2-ylméthyle, un thiazol-4-ylméthyle, un thiazol-5-ylméthyle, un 2-aminothiazol-4-ylméthyle, un thiénylméthyle, un furanylméthyle, un pyridylméthyle, un aminocarbonyle, un aminocarbonyl-alkyle en C₁ à C₆ ou un alcoxycarbonyle en C₁ à C₆-alkyle en C₁ à C₆, et R², R⁴ et R⁵ ont la signification indiquée dans la revendication 1,
avec un acide de formule générale 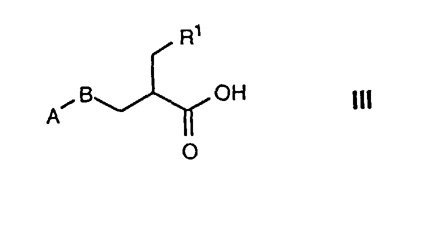 dans laquelle A, B et R¹ ont la signification indiquée dans la revendication 1,
ou un de ses dérivés activés, ou
b) on fait réagir un composé de formule générale 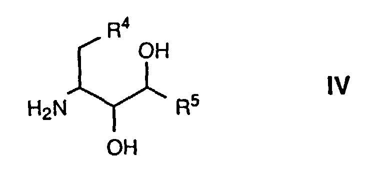
dans laquelle R⁴ et R⁵ ont la signification indiquée dans la revendication 1,
avec un acide de formule générale 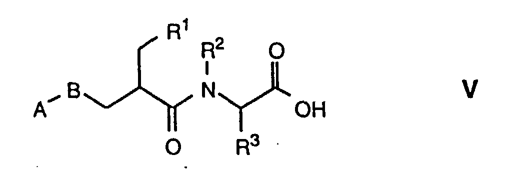
dans laquelle A, B, R¹, R² et R³ ont la signification indiquée dans la revendication 1, ou de ses dérivés activés, ou
c) pour préparer un composé de formule I, dans laquelle A contient des groupes amino primaires ou secondaires libres et/ou des groupes hydroxy secondaires libres, à partir d'un composé de formule générale 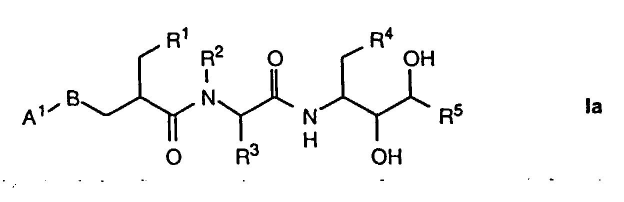
dans laquelle A¹ a la même signification que A, sous réserve que A représente un groupe protecteur de N ou de O,
on sépare le ou les groupes protecteurs de N ou de O, et
d) si on le désire, on dédouble un mélange de racémates diastéréomères en les racémates diastéréomères ou en les diastéréomères optiquement purs, et/ou
e) si on le désire, on sépare un mélange de diastéréomères en les diastéréomères optiquement purs, et/ou
f) si on le désire, on transforme un composé obtenu en un sel pharmaceutiquement acceptable.

33. Médicament contenant un dérivé d'acide aminé selon l'une des revendications 1 à 28 et un excipient thérapeutiquement inerte.

34. Agent pour combattre ou prévenir l'hypertension artérielle et l'insuffisance cardiaque, contenant un dérivé d'acides aminés selon l'une des revendications 1 à 28 et un excipient thérapeutiquement inerte.

35. Utilisation d'un dérivé d'acides aminés selon l'une des revendications 1 à 28 pour préparer des agents contre l'hypertension artérielle et/ou l'insuffisance cardiaque.
